(19) Europäisches Patentamt
European Patent Office
Office européen des brevets

(11) EP 3 541 793 B1

(12) **EUROPÄISCHE PATENTSCHRIFT**

(45) Veröffentlichungstag und Bekanntmachung des Hinweises auf die Patenterteilung:
**31.03.2021 Patentblatt 2021/13**

(21) Anmeldenummer: **17793674.7**

(22) Anmeldetag: **07.11.2017**

(51) Int Cl.:
*C07D 309/34* (2006.01) *C07C 271/20* (2006.01)
*C07C 271/24* (2006.01) *C07C 271/28* (2006.01)
*C07F 9/54* (2006.01) *C07D 233/58* (2006.01)
*C07D 335/02* (2006.01) *C07C 211/63* (2006.01)
*C07C 211/64* (2006.01) *C07C 271/16* (2006.01)
*C07D 233/02* (2006.01) *C07D 347/00* (2006.01)
*C07D 247/00* (2006.01)

(86) Internationale Anmeldenummer:
**PCT/EP2017/078415**

(87) Internationale Veröffentlichungsnummer:
**WO 2018/087064 (17.05.2018 Gazette 2018/20)**

(54) **VERFAHREN ZUR HERSTELLUNG VON TRIARYL-ORGANOBORATEN**

PROCESS FOR THE MANUFACTURING OF TRIARYL-ALKYL BORATES

PROCÉDÉ DE FABRICATION DE TRIARYL-ALKYLBORATES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.11.2016 EP 16197990**
**24.08.2017 EP 17187666**

(43) Veröffentlichungstag der Anmeldung:
**25.09.2019 Patentblatt 2019/39**

(60) Teilanmeldung:
**20205836.8**
**20205840.0**

(73) Patentinhaber: **Covestro Intellectual Property GmbH & Co. KG**
**51373 Leverkusen (DE)**

(72) Erfinder:
- **RÖLLE, Thomas**
**51381 Leverkusen (DE)**
- **BERNETH, Horst**
**51373 Leverkusen (DE)**
- **HÖNEL, Dennis**
**53909 Zülpich-Wichterich (DE)**
- **BRUDER, Friedrich-Karl**
**47802 Krefeld (DE)**
- **KINTRUP, Jürgen**
**51373 Leverkusen (DE)**

(74) Vertreter: **Levpat**
**c/o Covestro AG**
**Gebäude 4825**
**51365 Leverkusen (DE)**

(56) Entgegenhaltungen:
**WO-A1-2015/091427 WO-A1-2018/087062**
**DE-A1- 19 850 139 JP-A- 2001 233 881**
**JP-A- 2002 226 486**

- **DATABASE CA [Online] CHEMICAL ABSTRACTS SERVICE, COLUMBUS, OHIO, US; 1996, SARKER, ANANDA M. ET AL: "Visible light photopolymerization employing 2,4-diiodo-6-butoxy-3-fluorone and tetraorganylborate salts as photoinitiators", XP002769399, gefunden im STN Database accession no. 1996:533314**
- **DATABASE CA [Online] CHEMICAL ABSTRACTS SERVICE, COLUMBUS, OHIO, US; 1997, HOWELL, BARBARA F. ET AL: "Pigmented coatings cured with visible light", XP002769400, gefunden im STN Database accession no. 1997:737452**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- DATABASE CA [Online] CHEMICAL ABSTRACTS SERVICE, COLUMBUS, OHIO, US; 2009, MIYAGI, MARIE ET AL: "Onium cation borate-based antistatic agents, their resin compositions, coating solutions, coated films, moldings, and manufacture of the moldings, etc.", XP002776303, gefunden im STN Database accession no. 2009:172388
- A. F. Holleman ET AL: "Acidität und Basizität" In: "Lehrbuch der Anorganischen Chemie", 1 January 1985 (1985-01-01), De Gruyter, XP55739586, pages 235-243,

## Beschreibung

Die Erfindung betrifft ein Verfahren zur Herstellung von Triaryl-organoboraten ausgehend von Organo-Boronsäureestern in Gegenwart eines n-wertigen Kations 1/n $K^{n+}$ umfassend die wasserfreie Aufarbeitung des Reaktionsgemischs.

Triaryl-organoborate können zusammen mit geeigneten Sensibilisatoren wie beispielsweise kationischen, anionischen oder neutralen Farbstoffen Typ(II) Photoinitiatoren bilden, die durch aktinische Strahlung eine radikalische Photopolymerisation geeigneter Monomere auslösen. Ihre Herstellung ist breit beschrieben und ausgewählte Tetraalkylammonium-triaryl-alkylborate sind kommerziell erhältlich.

DE 198 50 139 A1 beschreibt die Synthese von Tetraalkylammonium-triaryl-alkylboraten ausgehend von einem Alkyl- oder Cycloalkyl-Boronsäureester und deren Umsetzung mit drei Äquivalenten eines metallorganischen Reagenzes insbesondere einem Grignard-Reagenz. Dabei wird in einem ersten Schritt der Alkyl- oder Cycloalkyl-Boronsäureester hergestellt, isoliert und dieser Alkyl- oder Cycloalkyl-Boronsäureester anschließend mit einem separat hergestellten metallorganischen Reagenz, i.d.R. einem Grignard-Reagenz, umgesetzt. In einem dritten Schritt wird das so entstandene Triaryl-alkylborat durch Zugabe einer Tetraalkylammonium-Verbindung als Salz gefällt und z.B. durch Kristallisation gereinigt. Somit ergeben sich zur Herstellung von Tetraalkylammonium-triaryl-alkylboraten insgesamt 3 Synthese Stufen. Da die Löslichkeit von metallorganischen Reagenzien insbesondere Grignard-Reagenzien in den üblicherweise verwendeten ätherischen Lösungsmitteln begrenzt ist, muss bei diesem Verfahren insbesondere auf der Stufe des metallorganischen Reagenzes in recht verdünnter Lösung gearbeitet werden, um alle Inhaltsstoffe homogen in Lösung zu halten. Die Löslichkeit von Grignard-Reagenzien in den typischerweise verwendeten Ethern Tetrahydrofuran oder Diethylether beträgt üblicherweise 1 bis 2 Mol pro Liter. Da jedoch auf Grund der Stöchiometrie drei Äquivalente Grignard-Reagenz benötigt werden, liegt die Zielkonzentration an Produkt bei einem Drittel dieses Wertes, d.h. die Löslichkeit des Grignard-Reagenzes gibt die Zielkonzentration vor. Für technische Anwendung ist eine optimierte Raum-Zeit-Ausbeute ein wichtiges Ziel. Dies kann nach den in der DE 198 50 139 A1 beschriebenen Verfahren jedoch nur begrenzt erreicht werden.

JP2002-226486 A beschreibt die Synthese von Ammonium- und Phosphonium-triaryl-alkylboraten ausgehend von einem Alkyl- oder Cycloalkyl-Boronsäureester und deren Umsetzung mit drei Äquivalenten eines metallorganischen Reagenzes insbesondere einem Grignard-Reagenz nach einer *in situ* Methode (Barbier), bei der das metallorganische Reagenz in Gegenwart des elektrophilen Alkyl- oder Cycloalkyl-Boronsäureesters erzeugt wird. So können höhere Raum-Zeit-Ausbeuten erzielt werden und die Kontrolle der Exothermie der metallorganischen Stufe gelingt so besser. Darüber hinaus sind reaktive metallorganische Reagenzien teilweise nur bedingt lagerstabil und müssen mit hohem Aufwand vor Wasser oder Luft-Sauerstoff geschützt werden, da sie ansonsten hydrolysieren oder oxidiert werden, was wiederum die Gesamtausbeute reduziert.

Beide oben beschriebenen Verfahren erfordern jedoch noch die Zugabe des gewählten Kations in einem weiteren Reaktionsschritt. Für eine wirtschaftliche Herstellung im industriellen Maßstab ist es vorteilhaft, wenn das gewählte Kation bereits bei der Herstellung des Triaryl-organoborats vorliegt, weil so ein weiterer Reaktionsschritt gespart und die Durchführung und Aufarbeitung effizienter gestaltet werden kann. Zudem können die Konzentration der Reagenzien weiter erhöht werden, da die ansonsten anfallenden Metall-Aryl-Ammonium-Salze in den verwendeten Lösungsmittel nur begrenzt löslich sind und durch voluminös ausfallenden Niederschlag die Kontrolle der Reaktionsenthalpie verhindern.

DE 198 50 139 A1 offenbart Chinoliniumfarbstoffe und Borate in photopolymerisierbaren Zusammensetzungen.

JP 2002 226486 A offenbart ein Verfahren zur Herstellung von Boratverbindungen.

JP 2001 233881 A offenbart ein Verfahren zur Herstellung von Triarylalkylboraten.

WO 2015/091427 A1 offenbart holographische Medien mit verbesserter Lichtempfindlichkeit.

Sarker, Ananda et al, Journal of Polymer Science, Part A: Polymer Chemistry (1996), 34(13), 2817-2824 offenbart Photopolymerisation mit sichtbarem Licht durch Anwendung von 2,4-dijod-6-butoxy-3-fluoron und Tetraorganylboratsalzen als Photoinitiatoren.

Howell, Barbara et al, ACS Symposium Series (1997), 673 (Photopolymerization: Fundamentals and Applications), 219-232 offenbart pigmentierte Beschichtungen die mit sichbarem Licht gehärtet wurden.

JP2009029857A offenbart Oniumkation-Borat basierte antistatische Mittel, ihre Harzzusammensetzungen, Beschichtungslösungen, beschichtete Filme, Formteile und die Herstellung der Formteile etc.

WO 2018/087062 A1 offenbart ein Verfahren zur Herstellung Triarylorganoboraten.

Es war daher die Aufgabe der vorliegenden Erfindung, ein Verfahren zur Herstellung von Triarylorganoboraten bereit zu stellen, bei dem eine verbesserte Raum-Zeit-Ausbeute zu einer besseren Wirtschaftlichkeit führt.

Diese Aufgabe wurde erfindungsgemäß durch ein Verfahren zur Herstellung von Triarylorganoboraten der Formel 1/n $1/n\ K^{n+}R^4_3B^{-}\text{-}R^1$ (IV) gelöst, bei dem ein Äquivalent Organo-Boronsäureester der Formel $B\text{-}R^1(OR^2)(OR^3)$ (I) mit 1/n Äquivalenten Salz $K^{n+}\ nX^-$ (II) und 3 Äquivalenten Metall M in einem Lösungsmittel oder einem Lösungsmittelgemisch S1 vorgelegt, 3 Äquivalente eines Halogen-Aromaten $R^4$-Y (III) zugegeben werden, ein Hilfsstoff L und gegebenenfalls ein zweites organisches Lösungsmittel oder Lösungsmittelgemisch S2 zugegeben wird und die Verbindung $1/n\ K^{n+} R^4_3 B^- \text{-} R^1$ (IV) mit der organischen Phase abgetrennt wird und

$R^1$ für einen gegebenenfalls mit Hydroxy und / oder Alkoxy und / oder Acyloxy und / oder Halogen substituierten $C_1$- bis $C_{22}$-Alkyl-, $C_3$- bis $C_{22}$-Alkenyl-, $C_3$- bis $C_{22}$-Alkinyl-, $C_5$- bis $C_7$-Cycloalkyl- oder $C_7$- bis $C_3$-Aralkylrest steht,

$R^2$ und $R^3$ unabhängig voneinander für einen gegebenenfalls verzweigten $C_1$- bis $C_{22}$-Alkyl-Rest oder einen gegebenenfalls durch Alkyl substituierten $C_3$- bis $C_7$-Cycloalkyl-Rest stehen oder $R^2$ und $R^3$ gemeinsam eine 2-8 gliedrige gegebenenfalls durch Alkyl substituierte und / oder durch Sauerstoff-Atome unterbrochene Kohlenstoff-Brücke bilden,

$R^4$ für einen $C_6$- bis $C_{10}$-Arylrest steht, der gegebenenfalls durch mindestens einen Rest der Auswahl Halogen, $C_1$-bis $C_4$-Alkyl, Trifluormethyl, $C_1$-bis $C_4$-Alkoxy, Trifluormethoxy, Phenyl oder Phenoxy substituiert ist,

K für ein beliebig substituiertes Organokation der Wertigkeit n auf Basis von Stickstoff, Phosphor, Sauerstoff, Schwefel, und / oder Iod steht und

L für einen mit M-Salzen $MY(OR^2)$, $MY(OR^3)$ und MXY in S1 und / oder S2 schwer-löslichen Komplex bildenden Hilfsstoff steht, wobei L eine Lewis-basische Verbindung ist, ausgewählt aus der Gruppe bestehend aus offen-kettigen oder cyclischen oder polycyclischen Ethern oder Polyethern oder (Poly)etherpolyolen, Amin- und/oder alkylamin-funktionalisierten Alkoholen oder Polyolen oder (Poly)ether-polyolen, schwach basischen organischen Verbindungen, schwach sauren makroporösen Kationenaustauschern und schwach alkalischen makroporösen Kationenaustauschern,

M für ein Metall beliebig ausgewählt aus den Alkalimetallen, Magnesium, Calcium oder Aluminium steht,

X für ein Halogenid oder Alkoxid oder Alkylsulfid steht,

Y für Iod oder Brom oder Chlor steht,

n für 1, 2 oder 3 steht,

S1 für ein aprotisches organisches Lösungsmittel oder ein Gemisch aprotischer Lösungsmittel steht und

S2 für ein aprotisches organisches Lösungsmittel oder ein Gemisch aprotischer Lösungsmittel steht,

wobei sich die Lösungsmittel oder Lösungsmittelgemische S1 und S2 von dem Hilfsstoff L unterscheiden.

Dabei wird durch Zugabe von 3 Äquivalenten eines Halogen-Aromaten $R^4$-Y (III) zu den vorgelegten 3 Äquivalenten Metall *in situ* ein metallorganisches Reagenz erzeugt, das mit den vorgelegten Substanzen (I) und (II) zum $1/n\ K^{n+} R^4_3 B^- \text{-} R^1$ 1/n Organoborat (IV) reagiert. Überraschenderweise wurde gefunden, dass sich die Verwendung von substituierten Organokationen der Wertigkeit n auf Basis von Stickstoff, Phosphor, Sauerstoff, Schwefel und / oder Iod als Kationen vorteilhaft auf die Reaktion auswirkt, da sie chemisch inert sind und die Reaktion mit einer höheren RaumZeitausbeute als bekannte Verfahren verläuft und weniger Nebenreaktionen auftreten und damit die Ausbeute erhöht wird und eine höhere Reinheit des Zielproduktes sehr viel leichter zu erzielen ist.

Als weiterhin vorteilhaft erweist sich die Aufarbeitung des Reaktionsgemischs durch Zugabe eines Hilfsstoffs L, der mit den M-Salzen $MY(OR^2)$, $MY(OR^3)$ und MXY einen Komplex bildet, und gegebenenfalls eines weiteren Lösungsmittels oder Lösungsmittelgemischs S2. Durch geeignete Wahl von L und S1 und / oder S2 werden so schwer lösliche Komplexe erhalten, die durch geeignete Verfahren, wie beispielsweise Dekantieren, Filtration, Zentrifugation o.ä. einfach von dem gewünschten, in der organischen Phase gelösten Triaryl-organoboraten der Formel $1/n\ K^{n+} R^4_3 B^- \text{-} R^1$ (IV) abgetrennt werden können.

Die erfindungsgemäßen Triaryl organoborate gemäß Formel IV sind vorzugsweise Triaryl-alkylborate, Triarylcycloalkylborate, Triaryl-alkenylborate, Triaryl-alkinylborate und / oder Triaryl-aralkylborate, bevorzugter Triaryl-alkylborate und / oder Triaryl-cycloalkylborate.

Ebenfalls Gegenstand der Erfindung ist daher ein Verfahren zur Herstellung von Verbindungen 1/n $K^{n+} R^4_3 B^- \text{-} R^1$ $K^{n+}$ der Formel IV umfassend die Schritte

i) das Vorlegen von einem Äquivalenten Organo-Boronsäureester B-$R^1(OR^2)(OR^3)$ (I) mit 1/n Äquivalenten Salz $K^{n+}$ $nX^-$ (II) und 3 Äquivalenten Metall M in einem Lösungsmittel oder einem Lösungsmittelgemisch S1,
ii) die Zugabe von 3 Äquivalenten eines Halogen-Aromaten $R^4$-Y (III), wodurch
iii) ein *in situ* erzeugtes metallorganisches Reagenz mit den vorgelegten Substanzen (I) und (II) zum $1/n\ K^{n+} R^4_3 B^- \text{-} R^1$ (IV) reagiert,
iv) die Zugabe eines Hilfsstoffs L und
v) gegebenenfalls eines zweiten organischen Lösungsmittel S2, wobei die Verbindung 1/n $K^{n+} R^4_3 B^- \text{-} R^1$ (IV) in der organischen Phase verbleibt und
vi) die Metallsalze $MY(OR^2)$, $MY(OR^3)$ und MXY als ausgefallene Feststoff-Komplexe $MY(OR^2)L$, $MY(OR^3)L$ und MXYL abgetrennt werden.

Dabei genügt die Umsetzung dem folgenden Reaktionsschema:

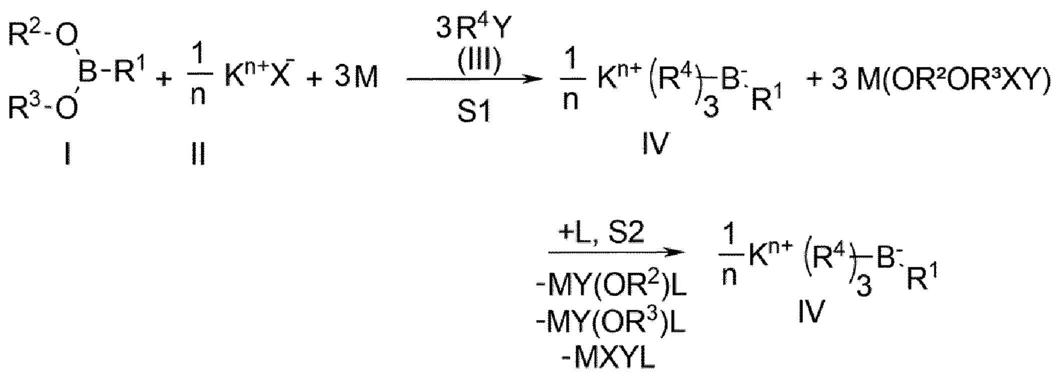


Bevorzugt steht $R^1$ für einen gegebenenfalls mit Hydroxy und / oder Alkoxy und / oder Acyloxy und oder Halogen substituierten $C_2$- bis $C_{18}$-Alkyl-, $C_3$- bis $C_{18}$-Alkenyl-, $C_3$- bis $C_{18}$-Alkinyl-, $C_5$- bis $C_6$-Cycloalkyl- oder $C_7$- bis $C_{13}$-Aralkylrest und besonders bevorzugt steht $R^1$ für einen gegebenenfalls mit Hydroxy und / oder Alkoxy und / oder Acyloxy und oder Halogen substituierten $C_4$- bis $C_{16}$-Alkyl-, $C_3$- bis $C_{16}$-Alkenyl-, $C_3$- bis $C_{16}$-Alkinyl-, Cyclohexyl- oder $C_7$- bis $C_{13}$-Aralkylrest.

Bevorzugt stehen $R^2$ und $R^3$ unabhängig voneinander für einen gegebenenfalls verzweigten $C_2$- bis $C_{18}$-Alkyl-Rest oder $R^2$ und $R^3$ bilden gemeinsam einen 4-7 gliedrigen gegebenenfalls substituierten Carbocylus und besonders bevorzugt stehen $R^2$ und $R^3$ unabhängig voneinander für einen gegebenenfalls verzweigten $C_3$- bis $C_{12}$-Alkyl-Rest oder $R^2$ und $R^3$ bilden gemeinsam einen 4-6 gliedrigen gegebenenfalls substituierten Carbocyclus.

Bevorzugt steht $R^4$ für einen $C_6$- bis $C_{10}$-Arylrest, der gegebenenfalls durch mindestens einen Rest der Auswahl Halogen, $C_1$-bis $C_4$-Alkyl, Trifluormethyl, $C_1$-bis $C_4$-Alkoxy, Trifluormethoxy, Phenyl und / oder Phenoxy substituiert ist und besonders bevorzugt steht $R^4$ für einen $C_6$-Arylrest, der gegebenenfalls durch mindestens einen Rest der Auswahl Halogen, $C_1$-bis $C_4$-Alkyl, Trifluormethyl, $C_1$-bis $C_4$-Alkoxy, Trifluormethoxy, Phenyl und / oder Phenoxy substituiert ist.

Unter einem beliebig substituierten Organokation K der Wertigkeit n auf Basis von Stickstoff werden beispielsweise Ammoniumionen, Pyridiniumionen, Pyridaziniumionen, Pyrimidiniumionen, Pyraziniumionen, Imidazoliumionen, 1H-Pyrazoliumionen, 3H-Pyrazoliumionen, 4H-Pyrazoliumionen, 1-Pyrazoliniumionen, 2-Pyrazoliniumionen, 3-Pyrazoliniumionen, Imidazoliniumionen, Thiazoliumionen, 1,2,4-Triazoliumionen, 1,2,3-Triazoliumionen, Pyrrolidiniumionen, Chinoliniumionen, die gegebenenfalls in einer oder mehreren Seitenketten weitere funktionelle Gruppen wie Ether, Ester, Amide und / oder Carbamate tragen und die auch in oligomerer oder polymerer oder verbrückender Form vorliegen können, verstanden. Bevorzugte Organokationen K der Wertigkeit n auf Basis von Stickstoff sind beispielsweise Ammoniumionen, Pyridiniumionen, Pyridaziniumionen, Pyrimidiniumionen, Pyraziniumionen, Imidazoliumionen, Pyrrolidiniumionen, die gegebenenfalls in einer oder mehreren Seitenketten weitere funktionelle Gruppen wie Ether, Ester, Amide und / oder Carbamate tragen und die auch in oligomerer oder polymerer oder verbrückender Form vorliegen können. Besonders bevorzugte Organokationen K der Wertigkeit n auf Basis von Stickstoff sind beispielsweise Ammoniumionen, Pyridiniumionen und Imidazoliumionen, die gegebenenfalls in einer oder mehreren Seitenketten weitere funktionelle

Gruppen wie Ether, Ester, Amide und / oder Carbamate tragen und die auch in oligomerer oder polymerer oder verbrückender Form vorliegen können. Auch polymere Kationen können unter den oben genannten Substitutionsmustern gemeint sein.

Unter einem beliebig substituierten Organokation K der Wertigkeit n auf Basis von Phosphor werden Phosphor(IV)-Verbindungen der Koordinationszahl 4 verstanden, wie beispielsweise beliebig substituierte Tetraalkyl-Phosphonium-, Trialkyl-Aryl-Phosphonium-, Dialkyl-Diaryl-Phosphonium-, Alkyl-Triaryl-Phosphonium- oder Tetraaryl-Phosphonium-Salze, die gegebenenfalls in einer oder mehreren Seitenketten weitere funktionelle Gruppen wie Ether, Ester, Carbonyle, Amide und / oder Carbamate tragen und die auch in oligomerer oder polymerer oder verbrückender Form vorliegen können, verstanden. Ebenfalls umfasst sind 5 bis 8-gliedrige aliphatische, quasi-aromatische oder aromatische cyclische Verbindungen, die 1, 2 oder 3 Phosphonium(IV)-Atome enthalten, die dann zur Herstellung der Vierbindigkeit des Phosphor-Atoms beliebig Alkyl- oder Aryl-substituiert sind. Aromatische Reste können zusätzlich beliebig viele Halogenatome, Nitro-, Cyano-, Trifluormethyl-, Ester-, und / oder Ether-Substituenten tragen. Selbstverständlich können die Alkyl- und Aryl-Reste untereinander durch Kohlenstoff- oder mono und / oder Polyether-Ketten verbrückt vorliegen, die dann mono- oder polycyclische Strukturen ausbilden. Bevorzugte Organokationen K der Wertigkeit n auf Basis von Phosphor sind beispielsweise beliebig substituierte Tetraalkyl-Phosphonium-, Trialkyl-Aryl-Phosphonium-, Dialkyl-Diaryl-Phosphonium-, Alkyl-Triaryl-Phosphonium- oder Tetraaryl-Phosphonium-Salze, die gegebenenfalls in einer oder mehreren Seitenketten weitere funktionelle Gruppen wie Carbonyle, Amide und / oder Carbamate tragen und die auch in oligomerer oder polymerer oder verbrückender Form vorliegen können, verstanden. Aromatische Reste können zusätzlich beliebig viele Halogenatome, Ester-, und / oder Ether-Substituenten tragen. Selbstverständlich können die Alkyl- und Aryl-Reste untereinander durch Kohlenstoff- oder mono und / oder Polyether-Ketten verbrückt vorliegen, die dann mono- oder polycyclische Strukturen ausbilden. Besonders bevorzugte Organokationen K der Wertigkeit n auf Basis von Phosphor sind beispielsweise beliebig substituierte Tetraalkyl-Phosphonium-, Trialkyl-Aryl-Phosphonium-, Dialkyl-Diaryl-Phosphonium-, Alkyl-Triaryl-Phosphonium- oder Tetraaryl-Phosphonium-Salze, die gegebenenfalls in einer oder mehreren Seitenketten weitere funktionelle Gruppen wie Carbonyle, Amide und / oder Carbamate tragen und die auch in oligomerer oder polymerer oder verbrückender Form vorliegen können, verstanden. Aromatische Reste können zusätzlich beliebig viele Halogenatome tragen. Selbstverständlich können die Alkyl- und Aryl-Reste untereinander durch Kohlenstoff- oder mono und / oder Polyether-Ketten verbrückt vorliegen, die dann mono- oder polycyclische Strukturen ausbilden. Auch polymere Kationen wie beispielsweise in US 3,125,555 genannt können unter den oben genannten Substitutionsmustern gemeint sein.

Unter einem beliebig substituierten Organokation K der Wertigkeit n auf Basis von Sauerstoff werden beispielsweise beliebig substituiertes Pyrylium, auch in annellierter Form wie im Benzopyrylium, Flavylium oder Naphthoxanthenium verstanden. Bevorzugte Organokationen K der Wertigkeit n auf Basis von Sauerstoff sind beispielsweise beliebig substituiertes Pyrylium, auch in annellierter Form wie im Benzopyrylium, Flavylium. Auch polymere Kationen können unter den oben genannten Substitutionsmustern gemeint sein.

Unter einem beliebig substituierten Organokation K der Wertigkeit n auf Basis von Schwefel werden Onium-Verbindungen des Schwefels verstanden, die gleiche oder verschiedene gegebenenfalls substituierte $C_1$- bis $C_{22}$-Alkyl-, $C_6$- bis $C_{14}$-Aryl-, $C_7$- bis $C_{15}$-Arylalkyl- oder $C_5$- bis $C_7$-Cycloalkylreste tragen und / oder oligomere oder polymere wiederkehrende verbindende Einheiten zu Sulfoniumsalzen mit $1 \leq n \leq 3$ aufbaut. Ebenfalls umfasst sind 5 bis 8-gliedrige aliphatische, quasi-aromatische oder aromatische cyclische Verbindungen, die 1 oder 2 Sulfonium(III)-Atome enthalten, die dann zur Herstellung der Dreibindigkeit des Schwefel-Atoms beliebig Alkyl- oder Aryl-substituiert sind. Bevorzugte Organokationen K der Wertigkeit n auf Basis von Schwefel sind Onium-Verbindungen des Schwefels, die gleiche oder verschiedene gegebenenfalls substituierte $C_4$- bis $C_{14}$-Alkyl-, $C_6$- bis $C_{10}$-Aryl-, $C_7$- bis $C_{12}$-Arylalkyl- oder $C_5$- bis $C_6$-Cycloalkylreste tragen und / oder oligomere oder polymere wiederkehrende verbindende Einheiten zu Sulfoniumsalzen mit $1 \leq n \leq 3$ aufbauen sowie Thiopyrylium. Besonders bevorzugte Organokationen K der Wertigkeit n auf Basis von Schwefel sind Onium-Verbindungen des Schwefels, die gleiche oder verschiedene gegebenenfalls substituierte $C_6$- bis $C_{12}$-Alkyl-, $C_6$- bis $C_{10}$-Aryl-, $C_7$- bis $C_{12}$-Arylalkyl- oder $C_5$- bis $C_6$-Cycloalkylreste tragen und / oder oligomere oder polymere wiederkehrende verbindende Einheiten zu Sulfoniumsalzen mit $1 \leq n \leq 3$ aufbauen sowie Thiopyrylium. Auch polymere Kationen können unter den oben genannten Substitutionsmustern gemeint sein.

Unter einem beliebig substituierten Organokation K der Wertigkeit n auf Basis von Iod werden Onium-Verbindungen des Iods verstanden, die gleiche oder verschiedene gegebenenfalls substituierte $C_1$- bis $C_{22}$-Alkyl-, $C_6$- bis $C_{14}$-Aryl-, $C_7$- bis $C_{15}$-Arylalkyl- oder $C_5$- bis $C_7$-Cycloalkylreste tragen und / oder oligomere oder polymere wiederkehrende verbindende Einheiten zu Iodoniumsalzen mit $1 \leq n \leq 3$ aufbauen. Bevorzugte Organokationen K der Wertigkeit n auf Basis von Iod sind Onium-Verbindungen des Iods, die gleiche oder verschiedene gegebenenfalls substituierte $C_4$- bis $C_{14}$-Alkyl-, $C_6$- bis $C_{10}$-Aryl-, $C_7$- bis $C_{12}$-Arylalkyl- oder $C_5$- bis $C_6$-Cycloalkylreste tragen und / oder oligomere oder polymere wiederkehrende verbindende Einheiten zu Iodoniumsalzen mit $1 \leq n \leq 3$ aufbauen. Besonders bevorzugte Organokationen K der Wertigkeit n auf Basis von Iod sind Onium-Verbindungen des Iods, die gleiche oder verschiedene gegebenenfalls substituierte $C_4$- bis $C_{12}$-Alkyl-, $C_6$- bis $C_{10}$-Aryl-, $C_7$- bis $C_{12}$-Arylalkyl- oder $C_5$- bis $C_6$-Cycloalkylreste tragen und / oder oligomere oder polymere wiederkehrende verbindende Einheiten zu Iodoniumsalzen mit $1 \leq n \leq 3$

aufbauen. Auch polymere Kationen können unter den oben genannten Substitutionsmustern gemeint sein.

Unter einem mit Metall-Salzen $MY(OR^2)$, $MY(OR^3)$ und MXY im Lösungsmittel oder Lösungsmittelgemisch S1 und / oder S2 schwer-löslichen Komplex bildenden Hilfsstoff L werden Lewis-basische Verbindungen verstanden, die in flüssiger oder fester oder polymer angebundener Form vorliegen können (Begriff Lewis-Base gemäß S. 1136 in Pure & Appl. Chem., 66 (5), 1077-1184, 1994). Dieser Hilfsstoff wird an Stelle der sonst üblichen wässrigen Aufarbeitung nach Ende der Reaktion zugegeben und bildet mit den Metall-Salzen $MY(OR^2)$, $MY(OR^3)$ und MXY einen durch Filtration abtrennbaren Feststoff. Beispiele für solche Hilfsstoffe L sind offen-kettige oder cyclische oder polycyclische Ether oder Polyether oder (Poly)ether-polyole wie 1,4-Dioxan oder 1,2-Ethandiol. Ebenfalls können Amin- und / oder alkylamin-funktionalisierte Alkohole, Polyole oder (Poly)ether-polyole wie Aminoethanol, Methylaminoethanol oder Dimethylaminoethanol verwendet werden. Auch schwach saure oder schwach alkalische makroporöse Kationenaustauscher, die u.a. selektiv für das Komplexieren von Erdalkaliionen entwickelt wurden, können verwendet werden. Darüber hinaus können schwach basische organische Verbindungen wie Pyridin, 2,6-Dimethylpyridin, Triethylamin, Diisopropylethylamin oder koordinierende Verbindungen wie Triphenylphosphin, Tri(o-tolyl)phosphin eingesetzt werden. Selbstverständlich können auch Gemische von Hilfsstoffen verwendet werden. Die Hilfsstoffe können darüber hinaus in substöchiometrischem, stöchiometrischem oder suprastöchiometrischem Verhältnis zugesetzt werden. Die Einwirkzeit des Hilfsstoffes kann beliebig lange, bevorzugt <24 h, besonders bevorzugt <12 h, insbesondere bevorzugt < 1 h und hervorragend bevorzugt <0.1 h sein. In einer bevorzugten Ausführungsform ist der Hilfsstoff L eine Lewis-basische Verbindung ausgewählt aus der Gruppe bestehend aus offen-kettigen oder cyclischen oder polycyclischen Ethern oder Polyethern oder (Poly)ether-polyolen, Amin- und/oder alkylamin-funktionalisierten Alkoholen oder Polyolen oder (Poly)ether-polyolen, schwach basischen organischen Verbindungen, schwach sauren makroporösen Kationenaustauschern und schwach alkalischen makroporösen Kationenaustauschern.Es handelt es sich bei dem Hilfsstoff L um Lewis-basische Verbindungen mit mindestens einer frei zur Verfügung stehenden Koordinationsstelle oder deren Gemische, vorzugsweise ausgewählt aus der Gruppe bestehend aus offen-kettigen oder cyclischen oder polycyclischen Ethern oder Polyethern oder (Poly)ether-polyolen, Amin- und/oder alkylamin-funktionalisierten Alkoholen oder Polyolen oder (Poly)ether-polyolen, schwach basischen organischen Verbindungen, schwach sauren makroporösen Kationenaustauschern und schwach alkalischen makroporösen Kationenaustauschern, bevorzugter ausgewählt aus der Gruppe bestehend aus cyclischen Ethern oder Polyethern oder (Poly)ether-polyolen, schwach sauren makroporösen Kationenaustauschern und schwach alkalischen makroporösen Kationenaustauschern, noch bevorzugter ausgewählt aus der Gruppe bestehend aus cyclischen Ethern, schwach sauren makroporösen Kationenaustauschern und schwach alkalischen makroporösen Kationenaustauschern.

Unter einem aprotischen organischen Lösungsmittel oder einem Gemisch aprotischer organischer Lösungsmittel S1 werden nicht ohne starke Base deprotonierbare Lösungsmittel verstanden (Reichardt, C., Solvents and Solvent Effects in Organic Chemistry, 3. Aufl.; Wiley-VCH: Weinheim, (2003)). Beispiele für aprotische organische Lösungsmittel S1 sind Alkane, Alkene, Alkine, Benzol und Aromaten mit aliphatischen und / oder aromatischen Substituenten, Carbonsäureester und / oder Ether. Bevorzugte aprotische organische Lösungsmittel S1 sind Alkane, Aromaten mit aliphatischen und / oder aromatischen Substituenten und / oder Ether. Verwendet werden z.B. aromatische Kohlenwasserstoffe wie Solvent-Naphtha, Toluol oder Xylol oder Ether wie Tetrahydrofuran, Methyltetrahydrofuran, Diethylether oder Dimethoxyethan. Das Lösungsmittel sollte möglichst wasserfrei sein. In einer bevorzugten Ausführungsform unterscheidet sich das Lösungsmittel oder Lösungsmittelgemische S1 von dem Hilfsstoff L, d.h. es handelt sich bei S1 und L nicht um dieselbe Substanz.

Unter einem aprotischen organischen Lösungsmittel oder einem Gemisch aprotischer organischer Lösungsmittel S2 werden nicht ohne starke Base deprotonierbare Lösungsmittel verstanden (Reichardt, C., Solvents and Solvent Effects in Organic Chemistry, 3. Aufl.; Wiley-VCH: Weinheim, (2003)). Beispiele für aprotische organische Lösungsmittel S2 sind Alkane, Alkene, Alkine, Benzol und Aromaten mit aliphatischen und / oder aromatischen Substituenten, Carbonsäureester und / oder Ether. Bevorzugte aprotische organische Lösungsmittel S2 sind Alkane, Aromaten mit aliphatischen und / oder aromatischen Substituenten und / oder Carbonsäureester. Verwendet werden z.B. aromatische Kohlenwasserstoffe wie Solvent-Naphtha, Toluol oder Xylol oder Ester wie Methylacetat, Ethylacetat, Butylacetat, Methoxypropylacetat oder Propylenglycoldiacetat. Das Lösungsmittel oder Lösungsmittelgemische S2 unterscheiden sich von dem Hilfsstoff L, d.h. es handelt sich bei S2 und L nicht um dieselbe Substanz.

Bevorzugt steht M für Magnesium, Calcium oder Aluminium und besonders bevorzugt steht M für Magnesium.

Bevorzugt steht X für ein Halogenid oder Alkoxid, besonders bevorzugt steht X für ein Halogenid und insbesondere bevorzugt steht X für Chlorid oder Bromid.

Bevorzugt steht Y für Brom oder Chlor und besonders bevorzugt steht Y für Brom.

Bevorzugt steht n für 1 oder 2 und besonders bevorzugt steht n für 1.

$C_1$-$C_{22}$-Alkyl steht im Rahmen der Erfindung beispielsweise für Methyl, Ethyl, n-Propyl, isoPropyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, 1,1-Dimethylpropyl, 1,2-Dimethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl,

1,1,2-Trimethylpropyl, 1,2,2-Trimethylpropyl, 1-Ethyl-1-methylpropyl, 1-Ethyl-2-methylpropyl oder 1-Ethyl-2-methylpropyl, n-Heptyl, n-Octyl, 2-Ethylhexyl, Pinakyl, n-Nonyl, n-Decyl, n-Dodecyl, n-Tridecyl, n-Tetradecyl, n-Hexadecyl, n-Octadecyl, n-Eicosyl, n-Docosyl. Gleiches gilt für den entsprechenden Alkylrest beispielsweise in Aralkyl- bzw. Alkylaryl-, Alkylphenyl- oder Alkylcarbonylresten.

Alkylreste, Alkenylreste oder Alkinylreste in den entsprechenden Hydroxyalkyl- oder Aralkyl- bzw. Alkylarylresten stehen beispielsweise für die den vorangehenden Alkylresten entsprechenden Alkylreste, Alkenylreste oder Alkinylreste.

Beispiele sind 2-Chlorethyl, Benzyl, Allyl, 2-Buten-1-yl, Propargyl.

Cycloalkyl steht im Rahmen der Erfindung beispielsweise für Cyclopropyl, Cyclobutyl, Cyclopentyl, Cyclohexyl, Cycloheptyl, Adamantyl oder die isomeren Menthyle.

Aryl steht für einen carbocyclischen aromatischen Rest mit 6 bis 14 Gerüstkohlenstoffatomen. Gleiches gilt für den aromatischen Teil eines Arylalkylrestes, auch Aralkylrest genannt, sowie für Arylbestandteile komplexerer Gruppen, wie z.B. Arylcarbonylresten.

Alkylen steht für ein Diradikal eines gesättigten, verzweigtem oder unverzweigtem Kohlenwasserstoffs, wie beispielsweise Methylen ($-CH_2-$), Ethylen ($-CH_2CH_2-$), Propylen ($-CH_2CH_2CH_2-$) usw..

Cyclopentylen steht für ein Diradikal eines gegebenenfalls Alkyl-substituierten Cyclopentan-Rings.

Cyclohexylen steht für ein Diradikal eines gegebenenfalls Alkyl-substituierten Cyclohexan-Rings.

Cycloheptylen steht für ein Diradikal eines gegebenenfalls Alkyl-substituierten Cycloheptan-Rings.

Aryldialkylen steht für ein Diradikal eines gesättigten, verzweigtem oder unverzweigtem Alkylarylalkyls, wobei der Arylring gegebenenfalls mit Alkyl, Aryl, Halogen substituiert sein kann, wie beispielsweise Xylylen (1,2- oder 1,3- oder 1,4-Phenylenbis(methylen)).

Arylen steht für ein Diradikal eines Aryls.

Heteroarylen steht für ein Diradikal eines Heteroaryls.

Alkylen-Cycloalkylen steht für ein für ein Diradikal eines Alkylcycloalkans, welches auf der einen Seite über die Alkylgruppe an einen ersten Rest gebunden ist und über die Cycloalkylgruppe an einen zweiten Rest gebunden ist.

Alkandicycloalkylen steht für ein Diradikal eines Dicycloalkylalkan wie beispielsweise Methylen-bis(cyclohexan-4,1-diyl).

Alkandiarylen steht für ein Diradikal eines Diarylalkans wie beispielsweise 4-Methylenbis(4,1-phenylen).

Beispiele für $C_6$- bis $C_{14}$-Aryl sind Phenyl, o-, p-, m-Tolyl, o-, p-, m-Ethylphenyl, Naphthyl, Phenanthrenyl, Anthracenyl, Fluorenyl, o-, p-, m-Fluorphenyl, o-, p-, m-Chlorphenyl, o-, p-, m-Methoxyphenyl, o-, p-, m-Trifluormethylphenyl, o-, p-, m-Trifluormethoxyphenyl, o-, m- oder p-Biphenylyl, o-, p-, m-Phenoxyphenyl, 3,4-Dimethylphenyl, 3,4-Dichlorphenyl, 3,4-Difluorphenyl, 3,4-Dimethoxyphenyl, 4-Methyl-3-fluorphenyl, 4-Methyl-3-chlorphenyl, 3,4,5-Trifluorphenyl.

Arylalkyl bzw. Aralkyl bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert sein kann.

Beispiele sind Benzyl, 4-Chlorbenzyl, Phenethyl, 2-Phenyl-1-propyl, 3-Phenyl-1-propyl, 1-Phenyl-2-propyl, Diphenylmethyl.

Beispiele für eine 2-8 gliedrige gegebenenfalls durch Alkyl substituierte und / oder durch Sauerstoff-Atome unterbrochene Kohlenstoff-Brücke sind $-CH_2-CH_2-$, $-CH_2-CH_2-CH_2-$, $-CH_2-CH_2-CH_2-CH_2-$, $-CH_2-CH_2-O-CH_2-CH_2-$, $-CH_2-CH(CH_3)-$.

Beispiele für einen 4-14 gliedrigen gegebenenfalls durch Alkyl- oder Aryl- substituierten und / oder durch Sauerstoff-Atome unterbrochenen Kohlenstoff-Tricyclus sind:

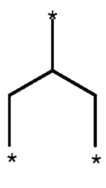 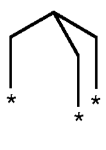 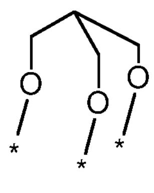 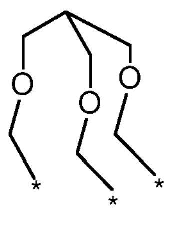 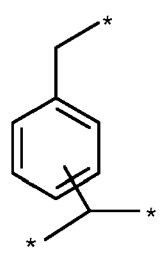 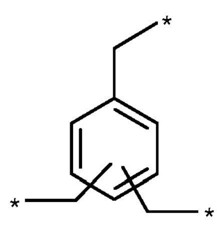

Die Umsetzung wird im allgemeinen ohne Anwendung von Überdruck ausgeführt. Praktisch wird man dann also bei mindestens dem Eigendruck arbeiten.

Die Umsetzung wird im allgemeinen bei Temperaturen von -20 °C bis 100 °C, bevorzugt von -10 °C bis 80 °C, besonders bevorzugt von 0 °C bis 70 °C und insbesondere bevorzugt von 10 °C bis 65 °C durchgeführt.

Die Umsetzung wird praktischerweise durch Zugabe von maximal 10 % der Gesamtmenge der Verbindung

(III) gestartet und die Verbindung (III) wird anschließend als 10-90 %-ige, bevorzugt 15-75%-ige besonders bevorzugt als 20-50 %-ige Lösung im Lösungsmittel S1 zugegeben, wobei sich die Temperatur T im oben beschriebenen Rahmen bewegt und durch gleichmäßige Zugabe der Verbindung (III) die Reaktion kontrolliert exotherm gehalten wird. Zum Anspringen der Reaktion können darüber hinaus optional die dem Fachmann bekannten Verbindungen wie Dibromethan oder Iod verwendet werden oder aber die Metalloberfläche M kann durch Ultraschall aktiviert werden.

Es ist von Vorteil, während der gesamten Dauer der Reaktion, z.B. durch Rühren, für eine gute Durchmischung des Ansatzes zu sorgen sowie durch Wahl eines geeigneten Lösungsmittels den Start der Reaktion gut zu ermöglichen.

Durch das erfindungsgemäße Verfahren werden deutlich bessere Ausbeuten im Vergleich zu den bekannten Verfahren erhalten, beispielsweise mindestens anderthalb mal so hohe Ausbeuten.

Auch offenbart sind die gemäß dem erfindungsgemäßen Verfahren herstellbaren oder hergestellten Triarylorganoborate. Die so erhältlichen oder erhaltenen Triarylorganoborate enthalten zudem bevorzugt < 10.000 ppm eines Tetraarylborates $R^3_4B^-$, besonders bevorzugt < 5.000 ppm eines Tetraarylborates $R^3_4B^-$ und insbesondere bevorzugt < 1.000 ppm eines Tetraarylborates $R^3_4B^-$. Die Einheit ppm bezieht sich auf die Gewichtsanteile. Die Anwesenheit von Tetraarylboraten $R^3_4B^-$ führt bei längeren Lagerung zu einer eingeschränkten Beschreibbarkeit unbelichteter Photopolymerfilme enthaltend die Triaryl-organoborate bzw. Verlust der holographischen Eigenschaften in belichteten Photopolymerfilmen.

Auch offenbart sind Verbindungen der Formel (C), wobei die Boratkomponente optional nach dem erfindungsgemäßen Verfahren hergestellt werden kann oder hergestellt wird

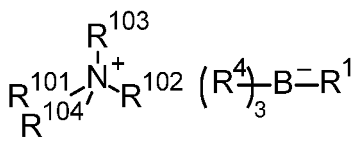

(C)

worin

$R^{101}$ für einen gegebenenfalls verzweigten $C_{14}$- bis $C_{22}$-Alkyl-Rest steht oder zusätzlich die Bedeutung von $R^{102}$ hat unter der Voraussetzung, dass $T^{101}$ und $R^{131}$ beziehungsweise $T^{102}$ und $R^{132}$jeweils zusammen mindestens 12 C-Atome enthalten,

$R^{102}$ für einen Rest der Formeln

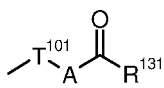

(CI) ,

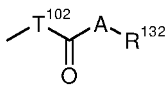

(CII)

steht,

$T^{101}$ für eine Brücke mit 2 bis 16 C-Atomen steht, von denen maximal ein Drittel durch O und / oder $NR^{200}$ ersetzt sein kann, wobei zwischen zwei O bzw. $NR^{200}$ mindestens 2 C-Atome stehen müssen, und die in Form einer gegebenenfalls verzweigten Kette und / oder einem fünf- oder sechsgliedrigen Ring angeordnet sein können,

$R^{131}$ für einen gegebenenfalls verzweigten und / oder gegebenenfalls substituierten $C_4$-bis $C_{22}$-Alkyl-Rest, einen gegebenenfalls verzweigten und / oder gegebenenfalls substituierten $C_4$- bis $C_{22}$-Alkenyl-Rest, einen Cyclopentyl-, Cyclohexyl- oder Cycloheptyl-Rest, einen $C_7$- bis $C_{10}$-Aralkyl-Rest, einen gegebenenfalls durch nichtionische Reste substituierten Phenyl-Rest oder heterocyclischen Rest, einen gegebenenfalls verzweigten und / oder gegebenenfalls substituierten $C_4$- bis $C_{22}$-Alkoxy-Rest, einen Cyclopentoxy-, Cyclohexoxy- oder Cycloheptoxy-Rest, einen $C_7$- bis $C_{10}$-Aralkoxy-Rest, einen gegebenenfalls durch nichtionische Reste substituierten Phenoxy-Rest oder Heteroaryloxy-Rest, einen gegebenenfalls verzweigten und / oder gegebenenfalls substituierten $C_4$- bis $C_{22}$-Alkylamino-Rest, einen Cyclopentylamino-, Cyclohexylamino- oder Cycloheptylamino-Rest, einen $C_7$- bis $C_{10}$-Aralkylamino-Rest, einen gegebenenfalls durch nichtionische Reste substituierten Phenylamino-Rest oder

Heteroarylamino-Rest steht,

$T^{102}$ für eine Brücke mit 1 bis 16 C-Atomen steht, die in Form einer gegebenenfalls verzweigten Kette und / oder einem fünf- oder sechsgliedrigen Ring angeordnet sein können,

$R^{132}$ für einen gegebenenfalls verzweigten und / oder gegebenenfalls substituierten $C_1$-bis $C_{22}$-Alkyl-Rest, einen Cyclopentyl-, Cyclohexyl- oder Cycloheptyl-Rest, einen $C_7$- bis $C_{10}$-Aralkyl-Rest, einen gegebenenfalls durch nichtionische Reste substituierten Phenyl-Rest oder heterocyclischen Rest steht,

A für $NR^{201}$ oder Sauerstoff steht,

$R^{200}$ und $R^{201}$ unabhängig voneinander für Wasserstoff oder $C_1$- bis $C_4$-Alkyl stehen,

$R^{103}$ und $R^{104}$ unabhängig voneinander für einen gegebenenfalls verzweigten und / oder gegebenenfalls substituierten $C_1$- bis $C_5$-Alkyl-Rest stehen

oder

$R^{102}$ für einen Rest der Formeln

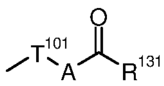 (CI),

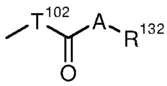 (CII)

steht,

$T^{101}$, $R^{131}$, $T^{102}$, $R^{132}$, $R^{200}$, $R^{201}$ und A die oben genannte Bedeutung haben unter der Voraussetzung, dass $T^{101}$ und $R^{131}$ beziehungsweise $T^{102}$ und $R^{132}$ jeweils zusammen mindestens 12 C-Atome enthalten,

$R^{101}$, $R^{103}$ und $R^{104}$ gemeinsam mit dem $N^+$-Atom einen Imidazol- oder Pyridin-Ring bilden, der mindestens durch einen Rest der Auswahl $C_1$- bis $C_8$-Alkyl, $C_1$- bis $C_8$-Alkoxy, $C_5$- bis $C_7$-Cycloalkyl, Benzyl oder Phenyl substituiert ist

oder

$R^{101}$ für einen gegebenenfalls verzweigten $C_{14}$- bis $C_{22}$-Alkyl-Rest steht oder zusätzlich die Bedeutung von $R^{102}$ hat unter der Voraussetzung, dass $T^{101}$ und $R^{131}$ beziehungsweise $T^{102}$ und $R^{132}$ jeweils zusammen mindestens 12 C-Atome enthalten,

$R^{102}$ für einen Rest der Formeln

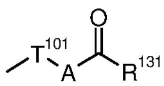 (CI),

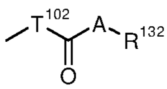 (CII),

steht und $T^{101}$, $R^{131}$, $T^{102}$, $R^{132}$, $R^{200}$, $R^{201}$ und A die oben genannte Bedeutung haben,

$R^{103}$ und $R^{104}$ gemeinsam eine $-(CH_2)_4-$, $-(CH_2)_5-$ oder $-(CH_2)_2-O-(CH_2)_2-$Brücke bilden und

$R^1$ und $R^4$ die oben genannte Bedeutung haben.

Auch offenbart sind Verbindungen der Formel (C), worin

$R^{101}$ für einen gegebenenfalls verzweigten $C_{14}$- bis $C_{22}$-Alkyl-Rest steht oder zusätzlich die Bedeutung von $R^{102}$ hat unter der Voraussetzung, dass $T^{101}$ und $R^{131}$ beziehungsweise $T^{102}$ und $R^{132}$ jeweils zusammen mindestens 12 C-Atome enthalten,

$R^{102}$ für einen Rest der Formeln

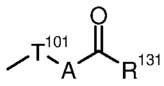 (CI) , 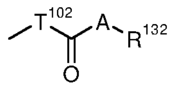 (CII)

steht,

$T^{101}$ für eine Brücke mit 2 bis 9 C-Atomen steht, von denen maximal ein Drittel durch O und / oder $NR^{200}$ ersetzt sein kann, wobei zwischen zwei O bzw. $NR^{200}$ mindestens 2 C-Atome stehen müssen, und die in Form einer gegebenenfalls verzweigten Kette und / oder einem fünf- oder sechsgliedrigen Ring angeordnet sein können,

$R^{131}$ für einen gegebenenfalls verzweigten und / oder gegebenenfalls substituierten $C_4$-bis $C_{16}$-Alkyl-Rest, einen gegebenenfalls verzweigten und / oder gegebenenfalls substituierten $C_4$- bis $C_{16}$-Alkenyl-Rest, einen Cyclopentyl- oder Cyclohexyl-Rest, einen Benzyl-, Phenethyl- oder Phenylpropyl-Rest, einen gegebenenfalls durch nichtionische Reste substituierten Phenyl-Rest oder heterocyclischen Rest, einen gegebenenfalls verzweigten und / oder gegebenenfalls substituierten $C_4$- bis $C_{16}$-Alkoxy-Rest, einen Cyclopentoxy- oder Cyclohexoxy-Rest, einen Benzyloxy-, Phenethyloxy- oder Phenylpropoxy-Rest, einen gegebenenfalls durch nichtionische Reste substituierten Phenoxy-Rest oder Heteroaryloxy-Rest, einen gegebenenfalls verzweigten und / oder gegebenenfalls substituierten $C_4$- bis $C_{22}$-Alkylamino-Rest, einen Cyclopentylamino- oder Cyclohexylamino-Rest, einen Benzylamino-, Phenethyamino- oder Phenylpropyl-amino-Rest, einen gegebenenfalls durch nichtionische Reste substituierten Phenylamino-Rest oder Heteroarylamino-Rest steht,

$T^{102}$ für eine Brücke mit 1 bis 9 C-Atomen steht, die in Form einer gegebenenfalls verzweigten Kette und / oder einem fünf- oder sechsgliedrigen Ring angeordnet sein können,

$R^{132}$ für einen gegebenenfalls verzweigten und / oder gegebenenfalls substituierten $C_1$-bis $C_{16}$-Alkyl-Rest, einen Cyclopentyl- oder Cyclohexyl-Rest, einen Benzyl-, Phenethyl- oder Phenylpropyl-Rest, einen gegebenenfalls durch nichtionische Reste substituierten Phenyl-Rest oder heterocyclischen Rest steht,

A für $NR^{201}$ oder Sauerstoff steht,

$R^{200}$ und $R^{201}$ unabhängig voneinander für Wasserstoff, Methyl oder Ethyl stehen,

$R^{103}$ und $R^{104}$ unabhängig voneinander für einen gegebenenfalls verzweigten und / oder gegebenenfalls substituierten $C_1$- bis $C_5$-Alkyl-Rest stehen

oder

$R^{102}$ für einen Rest der Formeln

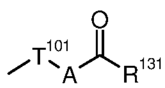 (CI), 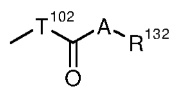 (CII)

steht,

$T^{101}$, $R^{131}$, $T^{102}$, $R^{132}$, $R^{200}$, $R^{201}$ und A die oben genannte Bedeutung haben unter der Voraussetzung, dass $T^{101}$ und $R^{131}$ beziehungsweise $T^{102}$ und $R^{132}$ jeweils zusammen mindestens 12 C-Atome enthalten,

$R^{101}$, $R^{103}$ und $R^{104}$ gemeinsam mit dem $N^+$-Atom einen Imidazol- oder Pyridin-Ring bilden, der mindestens durch einen Rest der Auswahl $C_1$- bis $C_6$-Alkyl, $C_1$- bis $C_6$-Alkoxy, Cyclopentyl, Cyclohexyl, Benzyl oder Phenyl substituiert ist

oder

$R^{101}$ für einen gegebenenfalls verzweigten $C_{14}$- bis $C_{22}$-Alkyl-Rest steht oder zusätzlich die Bedeutung von $R^{102}$ hat unter der Voraussetzung, dass $T^{101}$ und $R^{131}$ beziehungsweise $T^{102}$ und $R^{132}$jeweils zusammen mindestens 12 C-Atome enthalten,

$R^{102}$ für einen Rest der Formeln

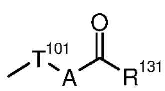 (CI), 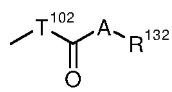 (CII),

steht und $T^{101}$, $R^{131}$, $T^{102}$, $R^{132}$, $R^{200}$, $R^{201}$ und A die oben genannte Be-

deutung haben,

R103 und R104 gemeinsam eine $-(CH_2)_4-$, $-(CH_2)_5-$ oder $-(CH_2)_2-O-(CH_2)_2-$Brücke bilden und

R1 und R4 die oben genannte Bedeutung haben.

Auch offenbart sind Verbindungen der Formel (C), worin

$R^{101}$ für einen gegebenenfalls verzweigten $C_{14}$- bis $C_{22}$-Alkyl-Rest steht,

$R^{102}$ für einen Rest der Formeln

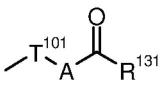 (CI) , 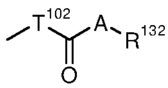 (CII)

steht,

$T^{101}$ für eine Brücke in Form einer gegebenenfalls verzweigten Kette mit 2 bis 8 C-Atomen steht, die 1 oder 2 O-Atome enthalten kann, wobei zwischen zwei O-Atomen mindestens 2 C-Atome stehen müssen, oder für eine Brücke der Formeln

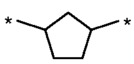 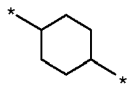 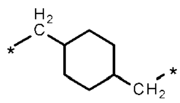 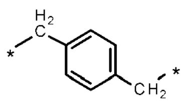

steht,

$R^{131}$ für einen gegebenenfalls verzweigten und / oder gegebenenfalls substituierten $C_4$-bis $C_{16}$-Alkyl-Rest, einen Cyclopentyl- oder Cyclohexyl-Rest, einen Benzyl-Rest, einen gegebenenfalls durch nichtionische Reste substituierten Phenyl-Rest oder einen Furyl-, Thienyl- oder Pyridyl-Rest, einen gegebenenfalls verzweigten und / oder gegebenenfalls substituierten $C_4$- bis $C_{16}$-Alkoxy-Rest, einen Cyclopentoxy- oder Cyclohexoxy-Rest, einen Benzyloxy-Rest, einen gegebenenfalls durch nichtionische Reste substituierten Phenoxy-Rest, einen gegebenenfalls verzweigten und / oder gegebenenfalls substituierten $C_4$- bis $C_{22}$-Alkylamino-Rest, einen Cyclopentylamino- oder Cyclohexylamino-Rest, einen Benzylamino-Rest, einen gegebenenfalls durch nichtionische Reste substituierten Phenylamino-Rest oder Pyridylamino-Rest steht,

$T^{102}$ für eine Brücke in Form einer gegebenenfalls verzweigten Kette mit 2 bis 8 C-Atomen steht oder für eine Brücke der Formeln

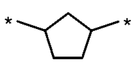 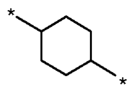 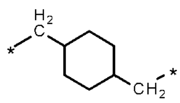 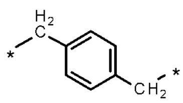

steht,

$R^{132}$ für einen gegebenenfalls verzweigten und / oder gegebenenfalls substituierten $C_1$-bis $C_{16}$-Alkyl-Rest, einen Cyclopentyl- oder Cyclohexyl-Rest, einen Benzyl-Rest, einen gegebenenfalls durch nichtionische Reste substituierten Phenyl-Rest oder Pyridyl-Rest steht,

A für $NR^{201}$ oder Sauerstoff steht,

$R^{201}$ für Wasserstoff oder Methyl steht,

$R^{103}$ und $R^{104}$ unabhängig voneinander für einen gegebenenfalls verzweigten und / oder gegebenenfalls substituierten $C_1$- bis $C_5$-Alkyl-Rest stehen

oder

$R^{102}$ für einen Rest der Formeln

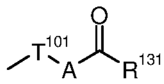 (CI), 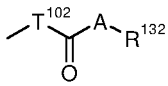 (CII)

steht,

$T^{101}$, $R^{131}$, $T^{102}$, $R^{132}$, $R^{201}$ und A die oben genannte Bedeutung haben unter der Voraussetzung, dass $T^{101}$ und $R^{131}$ beziehungsweise $T^{102}$ und $R^{132}$ jeweils zusammen mindestens 12 C-Atome enthalten,

$R^{101}$, $R^{103}$ und $R^{104}$ gemeinsam mit dem $N^+$-Atom einen Imidazol- oder Pyridin-Ring bilden, der mindestens durch einen Rest der Auswahl $C_1$- bis $C_4$-Alkyl, $C_1$- bis $C_4$-Alkoxy, Cyclopentyl, Cyclohexyl, Benzyl oder Phenyl substituiert ist

oder

$R^{101}$ für einen gegebenenfalls verzweigten $C_{14}$- bis $C_{22}$-Alkyl-Rest steht,

$R^{102}$ für einen Rest der Formeln

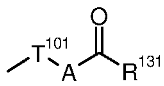 (CI), 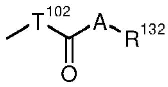 (CII),

steht und $T^{101}$, $R^{131}$, $T^{102}$, $R^{132}$, $R^{201}$ und A die oben genannte Bedeutung haben,

$R^{103}$ und $R^{104}$ gemeinsam eine $-(CH_2)_4-$, $-(CH_2)_5-$ oder $-(CH_2)_2-O-(CH_2)_2-$Brücke bilden und

$R^1$ und $R^4$ die oben genannte Bedeutung haben.

Unter $R^{131}$ und $R^{132}$ sind auch solche Reste zu verstehen, die über zwei oder mehrere Bindungen an die Gruppen der Formeln

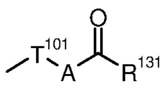 (CI), 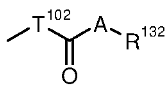 (CII)

angebunden sind.

Beispiele für solche bi- oder oligofunktionellen $R^{131}$ sind $-(CH_2)_4-$, $-NH-(CH_2)_6-NH-$,

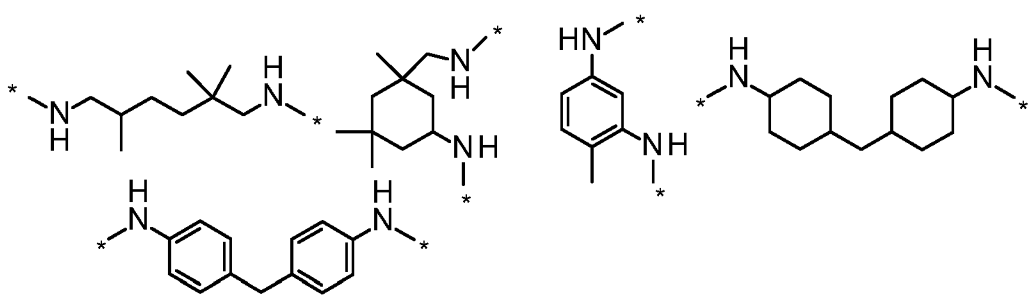

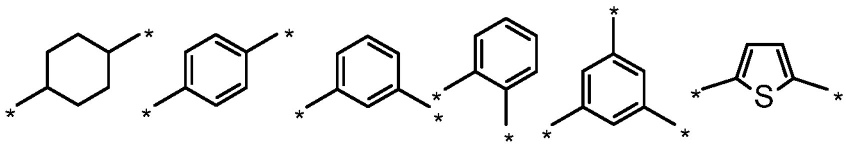

Beispiele für solche bi- oder oligofunktionellen $R^{132}$ sind $-(CH_2)_2-$, $-(CH_2)_4-$, $-(CH_2)_2-O-(CH_2)_2-$,

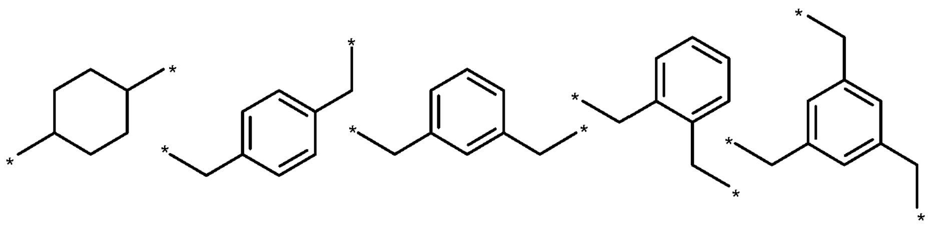

Beispiele für Brücken $T^{101}$ sind $-(CH_2)_2-$, $-(CH_2)_3-$, $-(CH_2)_4-$, $-CH_2CH(CH_3)-$, $-(CH_2)_2-O-(CH_2)_2-$, $-[(CH_2)_2-O-]_2(CH_2)_2-$, $-(CH_2)_4-O-CH_2-CH_2-$,

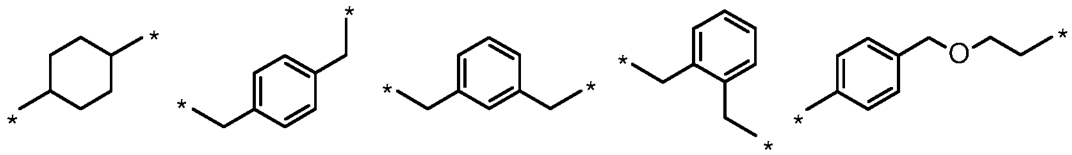

Beispiele für Brücken $T^{102}$ sind $-CH_2-$, $-(CH_2)_2-$, $-(CH_2)_3-$, $-(CH_2)_4-$, $-CH_2CH(CH_3)-$, $-(CH_2)_5-$,$-(CH_2)_6-$,

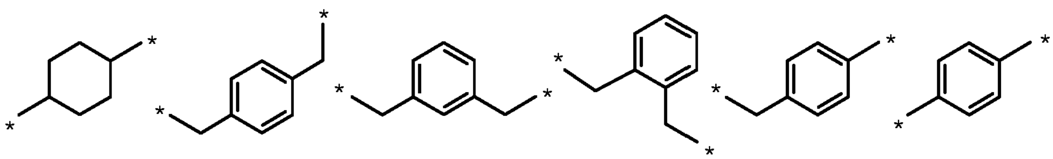

Unter einem heterocyclischen Rest ist zu verstehen ein fünf- oder sechsgliedriger, mindestens ein N, O oder S enthaltender, gegebenenfalls benzanellierter und / oder durch nichtionische Reste substituierter aromatischer oder quasiaromatischer, teilgesättigter oder gesättigter Ring. Gleiches ist gemeint mit dem heterocyclischen Rest in Heteroaryloxy und Heteroarylamino.

Beispiele sind: Pyridyl, Benzthiazolyl, Thienyl, Piperidyl.

Unter einem nicht-ionischen Rest ist zu verstehen: Halogen, Alkyl, Alkoxy, Cyano, Nitro, COO-Alkyl.

Beispiele für Halogen, Alkyl, Alkoxy sind Fluor, Chlor, Brom, Methyl, Ethyl, Methoxy.

Auch offenbart sind Verbindungen der Formel (CC), wobei die Boratkomponente optional nach dem erfindungsgemäßen Verfahren hergestellt werden kann oder hergestellt wird,

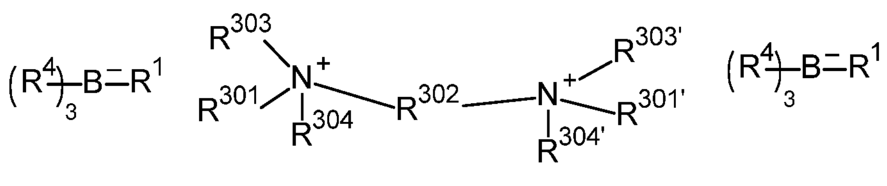

(CC)

worin

$R^{301}$ und $R^{301'}$ jeweils unabhängig voneinander für einen gegebenenfalls verzweigten $C_{14}$- bis $C_{22}$-Alkyl-Rest stehen oder zusätzlich die Bedeutung von $R^{302}$ haben unter der Voraussetzung, dass $T^{301}$ und $R^{331}$ beziehungsweise $T^{302}$ und $R^{332}$ jeweils zusammen mindestens 12 C-Atome enthalten,

$R^{302}$ für einen Rest der Formeln

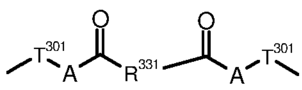 (CCI), 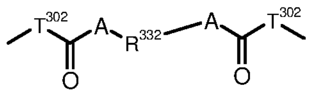 (CCII)

steht,

$T^{301}$ für eine Brücke mit 2 bis 16 C-Atomen steht, von denen maximal ein Drittel durch O und / oder $NR^{400}$ ersetzt sein kann, wobei zwischen zwei O bzw. $NR^{200}$ mindestens 2 C-Atome stehen müssen, und die in Form einer gegebenenfalls verzweigten Kette und / oder einem fünf- oder sechsgliedrigen Ring angeordnet sein können,

$R^{331}$ für einen gegebenenfalls verzweigten und / oder gegebenenfalls substituierten $C_4$- bis $C_{22}$-Alkylen-Rest, einen Cyclopentylen-, Cyclohexylen- oder Cycloheptylen-Rest, einen $C_8$- bis $C_{12}$-Aryldialkylen-Rest, einen gegebenenfalls durch nichtionische Reste substituierten Arylen-Rest oder Heteroarylen-Rest, einen gegebenenfalls verzweigten und / oder gegebenenfalls substituierten $C_4$- bis $C_{22}$-Alkandioxy-Rest, einen Cyclopentandioxy-, Cyclohexandioxy- oder Cycloheptandioxy-Rest, einen $C_7$- bis $C_{12}$-Oxyarylalkyloxy-Rest, einen $C_8$- bis $C_{12}$-Aryldi(alkyloxy)-Rest, einen gegebenenfalls durch nichtionische Reste substituierten Benzol-dioxy-Rest oder Heteroaryldioxy-Rest, einen gegebenenfalls verzweigten und / oder gegebenenfalls substituierten $C_4$- bis $C_{22}$-Alkandiamino-Rest, einen Cyclopentan-diamino, Cyclohexandiamino- oder Cycloheptandiamino-Rest, einen $C_8$- bis $C_{12}$-Aminoarylalkylamino-Rest, einen gegebenenfalls durch nichtionische Reste substituierten Benzoldiamino-Rest oder Heteroarylendiamino-Rest, einen Alkylen-Cycloalkylen-Rest, Alkandicycloalkylen-Rest oder Alkandiarylen-Rest steht,

$T^{302}$ für eine Brücke mit 1 bis 16 C-Atomen steht, die in Form einer gegebenenfalls verzweigten Kette und / oder einem fünf- oder sechsgliedrigen Ring angeordnet sein können,

$R^{332}$ für einen gegebenenfalls verzweigten und / oder gegebenenfalls substituierten $C_1$- bis $C_{22}$-Alkylen-Rest, einen Cyclopentylen-, Cyclohexylen- oder Cycloheptylen-Rest, einen $C_8$- bis $C_{12}$-Aralkylen-Rest, einen gegebenenfalls durch nichtionische Reste substituierten Arylen-Rest oder Heteroarylen--Rest steht,

A für $NR^{401}$ oder Sauerstoff steht,

$R^{400}$ und $R^{401}$ unabhängig voneinander für Wasserstoff oder $C_1$- bis $C_4$-Alkyl stehen,

$R^{303}$, $R^{304}$, $R^{303'}$ und $R^{304'}$ unabhängig voneinander für einen gegebenenfalls verzweigten und / oder gegebenenfalls substituierten $C_1$- bis $C_5$-Alkyl-Rest stehen,
oder

$R^{302}$ für einen Rest der Formeln

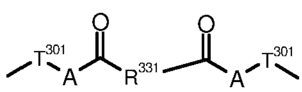 (CCI), 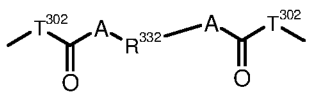 (CCII)

steht,

$T^{301}$, $R^{331}$, $T^{302}$, $R^{332}$, $R^{400}$, $R^{401}$ und A die oben genannte Bedeutung haben unter der Voraussetzung, dass $T^{301}$ und $R^{331}$ beziehungsweise $T^{302}$ und $R^{332}$ jeweils zusammen mindestens 12 C-Atome enthalten,

$R^{301}$, $R^{303}$ und $R^{304}$ gemeinsam mit dem $N^+$-Atom einen Imidazol- oder Pyridin-Ring bilden, der mindestens durch einen Rest der Auswahl $C_1$- bis $C_8$-Alkyl, $C_1$- bis $C_8$-Alkoxy, $C_5$- bis $C_7$-Cycloalkyl, Benzyl oder Phenyl substituiert ist, und / oder

$R^{301'}$, $R^{303'}$ und $R^{304'}$ gemeinsam mit dem $N^+$-Atom einen Imidazol- oder Pyridin-Ring bilden, der mindestens durch einen Rest der Auswahl $C_1$- bis $C_8$-Alkyl, $C_1$- bis $C_8$-Alkoxy, $C_5$- bis $C_7$-Cycloalkyl, Benzyl oder Phenyl substituiert ist, oder

$R^{301}$ und $R^{301'}$ jeweils unabhängig voneinander für einen gegebenenfalls verzweigten $C_{14}$- bis $C_{22}$-Alkyl-Rest stehen

oder zusätzlich die Bedeutung von $R^{302}$ haben unter der Voraussetzung, dass $T^{301}$ und $R^{331}$ beziehungsweise $T^{302}$ und $R^{332}$ jeweils zusammen mindestens 12 C-Atome enthalten,

$R^{302}$ für einen Rest der Formeln

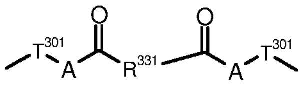 (CCI), 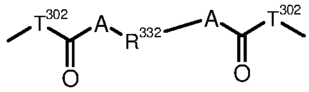 (CCII)

steht und

$T^{301}$, $R^{331}$, $T^{302}$, $R^{332}$, $R^{400}$, $R^{401}$ und A die oben genannte Bedeutung haben,

$R^{303}$ und $R^{304}$ gemeinsam eine $-(CH_2)_4-$, $-(CH_2)_5-$ oder $-(CH_2)_2$-O-$(CH_2)_2$-Brücke bilden, und / oder $R^{303'}$ und $R^{304'}$ gemeinsam eine $-(CH_2)_4-$, $-(CH_2)_5-$ oder $-(CH_2)_2$-O-$(CH_2)_2$-Brücke bilden,

und $R^1$ und $R^4$ die oben genannte Bedeutung haben.

Auch offenbart sind Verbindungen der Formel (CC),

worin

$R^{301}$ und $R^{301'}$ jeweils unabhängig voneinander für einen gegebenenfalls verzweigten $C_{14}$- bis $C_{22}$-Alkyl-Rest stehen oder zusätzlich die Bedeutung von $R^{302}$ haben unter der Voraussetzung, dass $T^{301}$ und $R^{331}$ beziehungsweise $T^{302}$ und $R^{332}$ jeweils zusammen mindestens 12 C-Atome enthalten,

$R^{302}$ für einen Rest der Formeln

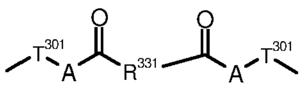 (CCI), 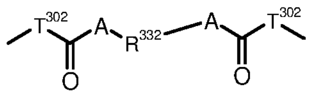 (CCII)

steht,

$T^{301}$ für eine Brücke mit 2 bis 9 C-Atomen steht, von denen maximal ein Drittel durch O und / oder $NR^{400}$ ersetzt sein kann, wobei zwischen zwei O bzw. $NR^{400}$ mindestens 2 C-Atome stehen müssen, und die in Form einer gegebenenfalls verzweigten Kette und / oder einem fünf- oder sechsgliedrigen Ring angeordnet sein können,

$R^{331}$ für einen gegebenenfalls verzweigten und / oder gegebenenfalls substituierten $C_4$- bis $C_{16}$-Alkylen-Rest, einen Cyclopentylen- oder Cyclohexylen-Rest, einen Xylylen-, Benzoldiethylen- oder Benzoldipropylen-Rest, einen gegebenenfalls durch nichtionische Reste substituierten Arylen-Rest oder Heteroarylen-Rest, einen gegebenenfalls verzweigten und / oder gegebenenfalls substituierten $C_4$- bis $C_{16}$-Alkandioxy-Rest, einen Cyclopentandioxy- oder Cyclohexandioxy-Rest, einen Benzoldi(methyloxy)-, Benzoldi(ethyloxy)- oder Benzoldi(propyloxy)-Rest, einen gegebenenfalls durch nichtionische Reste substituierten Benzoldioxy-Rest oder Heteroarylendioxy-Rest, einen gegebenenfalls verzweigten und / oder gegebenenfalls substituierten $C_4$- bis $C_{22}$- Alkandiamino-Rest, einen Cyclopentandiamino- oder Cyclohexandiamino-Rest, einen Benzoldi(methylamino)-, Benzoldi(ethylamino)- oder Benzoldi(propylamino)-Rest, einen gegebenenfalls durch nichtionische Reste substituierten Benzoldiamino-Rest, Methyldicyclohexylen-Rest, 4-Methylenbis(4,1-phenylen)-Rest oder Heteroarylendiamino-Rest steht,

$T^{302}$ für eine Brücke mit 1 bis 9 C-Atomen steht, die in Form einer gegebenenfalls verzweigten Kette und / oder einem fünf- oder sechsgliedrigen Ring angeordnet sein können,

$R^{332}$ für einen gegebenenfalls verzweigten und / oder gegebenenfalls substituierten $C_1$- bis $C_{16}$-Alkylen-Rest, einen Cyclopentylen- oder Cyclohexylen-Rest, Xylylen--, Benzoldiethylen- oder Benzoldipropylen-Rest, einen gegebenenfalls durch nichtionische Reste substituierten Arylen-Rest oder Heteroarylen-Rest steht,

A für $NR^{401}$ oder Sauerstoff steht,

$R^{400}$ und $R^{401}$ unabhängig voneinander für Wasserstoff, Methyl oder Ethyl stehen,

$R^{303}$ und $R^{304}$ unabhängig voneinander für einen gegebenenfalls verzweigten und / oder gegebenenfalls substituierten $C_1$- bis $C_5$-Alkyl-Rest stehen, und / oder

$R^{303'}$ und $R^{304'}$ unabhängig voneinander für einen gegebenenfalls verzweigten und / oder gegebenenfalls substituierten $C_1$- bis $C_5$-Alkyl-Rest stehen,
oder

$R^{302}$ für einen Rest der Formeln

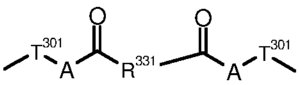 (CCI), 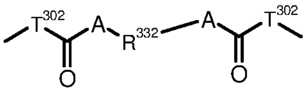 (CCII)

steht,

$T^{301}$, $R^{331}$, $T^{302}$, $R^{332}$, $R^{400}$, $R^{401}$ und A die oben genannte Bedeutung haben unter der Voraussetzung, dass $T^{301}$ und $R^{331}$ beziehungsweise $T^{302}$ und $R^{332}$ jeweils zusammen mindestens 12 C-Atome enthalten,

$R^{301}$, $R^{303}$ und $R^{304}$ gemeinsam mit dem $N^+$-Atom einen Imidazol- oder Pyridin-Ring bilden, der mindestens durch einen Rest der Auswahl $C_1$- bis $C_6$-Alkyl, $C_1$- bis $C_6$-Alkoxy, Cyclopentyl, Cyclohexyl, Benzyl oder Phenyl substituiert ist, und / oder

$R^{301'}$, $R^{303'}$ und $R^{304'}$ gemeinsam mit dem $N^+$-Atom einen Imidazol- oder Pyridin-Ring bilden, der mindestens durch einen Rest der Auswahl $C_1$- bis $C_6$-Alkyl, $C_1$- bis $C_6$-Alkoxy, Cyclopentyl, Cyclohexyl, Benzyl oder Phenyl substituiert ist,
oder

$R^{301}$ und $R^{301'}$ jeweils unabhängig voneinander für einen gegebenenfalls verzweigten $C_{14}$- bis $C_{22}$-Alkyl-Rest stehen oder zusätzlich die Bedeutung von $R^{302}$ haben unter der Voraussetzung, dass $T^{301}$ und $R^{331}$ beziehungsweise $T^{302}$ und $R^{332}$ jeweils zusammen mindestens 12 C-Atome enthalten,

$R^{302}$ für einen Rest der Formeln

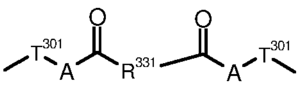 (CCI), 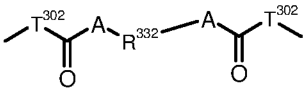 (CCII)

steht und

$T^{301}$, $R^{331}$, $T^{302}$, $R^{332}$, $R^{400}$, $R^{401}$ und A die oben genannte Bedeutung haben,

$R^{303}$ und $R^{304}$ gemeinsam eine $-(CH_2)_4-$, $-(CH_2)_5-$ oder $-(CH_2)_2-O-(CH_2)_2-$Brücke bilden, und / oder $R^{303'}$ und $R^{304'}$ gemeinsam eine $-(CH_2)_4-$, $-(CH_2)_5-$ oder $-(CH_2)_2-O-(CH_2)_2-$Brücke bilden
und

$R^1$ und $R^4$ die oben genannte Bedeutung haben.

Auch offenbart sind Verbindungen der Formel (CC),
worin
$R^{301}$ und $R^{301'}$ jeweils unabhängig voneinander für einen gegebenenfalls verzweigten $C_{14}$- bis $C_{22}$-Alkyl-Rest stehen,
$R^{302}$ für einen Rest der Formeln

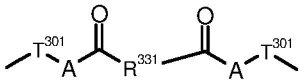 (CCI), 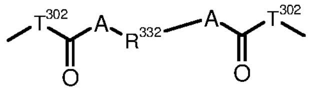 (CCII)

steht

$T^{301}$ für eine Brücke in Form einer gegebenenfalls verzweigten Kette mit 2 bis 8 C-Atomen steht, die 1 oder 2 O-Atome enthalten kann, wobei zwischen zwei O-Atomen mindestens 2 C-Atome stehen müssen, oder für eine Brücke der Formeln

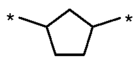 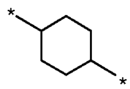 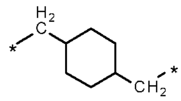 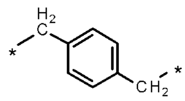

steht,

$R^{331}$ für einen gegebenenfalls verzweigten und / oder gegebenenfalls substituierten $C_4$- bis $C_{16}$-Alkylen-Rest, einen Cyclopentylen- oder Cyclohexylen-Rest, einen Xylylen-Rest, einen gegebenenfalls durch nichtionische Reste substituierten Arylen-Rest oder einen Furylen-, Thienylen- oder Pyridylen-Rest, einen gegebenenfalls verzweigten und / oder gegebenenfalls substituierten $C_4$- bis $C_{16}$-Alkandioxy-Rest, einen Cyclopentandioxy- oder Cyclohexandioxy-Rest, einen Benzol-di(methyloxy)-Rest, einen gegebenenfalls durch nichtionische Reste substituierten Benzoldioxy-Rest, einen gegebenenfalls verzweigten und / oder gegebenenfalls substituierten $C_4$- bis $C_{22}$-Alkandiamino-Rest, einen Cyclopentandiamino - oder Cyclohexandiamino -Rest, einen Benzol-di(methylamino)-Rest, einen gegebenenfalls durch nichtionische Reste substituierten Benzoldiamino-Rest oder Pyridindiamino-Rest steht,

$T^{302}$ für eine Brücke in Form einer gegebenenfalls verzweigten Kette mit 2 bis 8 C-Atomen steht oder für eine Brücke der Formeln

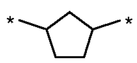 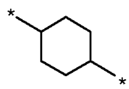 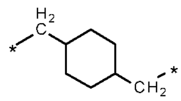 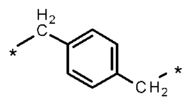

steht,

$R^{332}$ für einen gegebenenfalls verzweigten und / oder gegebenenfalls substituierten $C_1$- bis $C_{16}$-Alkylen-Rest, einen Cyclopentylen- oder Cyclohexylen-Rest, einen Xylylen-Rest, einen gegebenenfalls durch nichtionische Reste substituierten Arylen-Rest oder Pyridylen-Rest steht,

A für $NR^{401}$ oder Sauerstoff steht,

$R^{401}$ für Wasserstoff oder Methyl steht,

$R^{303}$ und $R^{304}$ unabhängig voneinander für einen gegebenenfalls verzweigten und / oder gegebenenfalls substituierten $C_1$- bis $C_5$-Alkyl-Rest stehen, und / oder

$R^{303'}$ und $R^{304'}$ unabhängig voneinander für einen gegebenenfalls verzweigten und / oder gegebenenfalls substituierten $C_1$- bis $C_5$-Alkyl-Rest stehen
oder

$R^{302}$ für einen Rest der Formeln

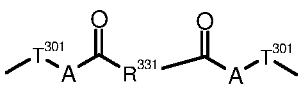 (CCI), 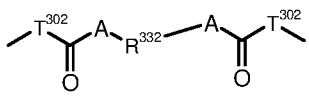 (CCII)

steht,

$T^{301}$, $R^{331}$, $T^{302}$, $R^{332}$, $R^{400}$, $R^{401}$ und A die oben genannte Bedeutung haben unter der Voraussetzung, dass $T^{301}$ und $R^{331}$ beziehungsweise $T^{302}$ und $R^{332}$ jeweils zusammen mindestens 12 C-Atome enthalten,

$R^{301}$, $R^{303}$ und $R^{304}$ gemeinsam mit dem $N^+$-Atom einen Imidazol- oder Pyridin-Ring bilden, der mindestens durch einen Rest der Auswahl $C_1$- bis $C_4$-Alkyl, $C_1$- bis $C_4$-Alkoxy, Cyclopentyl, Cyclohexyl, Benzyl oder Phenyl substituiert ist, und / oder

$R^{301'}$, $R^{303'}$ und $R^{304'}$ gemeinsam mit dem $N^+$-Atom einen Imidazol- oder Pyridin-Ring bilden, der mindestens durch einen Rest der Auswahl $C_1$- bis $C_4$-Alkyl, $C_1$- bis $C_4$-Alkoxy, Cyclopentyl, Cyclohexyl, Benzyl oder Phenyl substituiert ist
oder

$R^{301}$ und $R^{301'}$ jeweils unabhängig voneinander für einen gegebenenfalls verzweigten $C_{14}$- bis $C_{22}$-Alkyl-Rest stehen,

$R^{302}$ für einen Rest der Formeln

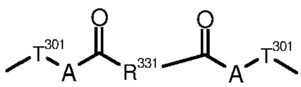 (CCI), 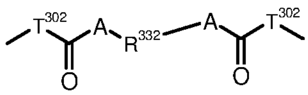 (CCII)

steht und

$T^{301}$, $R^{331}$, $T^{302}$, $R^{332}$, $R^{400}$, $R^{401}$ und A die oben genannte Bedeutung haben,
$R^{303}$ und $R^{304}$ gemeinsam eine $-(CH_2)_4-$, $-(CH_2)_5-$ oder $-(CH_2)_2-O-(CH_2)_2-$Brücke bilden, und / oder $R^{303'}$ und $R^{304'}$ gemeinsam eine $-(CH_2)_4-$, $-(CH_2)_5-$ oder $-(CH_2)_2-O-(CH_2)_2-$Brücke bilden
und
$R^1$ und $R^4$ die oben genannte Bedeutung haben.

Auch offenbart sind Verbindungen der Formel (CC),
worin
$R^{301}$ und $R^{301'}$, $R^{302}$ und $R^{302'}$ sowie $R^{303}$ und $R^{303'}$ paarweise gleich sind und die anderen Reste die oben genannten Bedeutungen besitzen.

Unter $R^{331}$ und $R^{332}$
in den Formeln

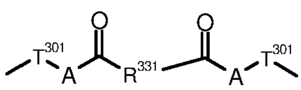 (CCI), 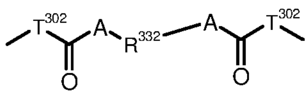 (CCII)

sind auch solche Reste zu verstehen, die über drei oder mehrere Bindungen an die Gruppen der Formeln

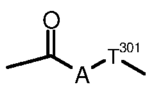 oder 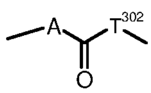

angebunden sind.

Beispiele für solche bi- oder oligofunktionellen $R^{331}$ sind $-(CH_2)_4-$, $-NH-(CH_2)_6-NH-$,

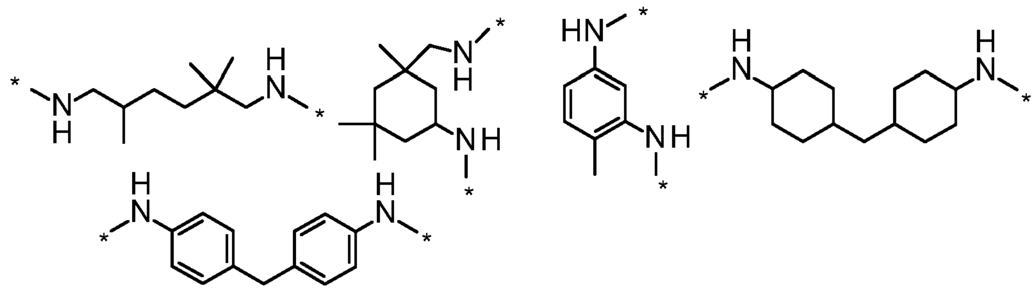

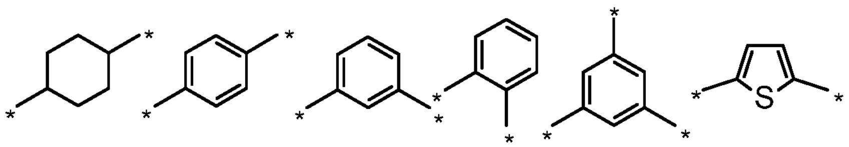

Beispiele für solche bi- oder oligofunktionellen $R^{332}$ sind $-(CH_2)_2-$, $-(CH_2)_4-$, $-(CH_2)_2-O-(CH_2)_2-$,

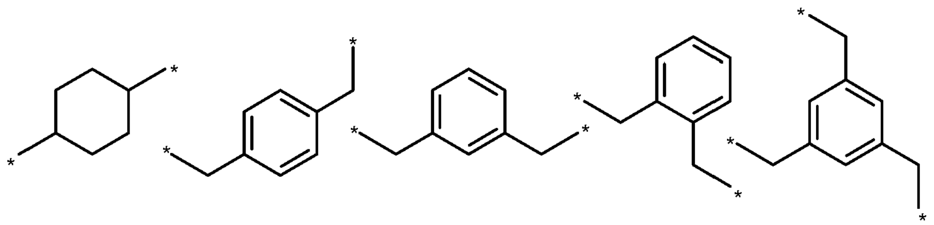

Beispiele für Brücken $T^{301}$ sind $-(CH_2)_2-$, $-(CH_2)_3-$, $-(CH_2)_4-$, $-CH_2CH(CH_3)-$, $-(CH_2)_2-O-(CH_2)_2-$, $-[(CH_2)_2-O-]_2(CH_2)_2-$, $-(CH_2)_4-O-CH_2-CH_2-$,

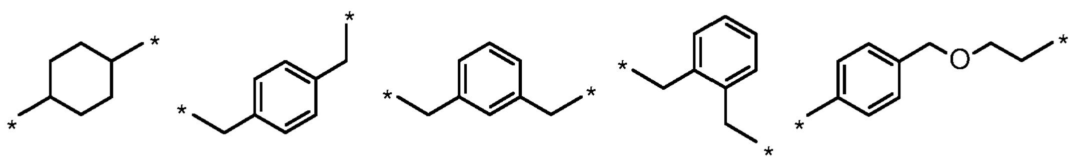

Beispiele für Brücken $T^{302}$ sind $-CH_2-$, $-(CH_2)_2-$, $-(CH_2)_3-$, $-(CH_2)_4-$, $-CH_2CH(CH_3)-$, $-(CH_2)_5-$,$-(CH_2)_6-$,

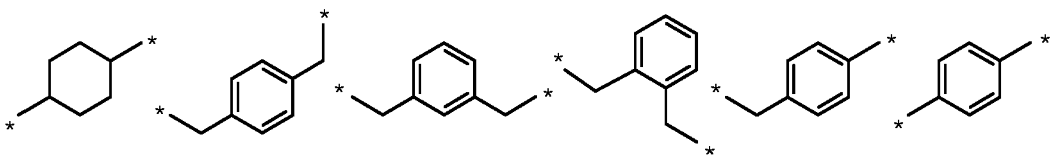

Unter einem heterocyclischen Rest ist zu verstehen ein fünf- oder sechsgliedriger, mindestens ein N, O oder S enthaltender, gegebenenfalls benzanellierter und / oder durch nichtionische Reste substituierter aromatischer oder quasiaromatischer, teilgesättigter oder gesättigter Ring. Gleiches ist gemeint mit dem heterocyclischen Rest in Heteroaryloxy und Heteroarylamino.

Beispiele sind: Pyridyl, Benzthiazolyl, Thienyl, Piperidyl.

Unter einem nicht-ionischen Rest ist zu verstehen: Halogen, Alkyl, Alkoxy, Cyano, Nitro, COO-Alkyl.

Beispiele für Halogen, Alkyl, Alkoxy sind Fluor, Chlor, Brom, Methyl, Ethyl, Methoxy.

Die Matrixpolymere der Photopolymer-Formulierung können insbesondere vernetzt und besonders bevorzugt dreidimensional vernetzt sein.

Auch offenbart sind die gemäß dem erfindungsgemäßen Verfahren herstellbaren oder hergestellten Triaryl-organoborate.

Auch offenbart ist ist die Verwendung der herstellbaren oder hergestellten Triaryl-organoborate oder der oben beschriebene Verbindungen der Formel (C) oder der oben beschriebene Verbindungen der Formel (CC) in Photoinitiatorsystemen, sowie Photopolymerzusammensetzungen enthaltend eine photopolymerisierbare Komponente und ein Photoinitiatorsystem enthaltend die hergestellten Triaryl-organoborate. Außerdem werden aus den genannten Photopolymerzusammensetzungen holographische Medien und die daraus erhältlichen Hologramme zugänglich.

Geeignete Matrixpolymere einer entsprechenden Photopolymer-Formulierung können insbesondere vernetzt und besonders bevorzugt dreidimensional vernetzt sein.

Vorteilhaft ist auch, wenn die Matrixpolymere Polyurethane sind, wobei die die Polyurethane insbesondere durch Umsetzung wenigstens einer Polyisocyanat-Komponente a) mit wenigstens einer Isocyanat-reaktiven-Komponente b) erhältlich sein können.

Die Polyisocyanat-Komponente a) umfasst bevorzugt wenigstens eine organische Verbindung mit wenigstens zwei NCO-Gruppen. Bei diesen organischen Verbindungen kann es sich insbesondere um monomere Di- und Triisocyanate, Polyisocyanate und / oder NCO-funktionelle Prepolymere handeln. Die Polyisocyanat-Komponente a) kann auch Mischungen monomerer Di- und Triisocyanate, Polyisocyanate und / oder NCO-funktioneller Prepolymere enthalten oder daraus bestehen.

Als monomere Di- und Triisocyanate können alle dem Fachmann an sich gut bekannten Verbindungen oder deren Mischungen eingesetzt werden. Diese Verbindungen können aromatische, araliphatische, aliphatische oder cycloaliphatische Strukturen aufweisen. In untergeordneten Mengen können die monomeren Di- und Triisocyanate auch Monoisocyanate, d.h. organische Verbindungen mit einer NCO-Gruppe umfassen.

Beispiele für geeignete monomere Di- und Triisocyanate sind 1,4-Butandiisocyanat, 1,5-Pentandiisocyanat, 1,6-Hexandiisocyanat (Hexamethylendiisocyanat, HDI), 2,2,4-Trimethylhexamethylendiisocyanat und / oder 2,4,4-Trimethylhexamethylendiisocyanat (TMDI), Isophorondiisocyanat (IPDI), 1,8-Diisocyanato-4-(isocyanatomethyl)-octan, Bis-(4,4'-isocyanatocyclohexyl)methan und / oder Bis-(2',4-isocyanatocyclohexyl)methan und / oder deren Mischungen beliebigen Isomerengehalts, 1,4-Cyclohexandiisocyanat, die isomeren Bis-(isocyanatomethyl)cyclohexane, 2,4- und / oder 2,6-Diisocyanato-1-methylcyclohexan (Hexahydro-2,4- und / oder 2,6-toluylendiisocyanat, $H_6$-TDI), 1,4-Phenylendiisocyanat, 2,4- und / oder 2,6-Toluylendiisocyanat (TDI), 1,5-Naphthylendiisocyanat (NDI), 2,4'- und / oder 4,4'-Diphenylmethandiisocyanat (MDI), 1,3-Bis-(isocyanatomethyl)benzol (XDI) und / oder das analoge 1,4-Isomere oder beliebige Mischungen der vorgenannten Verbindungen.

Geeignete Polyisocyanate sind Verbindungen mit Urethan-, Harnstoff-, Carbodiimid-, Acylharnstoff-, Amid-, Isocyanurat-, Allophanat-, Biuret-, Oxadiazintrion-, Uretdion- und / oder Iminooxadiazindionstrukturen, die aus den vorgenannten Di- oder Triisocyanaten erhältlich sind.

Besonders bevorzugt handelt es sich bei den Polyisocyanaten um oligomerisierte aliphatische und / oder cycloaliphatische Di- oder Triisocyanate, wobei insbesondere die oben stehenden aliphatischen und / oder cycloaliphatischen Di- oder Triisocyanate verwendet werden können.

Ganz besonders bevorzugt sind Polyisocyanate mit Isocyanurat-, Uretdion- und / oder Iminooxadiazindion-Strukturen sowie Biurete basierend auf HDI oder deren Mischungen.

Geeignete Prepolymere enthalten Urethan- und / oder Harnstoff-Gruppen sowie gegebenenfalls weitere durch Modifizierung von NCO-Gruppen entstandene Strukturen wie oben genannt. Derartige Prepolymere sind beispielsweise durch Umsetzung der oben genannten monomeren Di- und Triisocyanate und / oder Polyisocyanaten a1) mit isocyanatreaktiven Verbindungen b1) erhältlich.

Als isocyanatreaktive Verbindungen b1) können Alkohole, Amino oder Mercapto-Verbindungen, bevorzugt Alkohole, verwendet werden. Dabei kann es sich insbesondere um Polyole handeln. Ganz besonders bevorzugt können als isocyanatreaktive Verbindung b1) Polyester-, Polyether-, Polycarbonat-, Poly(meth)acrylat- und / oder Polyurethan-Polyole verwendet werden.

Als Polyesterpolyole sind beispielsweise lineare Polyesterdiole oder verzweigte Polyesterpolyole geeignet, die in bekannter Weise durch Umsetzung von aliphatischen, cycloaliphatischen oder aromatischen Di- bzw. Polycarbonsäuren bzw. ihren Anhydriden mit mehrwertigen Alkoholen einer OH-Funktionalität $\geq 2$ erhalten werden können. Beispiele für geeignete Di- bzw. Polycarbonsäuren sind mehrwertige Carbonsäuren wie Bernstein-, Adipin-, Kork-, Sebacin-, Decandicarbon-, Phthal-, Terephthal-, Isophthal-, Tetrahydrophthal- oder Trimellithsäure sowie Säureanhydride wie Phthal-, Trimellith- oder Bernsteinsäureanhydrid oder deren beliebige Gemische untereinander. Die Polyesterpolyole können auch auf natürlichen Rohstoffen wie Rizinusöl basieren. Es ist ebenfalls möglich, dass die Polyesterpolyole auf Homo- oder Mischpolymerisaten von Lactonen basieren, die bevorzugt durch Anlagerung von Lactonen bzw. Lactongemischen wie Butyrolacton, $\varepsilon$-Caprolacton und / oder Methyl-$\varepsilon$-caprolacton an hydroxyfunktionelle Verbindungen wie mehrwertige Alkohole einer OH-Funktionalität $\geq 2$ beispielsweise der nachstehend genannten Art erhalten werden können.

Beispiele für geeignete Alkohole sind alle mehrwertigen Alkohole wie z.B. die $C_2$ - $C_{12}$-Diole, die isomeren Cyclohexandiole, Glycerin oder deren beliebige Gemische untereinander.

Geeignete Polycarbonatpolyole sind in an sich bekannter Weise durch Umsetzung von organischen Carbonaten oder Phosgen mit Diolen oder Diol-Mischungen zugänglich.

Geeignete organische Carbonate sind Dimethyl-, Diethyl- und Diphenylcarbonat.

Geeignete Diole bzw. Mischungen umfassen die an sich im Rahmen der Polyestersegmente genannten mehrwertigen Alkohole einer OH-Funktionalität ≥ 2, bevorzugt Butandiol-1,4, Hexandiol-1,6 und / oder 3-Methylpentandiol. Auch Polyesterpolyole können zu Polycarbonatpolyolen umgearbeitet werden.

Geeignete Polyetherpolyole sind gegebenenfalls blockweise aufgebaute Polyadditionsprodukte cyclischer Ether an OH- oder NH-funktionelle Startermoleküle.

Geeignete cyclische Ether sind beispielsweise Styroloxide, Ethylenoxid, Propylenoxid, Tetrahydrofuran, Butylenoxid, Epichlorhydrin sowie ihre beliebigen Mischungen.

Als Starter können die an sich im Rahmen der Polyesterpolyole genannten mehrwertigen Alkohole einer OH-Funktionalität ≥ 2 sowie primäre oder sekundäre Amine und Aminoalkohole verwendet werden.

Bevorzugte Polyetherpolyole sind solche der vorgenannten Art ausschließlich basierend auf Propylenoxid oder statistische oder Block-Copolymere basierend auf Propylenoxid mit weiteren 1-Alkylenoxiden. Besonders bevorzugt sind Propylenoxid-homopolymere sowie statistische oder Block-Copolymere, die Oxyethylen-, Oxypropylen- und / oder Oxybutyleneinheiten aufweisen, wobei der Anteil der Oxypropyleneinheiten bezogen auf die Gesamtmenge aller Oxyethylen-, Oxypropylen- und Oxybutyleneinheiten mindestens 20 Gew.-%, bevorzugt mindestens 45 Gew.-% ausmacht. Oxypropylen und Oxybutylen umfassen hierbei alle jeweiligen linearen und verzweigten $C_3$- und $C_4$-Isomere.

Daneben sind als Bestandteile der Polyol-Komponente b1) als polyfunktionelle, isocyanatreaktive Verbindungen auch niedermolekulare, d.h. mit Molekulargewichten ≤ 500 g/mol, kurzkettige, d.h. 2 bis 20 Kohlenstoffatome enthaltende aliphatische, araliphatische oder cycloaliphatische di-, tri- oder polyfunktionelle Alkohole geeignet.

Dies können beispielsweise in Ergänzung zu den oben genannten Verbindungen Neopentylglykol, 2-Ethyl-2-butylpropandiol, Trimethylpentandiol, stellungs-isomere Diethyloctandiole, Cyclohexandiol, 1,4-Cyclohexandimethanol, 1,6-Hexandiol, 1,2- und 1,4-Cyclohexandiol, hydriertes Bisphenol A, 2,2-Bis(4-hydroxy-cyclohexyl)-propan oder 2,2-Dimethyl-3-hydroxypropionsäure, 2,2-dimethyl-3-hydroxypropyl-ester sein. Beispiele geeigneter Triole sind Trimethylolethan, Trimethylolpropan oder Glycerin. Geeignete höherfunktionelle Alkohole sind Di-(trimethylolpropan), Pentaerythrit, Dipenta-erythrit oder Sorbit.

Besonders bevorzugt ist, wenn die Polyolkomponente ein difunktioneller Polyether-, Polyester oder ein Polyether-polyester-block-copolyester oder ein Polyether-Polyester-Blockcopolymer mit primären OH-Funktionen ist.

Es ist ebenfalls möglich, als isocyanatreaktive Verbindungen b1) Amine einzusetzen. Beispiele geeigneter Amine sind Ethylendiamin, Propylendiamin, Diaminocyclohexan, 4,4'-Dicyclohexylmethandiamin, Isophorondiamin (IPDA), difunktionelle Polyamine wie z.B. die Jeffamine®, aminterminierte Polymere, insbesondere mit zahlenmittleren Molmassen ≤ 10.000 g/Mol. Mischungen der vorgenannten Amine können ebenfalls verwendet werden.

Es ist ebenfalls möglich, als isocyanatreaktive Verbindungen b1) Aminoalkohole einzusetzen. Beispiele geeigneter Aminoalkohole sind die isomeren Aminoethanole, die isomere Aminopropanole, die isomeren Aminobutanole und die isomeren Aminohexanole oder deren beliebige Mischungen.

Alle vorgenannten isocyanatreaktiven Verbindungen b1) können untereinander beliebig vermischt werden.

Bevorzugt ist auch, wenn die isocyanatreaktiven Verbindungen b1) eine zahlenmittlere Molmasse von ≥ 200 und ≤ 10.000 g/Mol, weiter bevorzugt ≥ 500 und ≤ 8.000 g/Mol und ganz besonders bevorzugt ≥ 800 und ≤ 5.000 g/Mol aufweisen. Die OH-Funktionalität der Polyole beträgt bevorzugt 1.5 bis 6.0, besonders bevorzugt 1.8 bis 4.0.

Die Prepolymere der Polyisocyanat-Komponente a) können insbesondere einen Restgehalt an freiem monomeren Di- und Triisocyanaten < 1 Gew.-%, besonders bevorzugt < 0.5 Gew.-% und ganz besonders bevorzugt < 0.3 Gew.-% aufweisen.

Es ist gegebenenfalls auch möglich, dass die Polyisocyanat-Komponente a) vollständig oder anteilsmäßig eine organische Verbindung enthält, deren NCO-Gruppen ganz oder teilweise mit aus der Beschichtungstechnologie bekannten Blockierungsmitteln umgesetzt sind. Beispiel für Blockierungsmittel sind Alkohole, Lactame, Oxime, Malonester, Pyrazole sowie Amine, wie z.B. Butanonoxim, Diisopropylamin, Malonsäurediethylester, Acetessigester, 3,5-Dimethylpyrazol, ε-Caprolactam oder deren Mischungen.

Besonders bevorzugt ist, wenn die Polyisocyanat-Komponente a) Verbindungen mit aliphatisch gebundenen NCO-Gruppen umfasst, wobei unter aliphatisch gebundenen NCO-Gruppen derartige Gruppen verstanden werden, die an ein primäres C-Atom gebunden sind. Die isocyanatreaktive Komponente b) umfasst bevorzugt wenigstens eine organische Verbindung, die im Mittel wenigstens 1.5 und bevorzugt 2 bis 3 isocyanatreaktive Gruppen aufweist. Im Rahmen der vorliegenden Erfindung werden als isocyanatreaktive Gruppen bevorzugt Hydroxy-, Amino- oder MercaptoGruppen angesehen.

Die isocyanatreaktive Komponente kann insbesondere Verbindungen umfassen, die im Zahlenmittel wenigstens 1.5 und bevorzugt 2 bis 3 isocyanatreaktive Gruppen aufweisen.

Geeignete polyfunktionelle, isocyanatreaktive Verbindungen der Komponente b) sind beispielsweise die oben beschriebenen Verbindungen b1).

Bei einer weiteren bevorzugten Ausführungsform ist vorgesehen, dass das Schreibmonomer c) wenigstens ein mono- und / oder ein multifunktionales Schreibmonomer umfasst oder daraus besteht. Weiter bevorzugt kann das Schreibmonomer wenigstens ein mono- und / oder ein multifunktionelles (Meth)acrylat-Schreibmonomere umfassen

oder daraus bestehen. Ganz besonders bevorzugt kann das Schreibmonomer wenigstens ein mono- und / oder ein multifunktionelles Urethan-(meth)acrylat umfassen oder daraus bestehen.

Geeignete Acrylat-Schreibmonomere sind insbesondere Verbindungen der allgemeinen Formel (V)

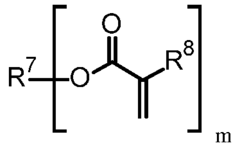

(V)

bei denen m≥1 und m≤4 ist und $R^7$ ein linearer, verzweigter, cyclischer oder heterocyclischer unsubstituierter oder gegebenenfalls auch mit Heteroatomen substituierter organischer Rest und / oder $R^8$ Wasserstoff, ein linearer, verzweigter, cyclischer oder heterocyclischer unsubstituierter oder gegebenenfalls auch mit Heteroatomen substituierter organischer Rest ist. Besonders bevorzugt ist $R^8$ Wasserstoff oder Methyl und / oder $R^7$ ein linearer, verzweigter, cyclischer oder heterocyclischer unsubstituierter oder gegebenenfalls auch mit Heteroatomen substituierter organischer Rest.

Als Acrylate bzw. Methacrylate werden vorliegend Ester der Acrylsäure bzw. Methacrylsäure bezeichnet. Beispiele bevorzugt verwendbarer Acrylate und Methacrylate sind Phenylacrylat, Phenylmethacrylat, Phenoxyethylacrylat, Phenoxyethylmethacrylat, Phenoxyethoxyethylacrylat, Phenoxyethoxyethylmethacrylat, Phenylthioethylacrylat, Phenylthioethylmethacrylat, 2-Naphthylacrylat, 2-Naphthylmethacrylat, 1,4-Bis-(2-thionaphthyl)-2-butylacrylat, 1,4-Bis-(2-thionaphthyl)-2-butylmethacrylat, Bisphenol A Diacrylat, Bisphenol A Dimethacrylat, sowie deren ethoxylierte Analogverbindungen oder N-Carbazolylacrylate.

Als Urethanacrylate werden vorliegend Verbindungen mit mindestens einer Acrylsäureestergruppe und mindestens eine Urethanbindung verstanden. Solche Verbindungen können beispielsweise durch Umsetzung eines Hydroxy-funktionellen Acrylats oder Methacrylats mit einer Isocyanatfunktionellen Verbindung erhalten werden.

Beispiele hierfür verwendbarer Isocyanat-funktionelle Verbindungen sind Monoisocyanate sowie die unter a) genannten monomeren Diisocyanate, Triisocyanate und / oder Polyisocyanate. Beispiele geeigneter Monoisocyanate sind Phenylisocyanat, die isomeren Methylthiophenylisocyanate. Di-, Tri- oder Polyisocyanate sind oben genannt sowie Triphenylmethan-4,4',4"-triisocyanat und Tris-(p-isocyanatophenyl)thiophosphat oder deren Derivate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur und Mischungen derselben. Bevorzugt sind dabei aromatische Di-, Tri- oder Polyisocyanate.

Als hydroxyfunktionelle Acrylate oder Methacrylate für die Herstellung von Urethanacrylaten kommen beispielsweise Verbindungen wie 2-Hydroxyethyl(meth)acrylat, Polyethylenoxid-mono-(meth)acrylate, Polypropylenoxidmono(meth)acrylate, Polyalkylenoxidmono(meth)-acrylate, Poly-(ε-caprolacton)mono(meth)acrylate, wie z.B. Tone® M100 (Dow, Schwalbach, DE), 2-Hydroxypropyl(meth)acrylat, 4-Hydroxybutyl(meth)acrylat, 3-Hydroxy-2,2-dimethylpropyl-(meth)acrylat, Hydroxypropyl(meth)acrylat, Acrylsäure-(2-hydroxy-3-phenoxypropylester), die hydroxyfunktionellen Mono-, Di- oder Tetraacrylate mehrwertiger Alkohole wie Trimethylolpropan, Glycerin, Pentaerythrit, Dipentaerythrit, ethoxyliertes, propoxyliertes oder alkoxyliertes Trimethylolpropan, Glycerin, Pentaerythrit, Dipentaerythrit oder deren technische Gemische in Betracht. Bevorzugt sind 2-Hydroxyethylacrylat, Hydroxypropylacrylat, 4-Hydroxybutylacrylat und Poly(ε-caprolacton)mono(meth)acrylat.

Ebenfalls verwendet werden können die an sich bekannten hydroxylgruppenhaltigen Epoxy(meth)-acrylate mit OH-Gehalten von 20 bis 300 mg KOH/g oder hydroxylgruppenhaltige Polyurethan-(meth)acrylate mit OH-Gehalten von 20 bis 300 mg KOH/g oder acrylierte Polyacrylate mit OH-Gehalten von 20 bis 300 mg KOH/g sowie deren Mischungen untereinander und Mischungen mit hydroxylgruppenhaltigen ungesättigten Polyestern sowie Mischungen mit Polyester(meth)acrylaten oder Mischungen hydroxylgruppenhaltiger ungesättigter Polyester mit Polyester(meth)acrylaten. Bevorzugt sind insbesondere Urethanacrylate erhältlich aus der Umsetzung von Tris(p-isocyanatophenyl)thiophosphat und / oder m-Methylthiophenylisocyanat mit alkoholfunktionellen Acrylaten wie Hydroxyethyl(meth)acrylat, Hydroxypropyl(meth)acrylat und / oder Hydroxybutyl(meth)-acrylat.

Ebenso ist es möglich, dass das Schreibmonomer weitere ungesättigte Verbindungen wie α,β-ungesättigte Carbonsäurederivate wie beispielsweise Maleinate, Fumarate, Maleimide, Acrylamide, weiterhin Vinylether, Propenylether, Allylether und Dicyclopentadienyl-Einheiten enthaltende Verbindungen sowie olefinisch ungesättigte Verbindungen wie z.B. Styrol, α-Methylstyrol, Vinyltoluol und / oder Olefine, umfasst oder daraus besteht.

Photoinitiatoren der Komponente d) sind üblicherweise durch aktinische Strahlung aktivierbare Verbindungen, die eine Polymerisation der Schreibmonomere auslösen können. Bei den Photoinitiatoren kann zwischen unimolekularen (Typ I) und bimolekularen (Typ II) Initiatoren unterschieden werden. Des Weiteren werden sie je nach ihrer chemischen Natur in Photoinitiatoren für radikalische, anionische, kationische oder gemischte Art der Polymerisation unterschieden.

Typ I-Photoinitiatoren (Norrish-Typ-I) für die radikalische Photopolymerisation bilden beim Bestrahlen durch

eine unimolekulare Bindungsspaltung freie Radikale. Beispiele für Typ I-Photoinitiatoren sind Triazine, Oxime, Benzoinether, Benzilketale, Bis-imidazole, Aroylphosphinoxide, Sulfonium- und Iodoniumsalze.

Typ II-Photoinitiatoren (Norrish-Typ-II) für die radikalische Polymerisation bestehen aus einem Farbstoff als Sensibilisator und einem Coinitiator und durchlaufen bei der Bestrahlung mit auf den Farbstoff angepasstem Licht eine bimolekulare Reaktion. Zunächst absorbiert der Farbstoff ein Photon und überträgt aus einem angeregten Zustand Energie auf den Coinitiator. Dieser setzt durch Elektronen- oder Protonentransfer oder direkte Wasserstoffabstraktion die polymerisationsauslösenden Radikale frei.

Im Sinne dieser Erfindung werden bevorzugt Typ II-Photoinitiatoren verwendet.

Solche Photoinitiatorsysteme sind prinzipiell in der EP 0 223 587 A beschrieben und bestehen bevorzugt aus einer Mischung von einem oder mehreren Farbstoffen mit den erfindungsgemäßen Verbindungen der Formel (IV).

Geeignete Farbstoffe, die zusammen mit einer Verbindung der Formel (IV) einen Typ II-Photoinitiator bilden, sind die in der WO 2012062655 beschriebenen kationischen Farbstoffe in Kombination mit den eben dort beschriebenen Anionen.

Unter kationischen Farbstoffen werden bevorzugt solche der folgenden Klassen verstanden: Acridin-Farbstoffe, Xanthen-Farbstoffe, Thioxanthen-Farbstoffe, Phenazin-Farbstoffe, Phenoxazin-Farbstoffe, Phenothiazin-Farbstoffe, Tri(het)arylmethan-Farbstoffe - insbesondere Diamino- und Triamino(het)arylmethan-Farbstoffe, Mono-, Di-, Tri- und Pentamethincyanin-Farbstoffe, Hemicyanin-Farbstoffe, extern kationische Merocyanin-Farbstoffe, extern kationische Neutrocyanin-Farbstoffe, Nullmethin-Farbstoffe - insbesondere Naphtholactam-Farbstoffe, Streptocyanin-Farbstoffe. Solche Farbstoffe sind beispielsweise in H. Berneth in Ullmann's Encyclopedia of Industrial Chemistry, Azine Dyes, Wiley-VCH Verlag, 2008, H. Berneth in Ullmann's Encyclopedia of Industrial Chemistry, Methine Dyes and Pigments, Wiley-VCH Verlag, 2008, T. Gessner, U. Mayer in Ullmann's Encyclopedia of Industrial Chemistry, Triarylmethane and Diarylmethane Dyes, Wiley-VCH Verlag, 2000 beschrieben.

Besonders bevorzugt sind Phenazin-Farbstoffe, Phenoxazin-Farbstoffe, Phenothiazin-Farbstoffe, Tri(het)arylmethan-Farbstoffe - insbesondere Diamino- und Triamino(het)arylmethan-Farbstoffe, Mono-, Di-, Tri- und Pentamethincyanin-Farbstoffe, Hemicyanin-Farbstoffe, Nullmethin-Farbstoffe - insbesondere Naphtholactam-Farbstoffe, Streptocyanin-Farbstoffe.

Beispiele für kationische Farbstoffe sind Astrazon Orange G, Basic Blue 3, Basic Orange 22, Basic Red 13, Basic Violett 7, Methylenblau, Neu Methylenblau, Azur A, 2,4-Diphenyl-6-(4-methoxyphenyl)pyrylium, Safranin O, Astraphloxin, Brilliant Grün, Kristallviolett, Ethylviolett und Thionin.

Bevorzugte Anionen sind insbesondere $C_8$- bis $C_{25}$-Alkansulfonat, vorzugsweise $C_{13}$- bis $C_{25}$-Alkansulfonat, $C_3$- bis $C_{18}$-Perfluoralkansulfonat, $C_4$- bis $C_{18}$-Perfluoralkansulfonat, das in der Alkylkette mindestens 3 Wasserstoffatome trägt, $C_9$- bis $C_{25}$-Alkanoat, $C_9$- bis $C_{25}$-Alkenoat, $C_8$- bis $C_{25}$-Alkylsulfat, vorzugsweise $C_{13}$- bis $C_{25}$-Alkylsulfat, $C_8$- bis $C_{25}$-Alkenylsulfat, vorzugsweise $C_{13}$- bis $C_{25}$-Alkenylsulfat, $C_3$- bis $C_{18}$-Perfluoralkylsulfat, $C_4$- bis $C_{18}$-Perfluoralkylsulfat, das in der Alkylkette mindestens 3 Wasserstoffatome trägt, Polyethersulfate basierend auf mindestens 4 Äquivalenten Ethylenoxid und / oder 4 Äquivalenten Propylenoxid, Bis-$C_4$- bis $C_{25}$-Alkyl-, $C_5$- bis $C_7$-Cycloalkyl-, $C_3$- bis $C_8$-Alkenyl- oder $C_7$- bis $C_{11}$-Aralkyl-sulfosuccinat, durch mindestens 8 Fluoratome substituiertes Bis-$C_2$-bis $C_{10}$-alkyl-sulfosuccinat, $C_8$- bis $C_{25}$-Alkyl-sulfoacetate, durch mindestens einen Rest der Gruppe Halogen, $C_4$- bis $C_{25}$-Alkyl, Perfluor-$C_1$- bis $C_8$-Alkyl und / oder $C_1$- bis $C_{12}$-Alkoxycarbonyl substituiertes Benzolsulfonat, ggf. durch Nitro, Cyano, Hydroxy, $C_1$- bis $C_{25}$-Alkyl, $C_1$- bis $C_{12}$-Alkoxy, Amino, $C_1$- bis $C_{12}$-Alkoxycarbonyl oder Chlor substituiertes Naphthalin- oder Biphenylsulfonat, ggf. durch Nitro, Cyano, Hydroxy, $C_1$- bis $C_{25}$-Alkyl, $C_1$- bis $C_{12}$-Alkoxy, $C_1$- bis $C_{12}$-Alkoxycarbonyl oder Chlor substituiertes Benzol-, Naphthalin- oder Biphenyldisulfonat, durch Dinitro, $C_6$- bis $C_{25}$-Alkyl, $C_4$- bis $C_{12}$-Alkoxycarbonyl, Benzoyl, Chlorbenzoyl oder Toluoyl substituiertes Benzoat, das Anion der Naphthalindicarbonsäure, Diphenyletherdisulfonat, sulfonierte oder sulfatierte, ggf. mindestens einfach ungesättigte $C_8$- bis $C_{25}$-Fettsäureester von aliphatischen $C_1$- bis $C_8$-Alkoholen oder Glycerin, Bis-(sulfo-$C_2$- bis $C_6$-alkyl)-$C_3$- bis $C_{12}$-alkandicarbonsäureester, Bis-(sulfo-$C_2$- bis $C_6$-alkyl)-itaconsäureester, (Sulfo-$C_2$- bis $C_6$-alkyl)-$C_6$- bis $C_{18}$-alkancarbonsäureester, (Sulfo-$C_2$- bis $C_6$-alkyl)-acryl- oder methacrylsäureester, ggf. durch bis zu 12 Halogenreste substituiertes Triscatecholphosphat, ein Anion der Gruppe Tetraphenylborat, Cyanotriphenylborat, Tetraphenoxyborat, $C_4$- bis $C_{12}$-Alkyl-triphenylborat, deren Phenyl- oder Phenoxy-Reste durch Halogen, $C_1$- bis $C_4$-Alkyl und / oder $C_1$- bis $C_4$-Alkoxy substituiert sein können, $C_4$- bis $C_{12}$-Alkyl-trinaphthylborat, Tetra-$C_1$- bis $C_{20}$-alkoxyborat, 7,8- oder 7,9-Dicarba-nido-undecaborat(1-) oder (2-), die gegebenenfalls an den B- und / oder C-Atomen durch eine oder zwei $C_1$- bis $C_{12}$-Alkyl- oder Phenyl-Gruppen substituiert sind, Dodecahydrodicarbadodecaborat(2-) oder B-$C_1$- bis $C_{12}$-Alkyl-C-phenyl-dodecahydro-dicarbadodecaborat(1-) steht, wobei bei mehrwertigen Anionen wie Naphthalindisulfonat $A^-$ für ein Äquivalent dieses Anions steht, und wobei die Alkan- und Alkylgruppen verzweigt sein können und / oder durch Halogen, Cyano, Methoxy, Ethoxy, Methoxycarbonyl oder Ethoxycarbonyl substituiert sein können.

Bevorzugt ist auch, wenn das Anion $A^-$ des Farbstoffs einen AClogP im Bereich von 1 bis 30, besonders bevorzugt im Bereich von 1 bis 12 und insbesondere bevorzugt im Bereich von 1 bis 6,5 aufweist. Der AClogP wird nach J. Comput. Aid. Mol. Des. 2005, 19, 453; Virtual Computational Chemistry Laboratory, http://www.vcclab.org berechnet.

Das Photoinitiatorsystem kann außerdem einen weiteren Co-Initiator enthalten wie beispielsweise Trichloromethylinitiatoren, Aryloxidinitiatoren, Bisimidazolinitiatoren, Ferroceninitiatoren, Aminoalkylinitiatoren, Oximinitiatoren, Thiolinitiatoren oder Peroxidinitiatoren.

Auch offenbart ist, dass die Photopolymer-Formulierung zusätzlich Urethane als Additive enthält, wobei die Urethane insbesondere mit wenigstens einem Fluoratom substituiert sein können.

Bevorzugt können die Urethane die allgemeine Formel (VI)

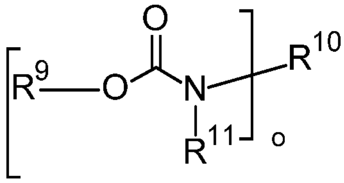

(VI)

haben, in der o≥1 und o≤8 ist und $R^9$, $R^{10}$ und $R^{11}$ lineare, verzweigte, cyclische oder heterocyclische unsubstituierte oder gegebenenfalls auch mit Heteroatomen substituierte organische Reste und / oder $R^{10}$, $R^{11}$ unabhängig voneinander Wasserstoff sind, wobei bevorzugt mindestens einer der Reste $R^9$, $R^{10}$, $R^{11}$ mit wenigstens einem Fluoratom substituiert ist und besonders bevorzugt $R^9$ ein organischer Rest mit mindestens einem Fluoratom ist. Besonders bevorzugt ist $R^{11}$ ein linearer, verzweigter, cyclischer oder heterocyclischer unsubstituierter oder gegebenenfalls auch mit Heteroatomen wie beispielsweise Fluor substituierter organischer Rest.

Auch offenbart ist ein Photopolymer enthaltend eine Photopolymer-Formulierung, insbesondere umfassend Matrixpolymere, ein Schreibmonomer und einen Photoinitiatorsystem, das zusätzlich eine Verbindung der Formel (IV) erhältlich oder erhalten nach dem erfindungsgemäßen Verfahren oder eine Verbindung der Formel (C) umfasst.

Die oben zu der Photopolymer-Formulierung getroffenen Aussagen bezüglich weiterer Ausführungsformen gelten analog auch für das Photopolymer.

Ebenfalls zugänglich wird ein holographisches Medium insbesondere in Form eines Films, enthaltend ein Photopolymer oder erhältlich unter Verwendung einer Photopolymer-Formulierung. Außerdem ist die Photopolymerzusammensetzung verwendbar zur Herstellung holographischer Medien.

In solche holographische Medien können holographische Informationen einbelichtet werden.

Die holographischen Medien können durch entsprechende Belichtungsprozesse für optische Anwendungen im gesamten sichtbaren und nahen UV-Bereich (300-800 nm) zu Hologrammen verarbeitet werden. Visuelle Hologramme umfassen alle Hologramme, die nach dem Fachmann bekannten Verfahren aufgezeichnet werden können. Darunter fallen unter anderem In-Line (Gabor) Hologramme, Off-Axis Hologramme, Full-Aperture Transfer Hologramme, Weißlicht-Transmissionshologramme ("Regenbogenhologramme), Denisyukhologramme, Off-Axis Reflektionshologramme, Edge-Lit Hologramme sowie holographische Stereogramme. Bevorzugt sind Reflektionshologramme, Denisyukhologramme, Transmissionshologramme.

Mögliche optische Funktionen der Hologramme, die mit den Photopolymer-Formulierungen hergestellt werden können entsprechen den optische Funktionen von Lichtelementen wie Linsen, Spiegel, Umlenkspiegel, Filter, Streuscheiben, Beugungselemente, Diffusoren, Lichtleiter, Lichtlenker (waveguides), Projektionsscheiben und / oder Masken. Ebenfalls können Kombinationen aus diesen optischen Funktionen unabhängig voneinander in einem Hologramm vereinigt sein. Häufig zeigen diese optischen Elemente eine Frequenzselektivität, je nachdem wie die Hologramme belichtet wurden und welche Dimensionen das Hologramm hat.

Zudem können mittels der holographischen Medien auch holographische Bilder oder Darstellungen hergestellt werden, wie zum Beispiel für persönliche Portraits, biometrische Darstellungen in Sicherheitsdokumenten, oder allgemein von Bilder oder Bildstrukturen für Werbung, Sicherheitslabels, Markenschutz, Markenbranding, Etiketten, Designelementen, Dekorationen, Illustrationen, Sammelkarten, Bilder und dergleichen sowie Bilder, die digitale Daten repräsentieren können u.a. auch in Kombination mit den zuvor dargestellten Produkten. Holographische Bilder können den Eindruck eines dreidimensionalen Bildes haben, sie können aber auch Bildsequenzen, kurze Filme oder eine Anzahl von verschiedenen Objekten darstellen, je nachdem aus welchem Winkel, mit welcher (auch bewegten) Lichtquelle etc. diese beleuchtet wird. Aufgrund dieser vielfältigen Design-Möglichkeiten stellen Hologramme, insbesondere Volumenhologramme, eine attraktive technische Lösung für die oben genannten Anwendung dar.

Die holographischen Medien können zur Aufzeichnung von In-Line, Off-Axis, Full-Aperture Transfer, Weißlicht-Transmissions, Denisyuk, Off-Axis Reflektions oder Edge-Lit Hologrammen sowie holographischen Stereogrammen, insbesondere zur Herstellung von optischen Elementen, Bildern oder Bilddarstellungen verwendet werden.

Hologramme werden aus holographischen Medien zugänglich.

Die folgenden Beispiele dienen der beispielhaften Erläuterung der Erfindung, ohne sie auf diese zu beschränken.

**Beispiele**

Messmethoden:

| | |
|---|---|
| OH Zahl: | Die angegebenen OH-Zahlen wurden gemäß DIN 53240-2 bestimmt. |
| NCO-Wert: | Die angegebenen NCO-Werte (Isocyanat-Gehalte) wurden gemäß DIN EN ISO 11909 bestimmt. |
| Festkörpergehalt: | Die angegebenen Festkörpergehalte wurden gemäß DIN EN ISO 3251 bestimmt. |
| Brechungsindexmodulation Δn: | Die holographische Eigenschaften Δn der holographischen Medien wurden mittels Zweistrahlinterferenz in Reflexionsanordnung wie in WO2015091427 beschrieben bestimmt, ein repräsentatives Ergebnis ist in Figur 1 abgebildet. |

**Substanzen:**

Die verwendeten Lösungsmittel, Reagenzien sowie alle Bromaromaten wurden im Chemikalienhandel bezogen. Wasserfreie Lösungsmittel enthalten < 50 ppm Wasser.

| | |
|---|---|
| Ethylboronsäure-Pinacolester | [82954-89-0] ist bei der TCI Europe N.V., Zwijndrecht, Belgien erhältlich. |
| Isopropylboronsäure-Pinacolester | [76347-13-2] ist bei der ABCR GmbH & Co. KG, Karlsruhe, Deutschland erhältlich. |
| 2-Isopropenylboronsäure-Pinacolester | [126726-62-3] ist bei der ABCR GmbH & Co. KG, Karlsruhe, Deutschland erhältlich. |
| 1-Dodecylboronsäure-Pinacolester | [177035-82-4] ist bei der ABCR GmbH & Co. KG, Karlsruhe, Deutschland erhältlich. |
| 3-Phenyl-1-propylboron- säure-Pinacolester | [329685-40-7] ist bei der ABCR GmbH & Co. KG, Karlsruhe, Deutschland erhältlich. |
| Diisopropylallylboronat | [51851-79-7] ist bei der ABCR GmbH & Co. KG, Karlsruhe, Deutschland erhältlich. |
| (1,3,2-Dioxaborinan-2-yl)cyclohexan | [30169-75-6] ist bei der ABCR GmbH & Co. KG, Karlsruhe, Deutschland erhältlich. |
| Dibromboran-Dimethyl- sulfid-Komplex | [55671-55-1] ist bei der Aldrich Chemie, Steinheim, Deutschland erhältlich. |
| 1-Octadecen | [112-41-4] ist bei der ABCR GmbH & Co. KG, Karlsruhe, Deutschland erhältlich. |
| Desmorapid Z | Dibutylzinn-dilaurat [77-58-7], Produkt der Covestro AG, Leverkusen, Deutschland. |

| | |
|---|---|
| Desmodur® N 3900 | Produkt der Covestro AG, Leverkusen, DE, He-xandiisocyanat-basiertes Polyisocyanat, Anteil an Imino¬oxa-diazindion mindestens 30 %, NCO-Gehalt: 23.5 %. |
| Fomrez UL 28 | Urethanisierungskatalysator, Handelsprodukt der Momentive Performance Chemicals, Wilton, CT, USA. |
| Lewatit® MDS TP 208 | ist bei der Lanxess Deutschland GmbH, Köln, Deutschland erhältlich. |

2-Hexyl-1,3,2-dioxaborinan [86290-24-6] wurde wie in Organometallics 1983, 2 (10), S. 1311-16, DOI:10.1021/om5.0004a008 beschrieben aus 1-Hexen, 1,3-Propandiol und Dibromboran-Dimethylsulfid-Komplex hergestellt.

2-Octadecyl-4,4,5,5-tetramethyl-1,3,2-dioxaborolan wurde analog zu Organometallics 1983, 2 (10), S. 1311-16, DOI:10.1021/om5.0004a008 aus 1-Octadecen, 1,3-Propandiol und Dibromboran-Dimethylsulfid-Komplex hergestellt.

Farbstoff 1 (3,7-Bis(diethylamino)-phenoxazin-5-ium bis(2-ethylhexyl)sulfobernsteinsäureester) wurde wie in WO 2012062655 beschrieben hergestellt.

Polyol 1 wurde wie in Polyol 1 in der WO2015091427 beschrieben hergestellt.

Urethanacrylat 1, (Phosphorothioyltris(oxybenzol-4,1-diylcarbamoyloxyethan-2,1-diyl)trisacrylat, [1072454-85-3]) wurde wie in WO2015091427 beschrieben hergestellt.

Urethanacrylat 2, (2-({[3-(Methylsulfanyl)phenyl]carbamoyl}oxy)-ethylprop-2-enoat, [1207339-61-4]) wurde wie in WO2015091427 beschrieben hergestellt.

Additiv 1, Bis(2,2,3,3,4,4,5,5,6,6,7,7-dodecafluorheptyl)-(2,2,4-trimethylhexan-1,6-diyl)biscarbamat [1799437-41-4] wurde wie in WO2015091427 beschrieben hergestellt.

**Herkunft der verwendeten Kationen 1/n $K^{n+}$:**

Die in der Tabelle 1 genannten Kationen 1/n $K^{n+}$ wurden von dem genannten Chemikalienhändler bezogen oder gemäß der genannten Quelle hergestellt oder die Herstellung ist nachfolgend beschrieben.

Tabelle 1: Übersicht der verwendeten Kationen der Formel (II)

| **Index** | **Name** | **Struktur** | CAS | **Quelle** |
|---|---|---|---|---|
| K-1 | Tetramethylammonium bromid | $Me_4NBr$ | [64-20-0] | 426296, Aldrich |
| K-2 | Tetrabutylammonium bromid | $Bu_4NBr$ | [1643-19-2] | 426288, Aldrich |
| K-3 | Benzyldimethylhexadecylammonium chlorid | $BnMe_2HexadecylNCl$ | [122-18-9] | AB252026, abcr GmbH |
| K-4 | Hexadecyltrimethylammonium bromid | 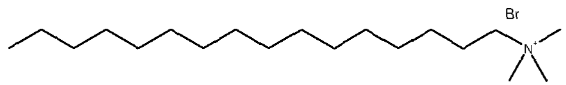 | [57-09-0] | AB117004, abcr GmbH |
| K-5 | Benzethonium chlorid | 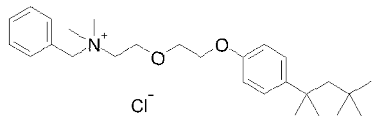 | [121-54-0] | AB 131627, abcr GmbH |
| K-6 | 1-Hexadecyl-3-methylimidazolium chlorid | 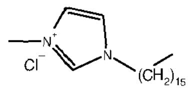 | [61546-01-8] | AB289677, abcr GmbH |
| K-7 | 1-Methyl-3-octylimidazolium chlorid | 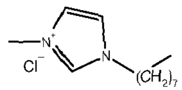 | [64697-40-1] | AB289637, abcr GmbH |
| K-8 | 1-Dodecyl-3-methylimidazolium chlorid | 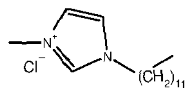 | [114569-84-5] | AB289681, abcr GmbH |
| K-9 | 1-Ethyl-3-imidazolium chlorid | 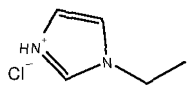 | [81505-35-3] | AB289800, abcr GmbH |
| K-10 | 1-Benzyl-3-hexadecyl-imidazolium bromid | 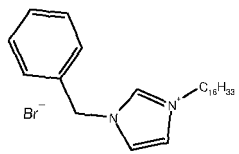 | [1224595-52-1] | Fuel Processing Technology (2014), 118, 296-301. |

(fortgesetzt)

| **Index** | **Name** | **Struktur** | CAS | **Quelle** |
|---|---|---|---|---|
| K-11 | N-Hexadecylpyridinium chloride monohydrat | 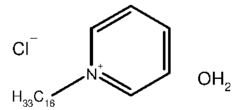 | [6004-24-6] | AB 117002, abcr GmbH |
| K-12 | N-Docosylpyridinium bromid | 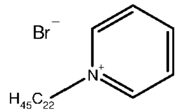 | [80039-83-4] | J. Am. Chem. Soc. (2002), 124 (11), 2604-2613. |
| K-13 | 4-tert.-Butyl-1-hexadecyl-pyridinium chlorid | 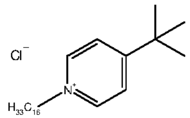 | [1702465-32-4] | WO 2015055576 A1 |
| K-14 | 1,1'-[1,3-Phenylenebis(methylen)] bis-pyridinium dichlorid | 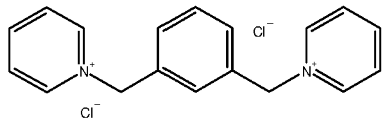 | [84002-71-1] | Zhurnal Neorganicheskoi Khimii (1978), 23, 825-6. |
| K-15 | N,N,N,N',N',N'-Hexaethyl- 1,3-benzendimethan aminium dibromid | 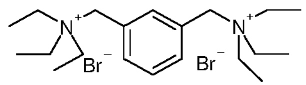 | [66753-59-1P] | Chemische Berichte (1984), 117 (4), 1487-96. |
| K-16 | N,N-Dioctadecyl-piperidinium chlorid | 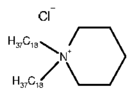 | [61550-95-6] | J. Am. Chem. Soc. (1955), 77, 485-6. |
| K-17 | N-Hexadecyl-N,N-dimethyl-anilinium bromid | 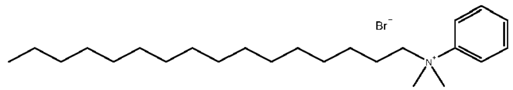 | [17695-00-0] | Taiwan Kexue (1959), 13, 95-8. |
| K-18 | N,N,N,N',N',N'-Hexabutyl-hexamethylen-diammonium dibromid | 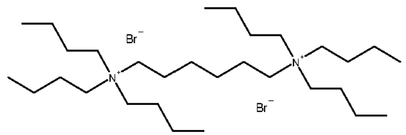 | [745829-82-7P] | Chemical Engineering Science, 69 (1), 483-491. |

(fortgesetzt)

| Index | Name | Struktur | CAS | Quelle |
|---|---|---|---|---|
| K-19 | N-(2-(Benzoyloxy)ethyl)-N,N-dimethyloctadecan-1-aminium bromid | 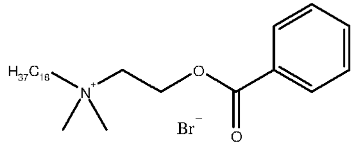 | [152167-30-1] | US 5194472 A |
| K-20 | N-(2-((2-Ethylhexanoyl)oxy)ethyl)-N,N-dimethylhexadecan-1-aminium bromid | 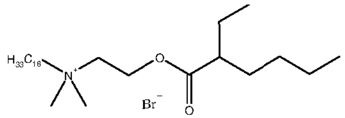 | | Herstellvorschrift siehe unten |
| K-21 | N-Benzyl-N,N-dimethyl-2-(2-(palmitoyloxy)ethoxy) ethan-1-aminium bromid | 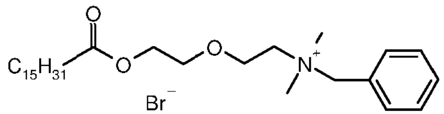 | | Herstellvorschrift siehe unten |
| K-22 | 2-(Benzoyloxy)-N,N-dimethyl-N-(2-(palmitoyloxy) ethyl)ethan-1-aminium bromid | 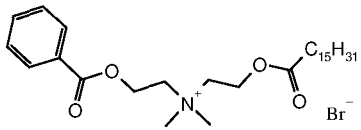 | | Herstellvorschrift siehe unten |
| K-23 | N-(3-((2-Ethylhexyl)oxy)-3-oxopropyl)-N,N-dimethyloctadecan-1-aminium bromid | 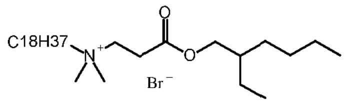 | | Herstellvorschrift siehe unten |
| K-24 | N-(2-((Hexylcarbamoyl)oxy)ethyl)-N,N-dimethylhexadecan-1-aminium bromid | 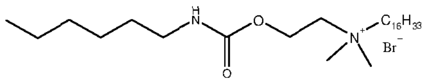 | | Herstellvorschrift siehe unten |
| K-25 | $N^1$,$N^{16}$-Dihexadecyl-$N^1$,$N^1$,$N^{16}N^{16}$,7,7,10-heptamethyl-4,13-dioxo-3,14-dioxa-5,12-diazahexadecan-1,16-diaminium dibromid | 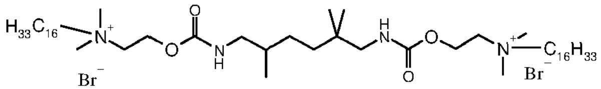 | | Herstellvorschrift siehe unten |
| K-26 | N-(2-((((5-(((2-(Hexadecyldimethylammonio) ethoxy)-carbonyl)amino)-1,3,3-trimethylcyclohexyl) methyl)-carbamoyl)oxy)ethyl)-N,N-dimethylhexadecan-1-aminium dibromid | 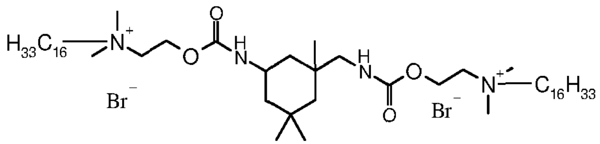 | | Herstellvorschrift siehe unten |

(fortgesetzt)

| Index | Name | Struktur | CAS | Quelle |
| --- | --- | --- | --- | --- |
| K-27 | N,N'-(((((Methylenebis(cyclohexan-4,1-diyl))bis (aza-nediyl))bis(carbonyl))bis(oxy))bis(ethane-2,1-diyl))-bis(N,N-dimethylhexadecan-1-aminium) dibromid | 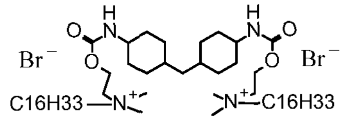 | | Herstellvorschrift siehe unten |
| K-28 | N,N'-(((((4-Methyl-1,3-phenylen)bis(azanediyl))-bis (carbonyl))bis(oxy))bis(ethan-2,1-diyl))bis(N,N-dimethylhexadecan-1-aminium) dibromid | 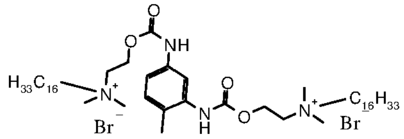 | [1073535-73-5] | Herstellvorschrift siehe unten |
| K-29 | N,N'-(((((Methylenbis(4,1-phenylen))bis (azanediyl))-bis(carbonyl))bis(oxy))bis(ethan-2,1-diyl))bis(N,N-dimethylhexadecan-1-aminium) dibromid | 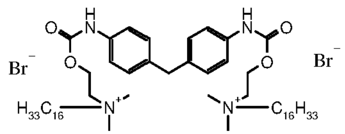 | | Herstellvorschrift siehe unten |
| K-30 | N-(2-(2-((Hexylcarbamoyl)oxy)ethoxy)ethyl)-N,N-dimethylhexadecan-1-aminium bromid | 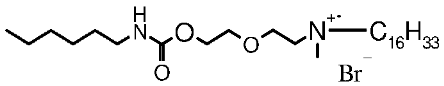 | | Herstellvorschrift siehe unten |
| K-31 | $N^1$ ,$N^{22}$-Dihexadecyl-$N^1$,$N^1$ ,$N^{22}$,$N^{22}$,10,10, 13-heptamethyl-7,16-dioxo-3,6,17,20-tetraoxa-8,15-diazadocosan-1,22-diaminium dibromid | 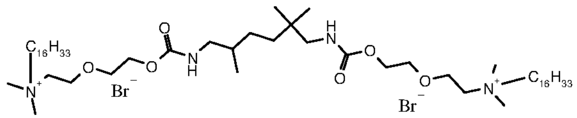 | | Herstellvorschrift siehe unten |
| K-32 | N-(2-(2-(((3-(10,10-Dimethyl-3-oxo-4,7-dioxa-2,10-diazahexacosan-10-ium-1-yl)-3,5,5-trimethyl-cyclohexyl)carbamoyl)oxy)ethoxy)ethyl)-N,N-dimethylhexadecan-1-aminium dibromid | 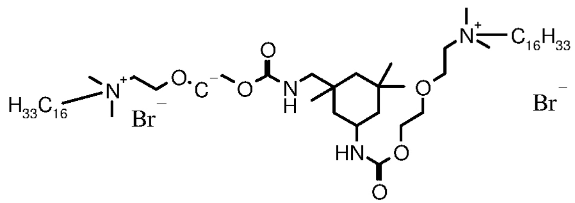 | | Herstellvorschrift siehe unten |
| K-33 | N,N'-(((((((Methylenbis(cyclohexan-4,1-diyl))bis (azanediyl))bis(carbonyl))bis(oxy))bis(ethan-2,1-diyl))bis(oxy))bis(ethan-2,1-diyl))bis(N,N-dimethylhexadecan-1-aminium) dibromid | 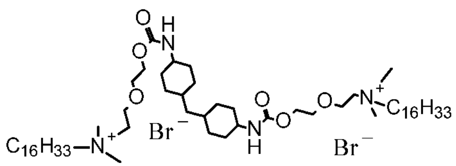 | | Herstellvorschrift siehe unten |

(fortgesetzt)

| Index | Name | Struktur | CAS | Quelle |
|---|---|---|---|---|
| K-34 | N,N'-(((((((4-Methyl-1,3-phenylen)bis(azanediyl)) bis-(carbonyl))bis(oxy))bis(ethan-2,1-diyl))bis(oxy)) bis(ethan-2,1-diyl))bis(N,N-dimethylhexadecan-1-aminium) dibromid | 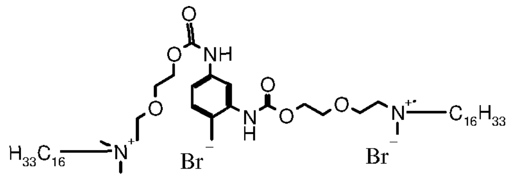 | | Herstellvorschrift siehe unten |
| K-35 | N,N'-(((((((Methylenbis(4,1-phenylen))bis (azandiyl))-bis(carbonyl))bis(oxy))bis(ethan-2,1-diyl))bis-(oxy))bis(ethan-2,1-diyl))bis(N,N-dimethylhexadecan-1-aminium) dibromid | 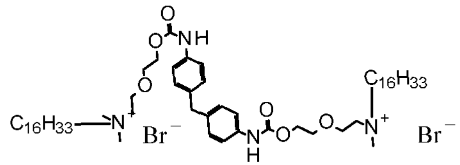 | | Herstellvorschrift siehe unten |
| K-36 | *N*[1],*N*[1],*N*[1],*N*[3],*N*[3],*N*[3],*N*[5],*N*[5],*N*[5]-Nonamethyl-1,3,5-benzentrimethanaminium triiodid | 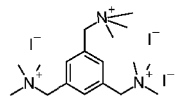 | [88888-13-5] | Chem. Ber. 117, 1487-1496 (1984). |
| K-37 | *N*-[2-[2-[2-(Benzoyloxy)ethoxy]ethoxy]ethyl]-*N*,*N*-dimethyl-octadecan-1-amminium bromid | 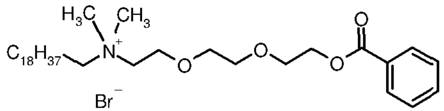 | [215591-20-1] | SK 278487 |
| K-38 | 1-[3-[(2-Ethylhexyl)oxy]-3-oxopropyl]-4-methyl-pyridinium chlorid | 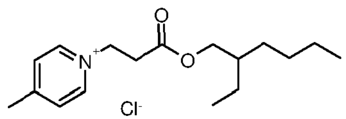 | [110250-87-8] | US 2857310 |
| K-39 | 4-Methyl-1-[2-[(1-oxotetradecyl)oxy] ethyl]-pyridinium chlorid | 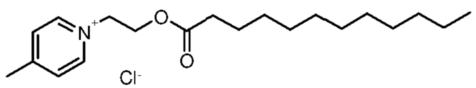 | [42936-94-7] | Izvestiya Vysshikh Uchebnykh Zavedenii, Khimiya i Khimicheskaya Tekhnologiya (1973), 16(6), 891. |

(fortgesetzt)

| Index | Name | Struktur | CAS | Quelle |
|---|---|---|---|---|
| K-40 | 1-[[3-(4-Metboxyphenyl)-2-oxo-5-oxazolidinyl] methyl-4-phenyl-pyridinium bromid | 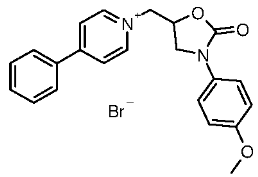 | [121082-85-7] | DE 3723797 |
| K-41 | 4-Methyl-1-[2-[(1-oxododecyl)amino] ethyl]-pyridinium chlorid | 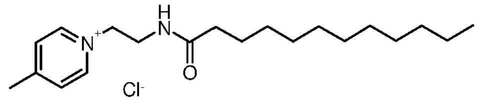 | [15147-43-0] | Chemicky-Prumysl (1967), 17(2), 67-9. |
| K-42 | 4,5-Dihydro-1-methy1-3-[2-[(1-oxotetradecyl) oxy]-ethyl]-2-pentadecyl-1 H-imidazolium chlorid | 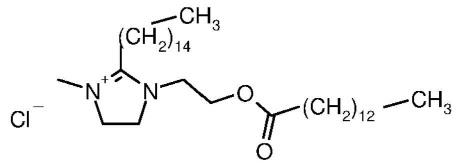 | [131625-89-3] | US 4954635 |
| K-43 | 4,4-Bis[2-[[(9Z)-1-oxo-9-octadecen-1-yl]oxy] ethyl]-morpholinium chlorid | 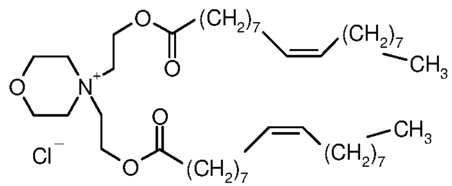 | [272462-96-1] | WO2000030444 |
| K-100 | Tributyltetradecylphosphonium chlorid | 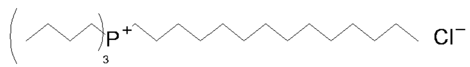 | [81741-28-8] | P444(14) Cl, Ionic Liquids Technologies GmbH |
| K-101 | Trihexyltetradecylphosphonium chlorid | 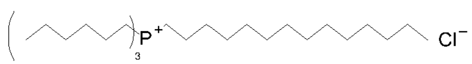 | [258864-54-9] | P666(14) Cl, Ionic Liquids Technologies GmbH |
| K-102 | Methyltriphenylphosphonium chlorid | 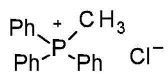 | [1031-15-8] | AB349191, abcr GmbH |
| K-103 | Triphenylbenzylphosphonium chlorid | 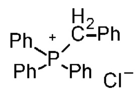 | [1100-88-5] | AB113982, abcr GmbH |

(fortgesetzt)

| Index | Name | Struktur | CAS | Quelle |
|---|---|---|---|---|
| K-104 | Tetraphenylphosphonium chlorid | 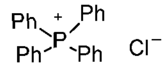 | [2001-45-8] | AB119018, abcr GmbH |
| K-105 | Allyltriphenylphosphonium bromid | 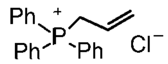 | [1560-54-9] | AB121395, abcr GmbH |
| K-106 | (2-Oxo-2-phenyl-ethyl)-triphenyl-phosphonium bromid | 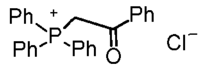 | [6048-29-9] | AB233312, abcr GmbH |
| K-200 | 4-Methyl-2,6-diphenylpyrylium tetrafluoroborat | 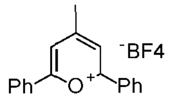 | [2340-23-0] | S919802, Aldrich |
| K-201 | 2,4,6-Triphenyllium tetrafluoroborat | 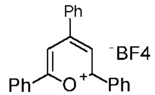 | [448-61-3] | AB 119969, abcr GmbH |
| K-202 | 2,4,6-Trimethylpyrylium tetrafluoroborat | 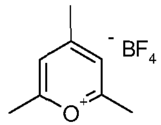 | [773-01-3] | AB 177250, abcr GmbH |
| K-203 | 4,4'-Bis(2,6-diphenylpyrylium tetrafluoroborat) | 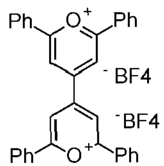 | [42559-29-5] | S873950, Aldrich |
| K-300 | Diphenyl[4-(phenylthio)phenyl]sulfonium hexafluorophosphat | 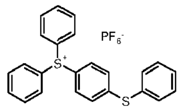 | [75482-18-7] | AB334122, abcr GmbH |
| K-301 | Bis(diphenylsulfonium)diphenylthioether hexafluorophosphat | 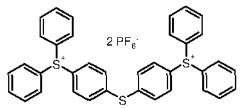 | [74227-35-3] | AB 134315, abcr GmbH |

(fortgesetzt)

| Index | Name | Struktur | CAS | Quelle |
|---|---|---|---|---|
| K-302 | Diphenyl[4-(phenylthio)phenyl]sulfonium triflat | 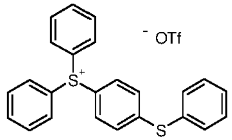 | [111281-12-0] | AB231614, abcr GmbH |
| K-303 | Mischung K-300 + K-301 (Cyracure UVI 6990) | | [104558-95-4] | BASF SE, Ludwigshafen, Germany |
| K-304 | Tris[4-[(4-acetylphenyl)thio]phenyl]sulfonium tris[(trifluoromethyl)sulfonyl] methanid | 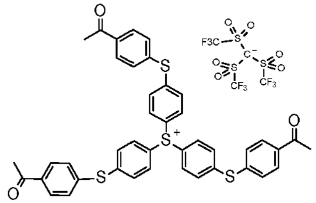 | [953084-20-3] | BASF SE, Ludwigshafen, Germany |
| K-305 | 2,4,6-Triphenyl thiopyrylium perchlorat | 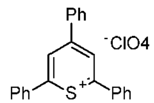 | [2930-37-2] | AKOS001017031, AKos GmbH Austr. 26 Steinen, D-79585 Germany |
| K-400 | (4-Methylphenyl) [4-(2-methylpropyl)phenyl iodonium] hexafluorophosphat | 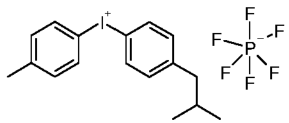 | [344562-80-7] | BASF SE, Ludwigshafen, Germany |
| K-401 | Diphenyliodonium chlorid | 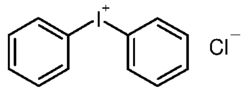 | [1483-72-3] | AB109613, abcr GmbH |
| K-402 | Bis(4-methylphenyl)iodonium hexafluorophosphat | 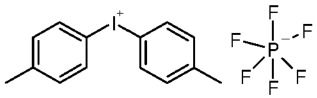 | [60565-88-0] | AB336755, abcr GmbH |
| K-403 | Diphenyleneiodonium chlorid | 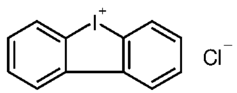 | [4673-26-1] | AB348986, abcr GmbH |

**Herstellung nicht kommerziell erhältlicher Kationen 1/n $K^{n+}$:**

**N-(2-((2-Ethylhexanoyl)oxy)ethyl)-N,N-dimethylhexadecan-1-aminium bromid (K-20)**

5.00 g N,N-Dimethylethanolamin wurden in trockenem Chloroform mit Eisbadkühlung vorgelegt und 9.12 g 2-Ethylhexansäurechlorid wurden vorsichtig zugetropft und 30 min bei Raumtemperatur nachgerührt. 30 mL gesättigte Natriumhydrogencarbonatlösung Lösung wurden zugegeben und die organische Lösung wurde so lange mit gesättigter Natriumhydrogencarbonatlösung extrahiert bis diese Chlorid-frei ist. Anschließend wurde die organische Phase mit 30 mL Wasser gewaschen, die Lösung getrocknet und das Lösungsmittel im Vakuum abdestilliert. Es wurden 10.99 g Aminoester erhalten.

Zu einer Lösung aus 10.99 g Aminoester in 30 mL Acetonitril wurden 15.58 g 1-Bromhexadecan gegeben und für 6 h am Rückfluss erhitzt. Das Lösungsmittel wurde fast vollständig im Vakuum abdestilliert, der ausgefallene Feststoff wurde isoliert und man erhielt 19.33 g eines farblosen klebrigen Harzes.

**N-Benzyl-N,N-dimethyl-2-(2-(palmitoyloxy)ethoxy)ethan-1-aminium bromid (K-21)**

6.00 g N,N-2-[2-(Dimethylamino)ethoxy]ethanol wurden in trockenem Chloroform mit Eisbadkühlung vorgelegt und 12.38 g Hexadecansäurechlorid wurden vorsichtig zugetropft und 30 min bei Raumtemperatur nachgerührt. 30 mL gesättigte Natriumhydrogencarbonatlösung Lösung wurden zugegeben und die organische Lösung wurde so lange mit gesättigter Natriumhydrogencarbonatlösung extrahiert bis diese Chlorid-frei ist. Anschließend wurde die organische Phase mit 30 mL Wasser gewaschen, die Lösung getrocknet und das Lösungsmittel im Vakuum abdestilliert. Es wurden 14.68 g Aminoester erhalten.

Zu einer Lösung aus 14.68 g Aminoester in 30 mL Acetonitril wurden 6.77 g Benzylbromid gegeben und für 6 h am Rückfluss erhitzt. Das Lösungsmittel wurde fast vollständig im Vakuum abdestilliert, der ausgefallene Feststoff wurde isoliert und man erhielt 8.23 g eines farblosen klebrigen Harzes.

**2-(Benzoyloxy)-N,N-dimethyl-N-(2-(palmitoyloxy)ethyl)ethan-1-aininium bromid (K-22)**

6.00 g N,N-2-[2-(Dimethylamino)ethoxy]ethanol wurden in trockenem Chloroform mit Eisbadkühlung vorgelegt und 12.38 g Hexadecansäurechlorid wurden vorsichtig zugetropft und 30 min bei Raumtemperatur nachgerührt. 30 mL gesättigte Natriumhydrogencarbonatlösung Lösung wurden zugegeben und die organische Lösung wurde so lange mit gesättigter Natriumhydrogencarbonatlösung extrahiert bis diese Chlorid-frei ist. Anschließend wurde die organische Phase mit 30 mL Wasser gewaschen, die Lösung getrocknet und das Lösungsmittel im Vakuum abdestilliert. Es wurden 14.68 g Aminoester erhalten.

Zu einer Lösung aus 14.68 g Aminoester in 30 mL Acetonitril wurden 6.77 g Benzylbromid gegeben und für 6 h am Rückfluss erhitzt. Das Lösungsmittel wurde fast vollständig im Vakuum abdestilliert, der ausgefallene Feststoff wurde isoliert und man erhielt 8.23 g eines farblosen klebrigen Harzes.

**N-(3-((2-Ethythexyt)oxy)-3-oxopropyt)-N,N-dimethyloctadecan-1-aminium bromid (K-23)**

Zu einer Lösung aus 10.0 g N,N-Dimethyl-β-Alanin-2-ethylhexylester ([184244-48-2], hergestellt wie in US5565290 A beschrieben) in 30 mL Acetonitril wurden 15.0 g Octadecylbromid gegeben und für 6 h am Rückfluss erhitzt. Das Lösungsmittel wurde fast vollständig im Vakuum abdestilliert und man erhielt 24.50 g eines farblosen Öls.

**N-(2-((Hexylcarbamoyl)oxy)ethyl)-N,N-dimethylhexadecan-1-aminium bromid (K-24)**

Zu einer Mischung von 29.4 g Hexylisocyanat und 0.05 g Desmorapid Z wurden bei 60 °C 20.6 g N,N-Dimethylethanol getropft und für 8 h bei dieser Temperatur gehalten. Nach Abkühlen auf Raumtemperatur wurden 50.0 g Amino-Urethan erhalten.

50.0 g Amino-Urethan wurden in 120 mL Acetonitril gelöst, 70.6 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 115.0 g eines farblosen klebrigen Harzes.

**$N^1$,$N^{16}$-Dihexadecyl-$N^1$,$N^1$,$N^{16}$,$N^{16}$,7,7,10-heptamethyl-4,13-dioxo-3,14-dioxa-5,12-diazahexadecan-1,16-diaminium dibromid (K-25)**

Zu einer Mischung von 27.0 g Vestanat TMDI (Produkt der EVONIK Deutschland) und 0.05 g Desmorapid Z wurden bei 60 °C 22.9 g N,N-Dimethylethanol getropft und für 8 h bei dieser Temperatur gehalten. Nach Abkühlen auf

Raumtemperatur wurden 50.0 g Amino-Urethan erhalten.
11.7 g Amino-Urethan wurden in 50 mL Acetonitril gelöst, 18.3 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 28.0 g eines farblosen klebrigen Harzes.

**N-(2-((((5-(((2-(Hexadecyldimethylammonio)ethoxy)carbonyl)amino)-1,3,3-trimethylcyclohexyl)-methyl)carbamoyl)oxy)ethyl)-N,N-dimethylhexadecan-1-aminium dibromid (K-26)**

Zu einer Mischung von 27.7 g Desmodur I (Produkt der COVESTRO Deutschland) und 0.05 g Desmorapid Z wurden bei 60 °C 22.2 g N,N-Dimethylethanol getropft und für 8 h bei dieser Temperatur gehalten. Nach Abkühlen auf Raumtemperatur wurden 50.0 g Amino-Urethan erhalten.
11.9 g Amino-Urethan wurden in 50 mL Acetonitril gelöst, 18.1 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 29.3 g eines farblosen klebrigen Harzes.

**N,N'-(((((Methylenebis(cyclohexan-4,1-diyl))bis(azanediyl))bis(carbonyl))bis(oxy))bis(ethan-2,1-diyl))bis(N,N-dimethylhexadecan-1-aminium) dibromid (K-27)**

Zu einer Mischung von 29.7 g Desmodur W (Produkt der COVESTRO Deutschland) und 0.05 g Desmorapid Z wurden bei 60°C 20.2 g N,N-Dimethylethanol getropft und für 8 h bei dieser Temperatur gehalten. Nach Abkühlen auf Raumtemperatur wurden 50.0 g Amino-Urethan erhalten.
12.6 g Amino-Urethan wurden in 50 mL Acetonitril gelöst, 17.4 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 28.9 g eines farblosen klebrigen Harzes.

**N,N'-(((((4-Methyl-1,3-phenyten)bis(azandiyl))bis(carbonyl))bis(oxy))bis(ethan-2,1-diyl))-bis(N,N-dimethylhexadecan-1-aminium) dibromid (K-28)**

Zu einer Mischung von 24.7 g Desmodur T80 (Produkt der COVESTRO Deutschland) und 0.05 g Desmorapid Z wurden bei 10 °C 25.3 g N,N-Dimethylethanol getropft und nach vollständiger Zugabe für 8 h bei 60°C gehalten. Nach Abkühlen auf Raumtemperatur wurden 50.0 g Amino-Urethan erhalten.
11.0 g Amino-Urethan wurden in 50 mL Acetonitril gelöst, 19.0 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 27.2 g eines farblosen klebrigen Harzes.

**N,N'-(((((Methylenbis(4,1-phenylene))bis(azandiyl))bis(carbonyl))bis(oxy))bis(ethan-2,1-diyl))bis(N,N-dimethylhexadecan-1-aminium) dibromid (K-29)**

Zu einer Mischung von 29.2 g Desmodur MDI (Produkt der COVESTRO Deutschland) und 0.05 g Desmorapid Z wurden bei 10 °C 20.8 g N,N-Dimethylethanol getropft und nach vollständiger Zugabe für 8 h bei 60°C gehalten. Nach Abkühlen auf Raumtemperatur wurden 50.0 g Amino-Urethan erhalten.
12.4 g Amino-Urethan wurden in 50 mL Acetonitril gelöst, 17.6 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 29.2 g eines farblosen klebrigen Harzes.

**N-(2-(2-((Hexylcarbamoyl)oxy)ethoxy)ethyl)-N,N-dimethylhexadecan-1-aminium bromid (K-30)**

Zu einer Mischung von 29.4 g Hexylisocyanat und 0.05 g Desmorapid Z wurden bei 60 °C 25.6 g 2-(2-Dimethylaminoethoxy)ethanol getropft und für 8 h bei dieser Temperatur gehalten. Nach Abkühlen auf Raumtemperatur wurden 50.0 g Amino-Urethan erhalten.
13.8 g Amino-Urethan wurden in 50 mL Acetonitril gelöst, 16.2 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 25.0 g farblosen Feststoff, Smp. 220-225 °C.

**$N^1$,$N^{22}$-Dihexadecyl-$N^1$,$N^1N^{22}$,$N^{22}$,10,10,13-heptamethyl-7,16-dioxo-3,6,17,20-tetraoxa-8,15-diazadocosan-1,22-diaminium dibromid (K-31)**

Zu einer Mischung von 23.0 g Vestanat TMDI (Produkt der EVONIK Deutschland) und 0.05 g Desmorapid Z

wurden bei 60 °C 27.9 g 2-(2-Dimethylaminoethoxy)ethanol getropft und für 8 h bei dieser Temperatur gehalten. Nach Abkühlen auf Raumtemperatur wurden 50.0 g Amino-Urethan erhalten.

13.1 g Amino-Urethan wurden in 50 mL Acetonitril gelöst, 16.9 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 27.5 g eines farblosen klebrigen Harzes.

**N-(2-(2-(((3-(10,10-Dimethyl-3-oxo-4,7-dioxa-2,10-diazahexaeosan-10-ium-1-yl)-3,5,5-trimethylcyclohexyl)carbamoyl)oxy)ethoxy)ethyl)-N,N-dimethylhexadecan-1-aminium dibromid (K-32)**

Zu einer Mischung von 22.7 g Desmodur I (Produkt der COVESTRO Deutschland) und 0.05 g Desmorapid Z wurden bei 60 °C 27.2 g 2-(2-Dimethylaminoethoxy)ethanol getropft und für 8 h bei dieser Temperatur gehalten. Nach Abkühlen auf Raumtemperatur wurden 50.0 g Amino-Urethan erhalten.

13.3 g Amino-Urethan wurden in 50 mL Acetonitril gelöst, 16.7 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 29.3 g eines farblosen klebrigen Harzes.

**N,N'-((((((Methylenbis(cydohexan-4,1-diyl))bis(azandiyl))bis(carbonyl))bis(oxy))bis(ethan-2,1-diyl))bis(oxy))bis(ethan-2,1-diyl))bis(N,N-dimethylhexadecan-1-aminium) dibromid (K-33)**

Zu einer Mischung von 24.8 g Desmodur W (Produkt der COVESTRO Deutschland) und 0.05 g Desmorapid Z wurden bei 60 °C 25.2 g 2-(2-Dimethylaminoethoxy)ethanol getropft und für 8 h bei dieser Temperatur gehalten. Nach Abkühlen auf Raumtemperatur wurden 50.0 g Amino-Urethan erhalten.

13.9 g Amino-Urethan wurden in 50 mL Acetonitril gelöst, 16.1 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 25.9 g eines farblosen klebrigen Harzes.

**N,N'-((((((4-Methyl-1,3-phenylen)bis(azandiyl))bis-(carbonyl))bis(oxy))bis(ethan-2,1-diyl))bis(oxy))bis(ethan-2,1-diyl))bis(N,N-dimethylhexadecan-1-aminium) dibromid (K-34)**

Zu einer Mischung von 9.90 g Desmodur T80 (Produkt der COVESTRO Deutschland) und 0.05 g Desmorapid Z wurden bei 10 °C 15.1 g 2-(2-Dimethylaminoethoxy)ethanol getropft und nach vollständiger Zugabe für 8 h bei 60°C gehalten. Nach Abkühlen auf Raumtemperatur wurden 25.0 g Amino-Urethan erhalten.

12.6 g Amino-Urethan wurden in 50 mL Acetonitril gelöst, 17.4 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 23.1 g eines farblosen klebrigen Harzes.

**N,N'-((((((Methylenbis(4,1-phenylen))bis(azandiyl))bis(carbonyl))bis(oxy))bis(ethan-2,1-diyl))bis(oxy))bis(ethan-2,1-diyl))bis(N,N-dimethylhexadecan-1-aminium) dibromid (K-35)**

Zu einer Mischung von 12.1 g Desmodur MDI (Produkt der COVESTRO Deutschland) und 0.05 g Desmorapid Z wurden bei 10 °C 12.9 g 2-(2-Dimethylaminoethoxy)ethanol getropft und nach vollständiger Zugabe für 8 h bei 60°C gehalten. Nach Abkühlen auf Raumtemperatur wurden 25.0 g Amino-Urethan erhalten.

13.8 g Amino-Urethan wurden in 50 mL Acetonitril gelöst, 16.2 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 29.4 g eines farblosen klebrigen Harzes.

## Beispiele

Sofern nicht abweichend vermerkt beziehen sich alle Prozentangaben auf Gewichtsprozent.

**Herstellungsvorschrift a1) für Triaryl-organoborate mit Kationen der Wertigkeit n=1**

10.0 mmol des angegebenen Boronsäureesters (I; $R^1$), 30.0 mmol Magnesiumspäne und 10.0 mmol des Salzes (II) wurden in einer Mischung aus 3.40 g wasserfreiem Toluol und 0.73 g wasserfreiem Tetrahydrofuran in einem trockenen Vierhalskolben mit mechanischem Rührer, Thermometer, Metallkühler und Tropftrichter mit Druckausgleich unter Stickstoffatmosphäre suspendiert und für 30 min intensiv gerührt. Zu dieser Lösung wurde zunächst unverdünnt der entsprechende Halogenaromat (III, $R^4$) gegeben, bis einsetzende Exothermie den Reaktionsstart signalisiert. Die restliche Menge von insgesamt 30.6 mmol Halogenaromat wurden mit 10.0 g wasserfreiem Toluol und 10.0 g wasserfreiem Tetrahydrofuran verdünnt und so zugetropft, dass die Innentemperatur 45 °C nicht überschreitet. Nach vollständiger Zugabe wurde das Reaktionsgemisch für 1 h oder bis zur vollständigen Auflösung des Magnesiums am Rückfluss erhitzt. Die Reaktionsmischung wurde auf Raumtemperatur gekühlt und 100 g 1,4-Dioxan wurden zugetropft. Nach Stehen über Nacht wurden die voluminös ausgefallenen Salze abfiltriert, die organische Phase wurde zur Trockne eingeengt und der Rückstand aus 100 mL i-Propanol umkristallisiert. Die Beispiele 15, 18, 20 und 27 sowie 46 - 52 wurden ohne Umkristallisation mit einer Reinheit >90% als Öle isoliert. Die isolierte Ausbeute betrug für nach diesem Verfahren hergestelltes Beispiel 1 2.87 g (72 % d. Theorie), wogegen nach DE 198 50 139 A1 hergestelltes Beispiel 1 in einer Ausbeute von 47 % der Theorie erhalten wurde.

**Herstellungsvorschrift a2) für Triaryl-organoborate mit Kationen der Wertigkeit n=1**

10.0 mmol des angegebenen Boronsäureesters (I; $R^1$), 30.0 mmol Magnesiumspäne und 10.0 mmol des Salzes (II) wurden in einer Mischung aus 3.40 g wasserfreiem Toluol und 0.73 g wasserfreiem Tetrahydrofuran in einem trockenen Vierhalskolben mit mechanischem Rührer, Thermometer, Metallkühler und Tropftrichter mit Druckausgleich unter Stickstoffatmosphäre suspendiert und für 30 min intensiv gerührt. Zu dieser Lösung wurde zunächst unverdünnt der entsprechende Halogenaromat (III, $R^4$) gegeben, bis einsetzende Exothermie den Reaktionsstart signalisiert. Die restliche Menge von insgesamt 30.6 mmol Halogenaromat wurden mit 10.0 g wasserfreiem Toluol und 10.0 g wasserfreiem Tetrahydrofuran verdünnt und so zugetropft, dass die Innentemperatur 45 °C nicht überschreitet. Nach vollständiger Zugabe wurde das Reaktionsgemisch für 1 h oder bis zur vollständigen Auflösung des Magnesiums am Rückfluss erhitzt. Die Reaktionsmischung wurde auf Raumtemperatur gekühlt und 100 g Lewatit® MDS TP 208 wurden zugegeben. Nach 1h wurde vom Harz abfiltriert, die organische Phase wurde zur Trockne eingeengt und der Rückstand aus 100 mL i-Propanol umkristallisiert. Die Beispiele 15, 18, 20 und 27 sowie 46 - 52 wurden ohne Umkristallisation mit einer Reinheit >90% als Öle isoliert. Die isolierte Ausbeute betrug für nach diesem Verfahren hergestelltes Beispiel 12.87 g (72 % d. Theorie), wogegen nach DE 198 50 139 A1 hergestelltes Beispiel 1 in einer Ausbeute von 47 % der Theorie erhalten wurde.

Die Zusammensetzung der Beispiele 1-92 und ausgewählte physikalische Eigenschaften sind in Tabelle 2 zusammengefasst.

Tabelle 2: Übersicht der Verbindungen der Formel (IV) mit n=1

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 1 | Ethylboronsäure-Pinacolester | 1-Brom-4-Fluorbenzol | K-1 | Ethyl | 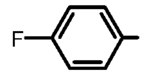 | -10.2 | 101-105 |
| 2 | Ethylboronsäure-Pinacolester | 1-Brom-4-Fluorbenzol | K-2 | Ethyl | 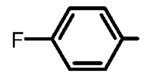 | -10.3 | 144-149 |
| 3 | Ethylboronsäure-Pinacolester | 1-Brom-3-chlor-4-methylbenzol | K-1 | Ethyl | 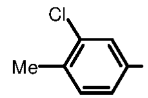 | -10.3 | 88-91 |
| 4 | Ethylboronsäure-Pinacolester | 1-Brom-3-chlor-4-methylbenzol | K-2 | Ethyl | 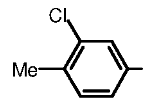 | -10.2 | 120-122 |
| 5 | Isopropylboronsäure-Pinacolester | 1-Brom-4-Fluorbenzol | K-2 | i-Pro-pyl | 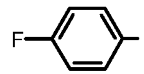 | -10.3 | 134-138 |
| 6 | Isopropylboronsäure-Pinacolester | 1-Brom-3-chlor-4-methylbenzol | K-2 | i-Propyl | 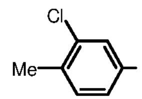 | -8.3 | 115-118 |
| 7 | 2-Isopropenylboronsäure-Pinacolester | 1-Brom-3-chlor-4-methylbenzol | K-2 | 2-Propenyl | 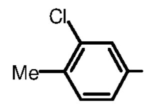 | -8.3 | amorph |
| 8 | Diisopropylallylboronat | 1-Brom-3-chlor-4-methylbenzol | K-1 | Allyl | 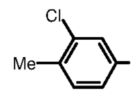 | -10.6 | amorph |
| 9 | Diisopropylallylboronat | 1-Brom-3-chlor-4-methylbenzol | K-2 | Allyl | 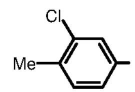 | -10.6 | 84-88 |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [$\delta$/ppm] | Smp. [°C] |
| 10 | (1,3,2-Dioxaborinan-2-yl)cyclohexan | 1-Brom-4-Fluorbenzol | K-1 | Cyclohexyl | 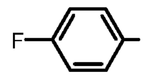 | -10.4 | 59-60 |
| 11 | (1,3,2-Dioxaborinan-2-yl)cyclohexan | 1-Brom-4-Fluorbenzol | K-2 | Cyclohexyl | 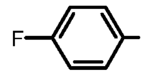 | -10.3 | 166-168 |
| 12 | (1,3,2-Dioxaborinan-2-yl)cyclohexan | 1-Brom-3-chlor-4-methylbenzol | K-2 | Cyclohexyl | 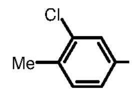 | -8.7 | 175-177 |
| 13 | 2-Hexyl-1,3,2-Dioxaborinan | 1-Brom-4-Fluorbenzol | K-2 | n-Hexyl | 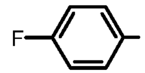 | -10.3 | 59-60 |
| 14 | 1-Dodecylboronsäure-Pinacolester | 1-Brom-4-Fluorbenzol | K-2 | n-Dodecyl | 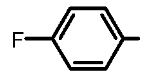 | -10.4 | amorph |
| 15 | 2-Octadecyl-4,4,5,5-tetramethyl-1,3,2-Dioxaborolan | 1-Brom-4-Fluorbenzol | K-2 | n-Octadecyl | 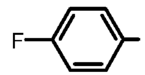 | -10.2 | Öl |
| 16 | 2-Hexyl-1,3,2-Dioxaborinan | 1-Brom-4-chlorbenzol | K-2 | n-Hexyl | 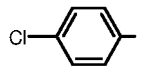 | -10.4 | 80-81 |
| 17 | 1-Dodecylboronsäure-Pinacolester | 1-Brom-4-chlorbenzol | K-2 | n-Dodecyl | 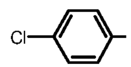 | -10.3 | amorph |
| 18 | 2-Octadecyl-4,4,5,5-tetramethyl-1,3,2-Dioxaborolan | 1-Brom-4-chlorbenzol | K-2 | n-Octadecyl | 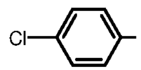 | -10.4 | Öl |
| 19 | 2-Hexyl-1,3,2-Dioxaborinan | 1-Brom-4-trifluormethylbenzol | K-2 | n-Hexyl | 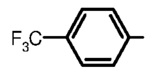 | -9.8 | 80-82 |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 20 | 2-Octadecyl-4,4,5,5-tetramethyl-1,3,2-Dioxaborolan | 1-Brom-3-chlor-4-methylbenzol | K-2 | n-Octadecyl | 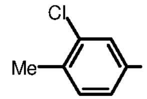 | -10.2 | Öl |
| 21 | 3-Phenyl-1-propylboronsäure-Pinacolester | 1-Brom-4-chlorbenzol | K-2 | 3-Phenylpropyl | 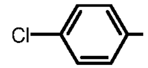 | -10.3 | 101-105 |
| 22 | 3-Phenyl-1-propylboronsäure-Pinacolester | 1-Brom-4-methoxybenzol | K-2 | 3-Phenylpropyl | 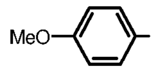 | -10.3 | 116-118 |
| 23 | 3-Phenyl-1-propylboronsäure-Pinacolester | 1-Brom-3,4,5-trifluorbenzol | K-2 | 3-Phenylpropyl | 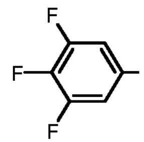 | -10.1 | 78-86 |
| 24 | 3-Phenyl-1-propylboronsäure-Pinacolester | 1-Brom-3-methyl-4-fluorbenzol | K-2 | 3-Phenylpropyl | 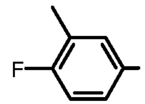 | -13.6 | 85-87 |
| 25 | 3-Phenyl-1-propylboronsäure-Pinacolester | 1-Brom-4-trifluormethoxybenzol | K-2 | 3-Phenylpropyl | 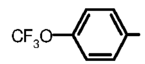 | -10.2 | 101-108 |
| 26 | 2-Hexyl-1,3,2-Dioxaborinan | 1-Brom-3-chlor-4-methylbenzol | K-3 | n-Hexyl | 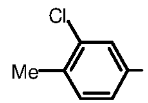 | -10.6 | amorph |
| 27 | 2-Hexyl-1,3,2-Dioxaborinan | 1-Brom-3-chlor-4-methylbenzol | K-4 | n-Hexyl | 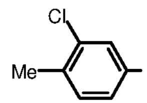 | -10.6 | Öl |
| 28 | 2-Hexyl-1,3,2-Dioxaborinan | 1-Brom-3-chlor-4-methylbenzol | K-5 | n-Hexyl | 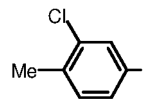 | -10.6 | amorph |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 29 | 2-Hexyl-1,3,2-Dioxaborinan | 1-Brom-3-chlor-4-methylbenzol | K-6 | n-Hexyl | 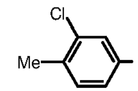 | -10.6 | amorph |
| 30 | 2-Hexyl-1,3,2-Dioxaborinan | 1-Brom-3-chlor-4-methylbenzol | K-7 | n-Hexyl | 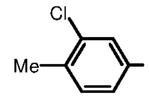 | -10.6 | amorph |
| 31 | 2-Hexyl-1,3,2-Dioxaborinan | 1-Brom-3-chlor-4-methylbenzol | K-8 | n-Hexyl | 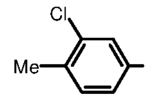 | -10.6 | amorph |
| 32 | 2-Hexyl-1,3,2-Dioxaborinan | 1-Brom-3-chlor-4-methylbenzol | K-9 | n-Hexyl | 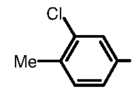 | -10.6 | amorph |
| 33 | 2-Hexyl-1,3,2-Dioxaborinan | 1-Brom-3-chlor-4-methylbenzol | K-10 | n-Hexyl | 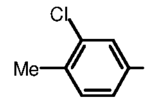 | -10.6 | amorph |
| 34 | 2-Hexyl-1,3,2-Dioxaborinan | 1-Brom-3-chlor-4-methylbenzol | K-11 | n-Hexyl | 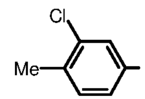 | -10.6 | amorph |
| 35 | 2-Hexyl-1,3,2-Dioxaborinan | 1-Brom-3-chlor-4-methylbenzol | K-12 | n-Hexyl | 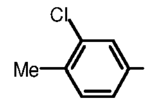 | -10.6 | amorph |
| 36 | 2-Hexyl-1,3,2-Dioxaborinan | 1-Brom-3-chlor-4-methylbenzol | K-13 | n-Hexyl | 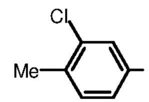 | -10.6 | amorph |
| 37 | 2-Hexyl-1,3,2-Dioxaborinan | 1-Brom-3-chlor-4-methylbenzol | K-16 | n-Hexyl | 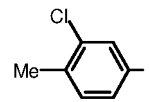 | -10.6 | amorph |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [$\delta$/ppm] | Smp. [°C] |
| 38 | 2-Hexyl-1,3,2-Dioxaborinan | 1-Brom-3-chlor-4-methylbenzol | K-17 | n-Hexyl | 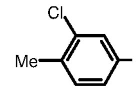 | -10.6 | amorph |
| 39 | 2-Hexyl-1,3,2-Dioxaborinan | 1-Brom-3-chlor-4-methylbenzol | K-19 | n-Hexyl | 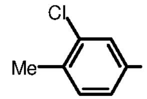 | -10.6 | amorph |
| 40 | 2-Hexyl-1,3,2-Dioxaborinan | 1-Brom-3-chlor-4-methylbenzol | K-20 | n-Hexyl | 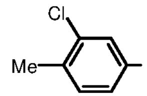 | -10.6 | amorph |
| 41 | 2-Hexyl-1,3,2-Dioxaborinan | 1-Brom-3-chlor-4-methylbenzol | K-21 | n-Hexyl | 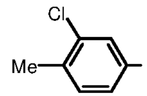 | -10.6 | amorph |
| 42 | 2-Hexyl-1,3,2-Dioxaborinan | 1-Brom-3-chlor-4-methylbenzol | K-22 | Hexyl | 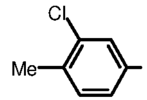 | -10.6 | amorph |
| 43 | 2-Hexyl-1,3,2-Dioxaborinan | 1-Brom-3-chlor-4-methylbenzol | K-23 | n-Hexyl | 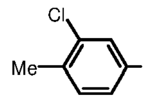 | -10.6 | amorph |
| 44 | 2-Hexyl-1,3,2-Dioxaborinan | 1-Brom-3-chlor-4-methylbenzol | K-24 | n-Hexyl | 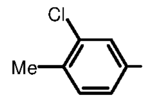 | -10.6 | amorph |
| 45 | 2-Hexyl-1,3,2-Dioxaborinan | 1-Brom-3-chlor-4-methylbenzol | K-30 | n-Hexyl | 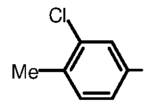 | -10.6 | amorph |
| 46 | 1-Dodecylboronsäure-Pinacolester | 1-Brom-4-chlorbenzol | K-2 | n-Dodecyl | 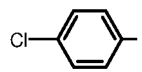 | -10.3 | Öl |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [$\delta$/ppm] | Smp. [°C] |
| 47 | 1-Dodecylboronsäure-Pinacolester | 1-Brom-4-methoxybenzol | K-2 | n-Dodecyl | 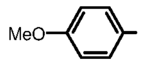 | -10.4 | Öl |
| 48 | 1-Dodecylboronsäure-Pinacolester | 1-Brom-3,4,5-trifluorbenzol | K-2 | n-Dodecyl | 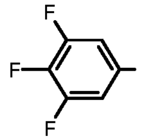 | -10.1 | Öl |
| 49 | 1-Dodecylboronsäure-Pinacolester | 1-Brom-3-methyl-4-fluorbenzol | K-2 | n-Dodecyl | 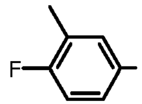 | -10.6 | Öl |
| 50 | 1-Dodecylboronsäure-Pinacolester | 1-Brom-4-trifluormethoxybenzol | K-2 | n-Dodecyl | 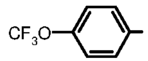 | -10.8 | Öl |
| 51 | 2-Hexyl-1,3,2-Dioxaborinan | 1-Brom-3-chlor-4-methylbenzol | K-100 | n-Hexyl | 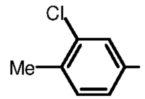 | -10.6 | Öl |
| 52 | 2-Hexyl-1,3,2-Dioxaborinan | 1-Brom-3-chlor-4-methylbenzol | K-101 | n-Hexyl | 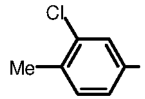 | -10.6 | Öl |
| 53 | 2-Hexyl-1,3,2-Dioxaborinan | 1-Brom-3-chlor-4-methylbenzol | K-102 | n-Hexyl | 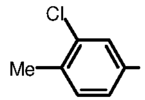 | -10.6 | Zers. |
| 54 | 2-Hexyl-1,3,2-Dioxaborinan | 1-Brom-3-chlor-4-methylbenzol | K-103 | n-Hexyl | 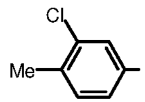 | -10.6 | 102-105 |
| 55 | 2-Hexyl-1,3,2-Dioxaborinan | 1-Brom-3-chlor-4-methylbenzol | K-104 | n-Hexyl | 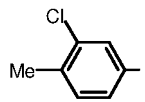 | -10.6 | 134-138 |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [$\delta$/ppm] | Smp. [°C] |
| 56 | 2-Hexyl-1,3,2-Dioxaborinan | 1-Brom-3-chlor-4-methylbenzol | K-105 | n-Hexyl | 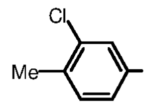 | -10.6 | 145-146 |
| 57 | 2-Hexyl-1,3,2-Dioxaborinan | 1-Brom-3-chlor-4-methylbenzol | K-106 | n-Hexyl | 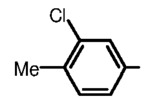 | -10.6 | 155-159 |
| 58 | 2-Hexyl-1,3,2-Dioxaborinan | 1-Brom-3-chlor-4-methylbenzol | K-200 | n-Hexyl | 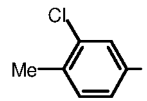 | -10.4 | amorph |
| 59 | 2-Hexyl-1,3,2-Dioxaborinan | 1-Brom-3-chlor-4-methylbenzol | K-201 | n-Hexyl | 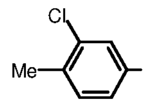 | -10.4 | amorph |
| 60 | 2-Hexyl-1,3,2-Dioxaborinan | 1-Brom-3-chlor-4-methylbenzol | K-202 | n-Hexyl | 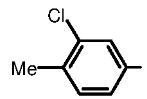 | -10.4 | amorph |
| 61 | 2-Hexyl-1,3,2-Dioxaborinan | 1-Brom-3-chlor-4-methylbenzol | K-300 | n-Hexyl | 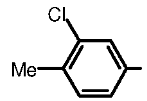 | -10.6 | amorph |
| 62 | 2-Hexyl-1,3,2-Dioxaborinan | 1-Brom-3-chlor-4-methylbenzol | K-303 | n-Hexyl | 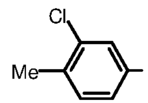 | -10.6 | amorph |
| 63 | 2-Hexyl-1,3,2-Dioxaborinan | 1-Brom-3-chlor-4-methylbenzol | K-303 | n-Hexyl | 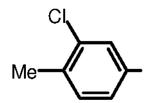 | -10.6 | amorph |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 64 | 2-Hexyl-1,3,2-Dioxaborinan | 1-Brom-3-chlor-4-methylbenzol | K-304 | n-Hexyl | 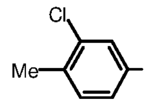 | -10.6 | amorph |
| 65 | 2-Hexyl-1,3,2-Dioxaborinan | 1-Brom-3-chlor-4-methylbenzol | K-305 | n-Hexyl | 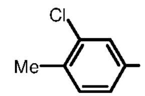 | -10.6 | amorph |
| 66 | 2-Hexyl-1,3,2-Dioxaborinan | 1-Brom-3-chlor-4-methylbenzol | K-400 | n-Hexyl | 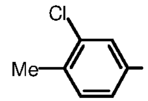 | -10.5 | amorph |
| 67 | 2-Hexyl-1,3,2-Dioxaborinan | 1-Brom-3-chlor-4-methylbenzol | K-401 | n-Hexyl | 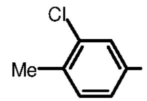 | -10.5 | amorph |
| 68 | 2-Hexyl-1,3,2-Dioxaborinan | 1-Brom-3-chlor-4-methylbenzol | K-402 | n-Hexyl | 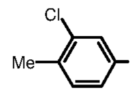 | -10.5 | amorph |
| 69 | 2-Hexyl-1,3,2-Dioxaborinan | 1-Brom-3-chlor-4-methylbenzol | K-403 | n-Hexyl | 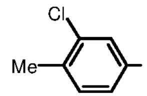 | -10.5 | amorph |
| 86 | 2-Hexyl-1,3,2-Dioxaborinan | 1-Brom-3-chlor-4-methylbenzol | K-37 | n-Hexyl | 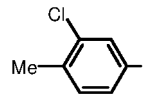 | -10.5 | amorph |
| 87 | 2-Hexyl-1,3,2-Dioxaborinan | 1-Brom-3-chlor-4-methylbenzol | K-38 | n-Hexyl | 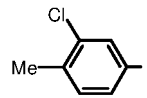 | -10.5 | amorph |
| 88 | 2-Hexyl-1,3,2-Dioxaborinan | 1-Brom-3-chlor-4-methylbenzol | K-39 | n-Hexyl | 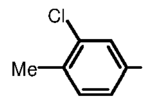 | -10.5 | amorph |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 89 | 2-Hexyl-1,3,2-Dioxaborinan | 1-Brom-3-chlor-4-methylbenzol | K-40 | n-Hexyl | 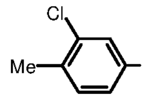 | -10.5 | amorph |
| 90 | 2-Hexyl-1,3,2-Dioxaborinan | 1-Brom-3-chlor-4-methylbenzol | K-41 | n-Hexyl | 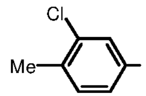 | -10.5 | amorph |
| 91 | 2-Hexyl-1,3,2-Dioxaborinan | 1-Brom-3-chlor-4-methylbenzol | K-42 | n-Hexyl | 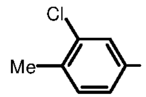 | -10.5 | amorph |
| 92 | 2-Hexyl-1,3,2-Dioxaborinan | 1-Brom-3-chlor-4-methylbenzol | K-43 | n-Hexyl | 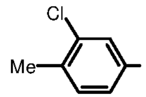 | -10.5 | amorph |

**Herstellungsvorschrift b1) für Triaryl-organoborate mit Kationen der Wertigkeit n=2**

10.0 mmol des angegebenen Boronsäureesters (I; $R^1$), 30.0 mmol Magnesiumspäne und 5.0 mmol des Salzes (II) wurden in einer Mischung aus 3.40 g wasserfreiem Toluol und 0.73 g wasserfreiem Tetrahydrofuran in einem trockenen Vierhalskolben mit mechanischem Rührer, Thermometer, Metallkühler und Tropftrichter mit Druckausgleich unter Stickstoffatmosphäre suspendiert und für 30 min intensiv gerührt. Zu dieser Lösung wurde zunächst unverdünnt der entsprechende Halogenaromat (III, $R^4$) gegeben, bis einsetzende Exothermie den Reaktionsstart signalisiert. Die restliche Menge von insgesamt 30.6 mmol Halogenaromat wurden mit 10.0 g wasserfreiem Toluol und 10.0 g wasserfreiem Tetrahydrofuran verdünnt und so zugetropft, dass die Innentemperatur 45 °C nicht überschreitet. Nach vollständiger Zugabe wurde das Reaktionsgemisch für 1 h oder bis zur vollständigen Auflösung des Magnesiums am Rückfluss erhitzt. Die Reaktionsmischung wurde auf Raumtemperatur gekühlt und 100 g 1,4-Dioxan wurden zugetropft. Nach Stehen über Nacht wurden die voluminös ausgefallenen Salze abfiltriert, die organische Phase wurde zur Trockne eingeengt und der Rückstand aus 100 mL i-Propanol umkristallisiert.

**Herstellungsvorschrift b2) für Triaryl-organoborate mit Kationen der Wertigkeit n=2**

10.0 mmol des angegebenen Boronsäureesters (I; $R^1$), 30.0 mmol Magnesiumspäne und 5.0 mmol des Salzes (II) wurden in einer Mischung aus 3.40 g wasserfreiem Toluol und 0.73 g wasserfreiem Tetrahydrofuran in einem trockenen Vierhalskolben mit mechanischem Rührer, Thermometer, Metallkühler und Tropftrichter mit Druckausgleich unter Stickstoffatmosphäre suspendiert und für 30 min intensiv gerührt. Zu dieser Lösung wurde zunächst unverdünnt der entsprechende Halogenaromat (III, $R^4$) gegeben, bis einsetzende Exothermie den Reaktionsstart signalisiert. Die restliche Menge von insgesamt 30.6 mmol Halogenaromat wurden mit 10.0 g wasserfreiem Toluol und 10.0 g wasserfreiem Tetrahydrofuran verdünnt und so zugetropft, dass die Innentemperatur 45 °C nicht überschreitet. Nach vollständiger Zugabe wurde das Reaktionsgemisch für 1 h oder bis zur vollständigen Auflösung des Magnesiums am Rückfluss erhitzt. Die Reaktionsmischung wurde auf Raumtemperatur gekühlt und 100 g Lewatit® MDS TP 208 wurden zugegeben. Nach 1h wurde vom Harz abfiltriert, die organische Phase wurde zur Trockne eingeengt und der Rückstand aus 100 mL i-Propanol umkristallisiert.

Die Zusammensetzung der Beispiele 93 - 107 und ausgewählte physikalische Eigenschaften sind in Tabelle 3 zusammengefasst.

Tabelle 3: Übersicht der Verbindungen der Formel (IV) mit n=2

| Beispiel | Einsatzstoffe gemäß Herstellungsvorschrift b1) & b2) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 93 | 2-Hexyl-1,3,2-Dioxaborinan | 1-Brom-3-chlor-4-methylbenzol | K-14 | n-Hexyl | 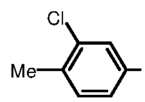 | -10.6 | amorph |
| 94 | 2-Hexyl-1,3,2-Dioxaborinan | 1-Brom-3-chlor-4-methylbenzol | K-15 | n-Hexyl | 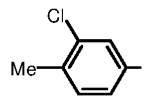 | -10.6 | amorph |
| 95 | 2-Hexyl-1,3,2-Dioxaborinan | 1-Brom-3-chlor-4-methylbenzol | K-18 | n-Hexyl | 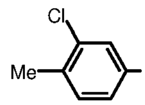 | -10.6 | amorph |
| 96 | 2-Hexyl-1,3,2-Dioxaborinan | 1-Brom-3-chlor-4-methylbenzol | K-25 | n-Hexyl | 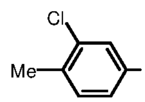 | -10.6 | amorph |
| 97 | 2-Hexyl-1,3,2-Dioxaborinan | 1-Brom-3-chlor-4-methylbenzol | K-26 | n-Hexyl | 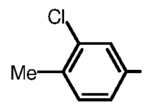 | -10.6 | amorph |

(fortgesetzt)

| Beispiel | Einsatzstoffe gemäß Herstellungsvorschrift b1) & b2) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 98 | 2-Hexyl-1,3,2-Dioxaborinan | 1-Brom-3-chlor-4-methylbenzol | K-27 | n-Hexyl | 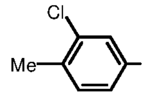 | -10.6 | amorph |
| 99 | 2-Hexyl-1,3,2-Dioxaborinan | 1-Brom-3-chlor-4-methylbenzol | K-28 | n-Hexyl | 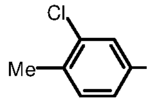 | -10.6 | amorph |
| 100 | 2-Hexyl-1,3,2-Dioxaborinan | 1-Brom-3-chlor-4-methylbenzol | K-29 | n-Hexyl | 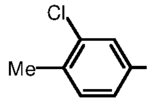 | -10.6 | amorph |
| 101 | 2-Hexyl-1,3,2-Dioxaborinan | 1-Brom-3-chlor-4-methylbenzol | K-31 | n-Hexyl | 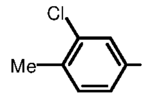 | -10.6 | amorph |
| 102 | 2-Hexyl-1,3,2-Dioxaborinan | 1-Brom-3-chlor-4-methylbenzol | K-32 | n-Hexyl | 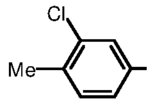 | -10.6 | amorph |
| 103 | 2-Hexyl-1,3,2-Dioxaborinan | 1-Brom-3-chlor-4-methylbenzol | K-33 | n-Hexyl | 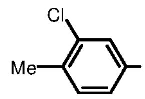 | -10.6 | amorph |
| 104 | 2-Hexyl-1,3,2-Dioxaborinan | 1-Brom-3-chlor-4-methylbenzol | K-34 | n-Hexyl | 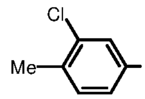 | -10.6 | amorph |
| 105 | 2-Hexyl-1,3,2-Dioxaborinan | 1-Brom-3-chlor-4-methylbenzol | K-35 | n-Hexyl | 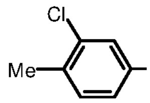 | -10.6 | amorph |
| 106 | 2-Hexyl-1,3,2-Dioxaborinan | 1-Brom-3-chlor-4-methylbenzol | K-203 | n-Hexyl | 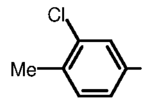 | -10.4 | amorph |
| 107 | 2-Hexyl-1,3,2-Dioxaborinan | 1-Brom-3-chlor-4-methylbenzol | K-301 | n-Hexyl | 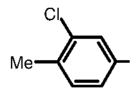 | -10.6 | amorph |

**Beispiel 108 (Kation der Wertigkeit n=3), Fahrweise c1:**

1.70 g 2-Hexyl-1,3,2-Dioxaborinane (10.0 mmol; I; $R^1$ = n-Hexyl), 0.73 g (30.0 mmol) Magnesiumspäne und 2.25 g $N^1$,$N^1$,$N^1$,$N^3$,$N^3$,$N^3$,$N^5$,$N^5$,$N^5$-Nonamethyl-1,3,5-benzentrimethanaminium triiodid (3.33 mmol; K-36; II) werden in einer Mischung aus 3.40 g wasserfreiem Toluol und 0.73 g wasserfreiem Tetrahydrofuran in einem trockenen Vierhalskolben mit mechanischem Rührer, Thermometer, Metallkühler und Tropftrichter mit Druckausgleich unter Stickstoffatmosphäre suspendiert und für 30 min intensiv gerührt. Zu dieser Lösung wird zunächst unverdünntes 1-Brom-3-chlor-4-methylbenzol (III, $R^4$) gegeben, bis einsetzende Exothermie den Reaktionsstart signalisiert. Die restliche Menge von insgesamt 6.29 g (30.6 mmol) 1-Brom-3-chlor-4-methylbenzol werden mit 10.0 g wasserfreiem Toluol und 10.0 g wasserfreiem Tetrahydrofuran verdünnt und so zugetropft, dass die Innentemperatur 45 °C nicht überschreitet. Nach vollständiger Zugabe wird das Reaktionsgemisch für 1 h oder bis zur vollständigen Auflösung des Magnesiums am Rückfluss erhitzt. Die Reaktionsmischung wurde auf Raumtemperatur gekühlt und 100 g 1,4-Dioxan wurden zugetropft. Nach Stehen über Nacht wurden die voluminös ausgefallenen Salze abfiltriert, die organische Phase wurde zur Trockne eingeengt und der Rückstand aus 100 mL i-Propanol umkristallisiert.
$^{11}$B-NMR [$\delta$/ppm] -10.6.

**Beispiel 108 (Kation der Wertigkeit n=3), Fahrweise c2:**

1.70 g 2-Hexyl-1,3,2-Dioxaborinan (10.0 mmol; I; $R^1$ = n-Hexyl), 0.73 g (30.0 mmol) Magnesiumspäne und 2.25 g *N*$^1$,*N*$^1$,*N*$^1$,*N*$^3$,*N*$^3$,*N*$^3$,*N*$^3$,*N*$^5$,*N*$^5$-Nonainethyl-1,3,5-benzenetrimethanaminium triiodid (3.33 mmol; K-36; II) werden in einer Mischung aus 3.40 g wasserfreiem Toluol und 0.73 g wasserfreiem Tetrahydrofuran in einem trockenen Vierhalskolben mit mechanischem Rührer, Thermometer, Metallkühler und Tropftrichter mit Druckausgleich unter Stickstoffatmosphäre suspendiert und für 30 min intensiv gerührt. Zu dieser Lösung wird zunächst unverdünntes 1-Brom-3-chlor-4-methylbenzol (III, $R^4$) gegeben, bis einsetzende Exothermie den Reaktionsstart signalisiert. Die restliche Menge von insgesamt 6.29 g (30.6 mmol) 1-Brom-3-chlor-4-methylbenzol werden mit 10.0 g wasserfreiem Toluol und 10.0 g wasserfreiem Tetrahydrofuran verdünnt und so zugetropft, dass die Innentemperatur 45 °C nicht überschreitet. Nach vollständiger Zugabe wird das Reaktionsgemisch für 1 h oder bis zur vollständigen Auflösung des Magnesiums am Rückfluss erhitzt. Die Reaktionsmischung wurde auf Raumtemperatur gekühlt und 100 g Lewatit® MDS TP 208 wurden zugegeben. Nach 1h wurde vom Harz abfiltriert, die organische Phase wurde zur Trockne eingeengt und der Rückstand aus 100 mL i-Propanol umkristallisiert.
$^{11}$B-NMR [$\delta$/ppm] -10.6.

**Herstellung der Medien zur Bestimmung der holographischen Eigenschaften**

**Beispiel-Medium I**

3.38 g der Polyol-Komponente 1 wurden mit 0.010 g Beispiel 1, 2.00 g Urethanacrylat 1, 2.00 g Urethanacrylat 2, 1.50 g Additiv 1, 0.10 g Triaryl-alkylborat 1 gemäß Formel (IV), 0.010 g Farbstoff 1 und 0.35 g N-Ethylpyrrolidon bei 60 °C gemischt, so dass eine klare Lösung erhalten wurde. Anschließend wurde auf 30 °C abgekühlt, 0.65 g Desmodur® N3900 zugegeben und erneut gemischt. Schließlich wurden 0.01 g Fomrez UL 28 zugegeben und erneut kurz gemischt. Die erhaltene, flüssige Masse wurde dann auf eine Glasplatte gegeben und dort mit einer zweiten Glasplatte abgedeckt. Dieser Probenkörper wurde 12 Stunden bei Raumtemperatur liegen gelassen und gehärtet.

**Beispiel-Medium II-XX**

Es wurde gearbeitet wie im Beispiel Medium I, aber unter Verwendung von 0.10 g des angegebenen Triaryl-organoborates gemäß Formel (IV) anstatt 0.10 g Triaryl-organoborat 1.
Die Eigenschaften der Beispiel-Medien I - XX sind in Tabelle 4 zusammengefasst.

Tabelle 4: Übersicht der holographischen Eigenschaften der Beispiel-Medien I-XX

| **Beispiel-Medium** | **Beispiel gemäß Formel (IV)** | **$\Delta$n** |
|---|---|---|
| I | 1 | 0.033 |
| II | 13 | 0.029 |
| III | 16 | 0.029 |
| IV | 17 | 0.034 |
| V | 18 | 0.033 |
| VI | 19 | 0.032 |
| VII | 20 | 0.037 |
| VIII | 24 | 0.033 |
| IX | 29 | 0.034 |
| X | 45 | 0.039 |
| XI | 51 | 0.035 |

(fortgesetzt)

| Beispiel-Medium | Beispiel gemäß Formel (IV) | $\Delta n$ |
|---|---|---|
| XII | 52 | 0.034 |
| XIII | 57 | 0.037 |
| XIV | 66 | 0.038 |
| XV | 93 | 0.037 |
| XVI | 95 | 0.040 |
| XVII | 97 | 0.040 |
| XVIII | 101 | 0.037 |
| XIX | 102 | 0.035 |
| XX | 104 | 0.037 |

Die gefundenen Werte für die Beispiel-Medien I bis XX zeigen, dass die in den Photopolymer-Formulierungen eingesetzten Verbindungen der Formel (IV) für die Verwendung in holographischen Medien sehr gut geeignet sind.

**Patentansprüche**

1. Verfahren zur Herstellung von Triaryl-organoboraten der Formel $1/n\ K^{n+}R^4_3B^- \text{-} R^1$ (IV), wobei ein Äquivalent Organo-Boronsäureester der Formel $B\text{-}R^1(OR^2)(OR^3)$ (I) mit 1/n Äquivalenten Salz $K^{n+}\ nX^-$ (II) und 3 Äquivalenten Metall M in einem Lösungsmittel oder einem Lösungsmittelgemisch S1 vorgelegt, 3 Äquivalente eines Halogen-Aromaten $R^4$-Y (III) zugegeben werden, einen Hilfsstoff L und gegebenenfalls ein zweites organisches Lösungsmittel oder Lösungsmittelgemisch S2 zugegeben wird und die Verbindung 1/n $K^{n+}$ $R^4_3B^- \text{-} R^1$ (IV) mit der organischen Phase abgetrennt wird und

   $R^1$ für einen gegebenenfalls mit Hydroxy und / oder Alkoxy und / oder Acyloxy und / oder Halogen substituierten $C_1$- bis $C_{22}$-Alkyl-, $C_3$- bis $C_{22}$-Alkenyl-, $C_3$- bis $C_{22}$-Alkinyl-, $C_5$-bis $C_7$-Cycloalkyl- oder $C_7$- bis $C_{13}$-Aralkylrest steht,
   $R^2$ und $R^3$ unabhängig voneinander für einen gegebenenfalls verzweigten $C_1$- bis $C_{22}$-Alkyl-Rest oder einen gegebenenfalls durch Alkyl substituierten $C_3$- bis $C_7$-Cycloalkyl-Rest stehen oder $R^2$ und $R^3$ gemeinsam eine 2-8 gliedrige gegebenenfalls durch Alkyl substituierte und / oder durch Sauerstoff-Atome unterbrochene Kohlenstoff-Brücke bilden,
   $R^4$ für einen $C_6$- bis $C_{10}$-Arylrest steht, der gegebenenfalls durch mindestens einen Rest der Auswahl Halogen, $C_1$-bis $C_4$-Alkyl, Trifluormethyl, $C_1$-bis $C_4$-Alkoxy, Trifluormethoxy, Phenyl oder Phenoxy substituiert ist,
   K für ein beliebig substituiertes Organokation der Wertigkeit n auf Basis von Stickstoff, Phosphor, Sauerstoff, Schwefel, und / oder Iod steht und
   L für einen mit M-Salzen $MY(OR^2)$, $MY(OR^3)$ und MXY in S1 und / oder S2 schwerlöslichen Komplex bildenden Hilfsstoff steht, wobei L eine Lewis-basische Verbindung ist, ausgewählt aus der Gruppe bestehend aus offenkettigen oder cyclischen oder polycyclischen Ethern oder Polyethern oder (Poly)ether-polyolen, Amin- und/oder alkylamin-funktionalisierten Alkoholen oder Polyolen oder (Poly)ether-polyolen, schwach basischen organischen Verbindungen, schwach sauren makroporösen Kationenaustauschern und schwach alkalischen makroporösen Kationenaustauschern,
   M für ein Metall beliebig ausgewählt aus den Alkalimetallen, Magnesium, Calcium oder Aluminium steht,
   X für ein Halogenid oder Alkoxid oder Alkylsulfid steht,
   Y für Iod oder Brom oder Chlor steht,
   n für 1, 2 oder 3 steht,
   S1 für ein aprotisches organisches Lösungsmittel oder ein Gemisch aprotischer Lösungsmittel steht und,
   S2 für ein aprotisches organisches Lösungsmittel oder ein Gemisch aprotischer Lösungsmittel steht,

   wobei sich die Lösungsmittel oder Lösungsmittelgemische S1 und S2 von dem Hilfsstoff L unterscheiden

**2.**

Verfahren zur Herstellung von Verbindungen $1/n\ K^{n+}\ R_3^4B^--R^1$ der Formel IV gemäß Anspruch 1 umfassend die Schritte

i) das Vorlegen von einem Äquivalent Organo-Boronsäureester B-$R^1(OR^2)(OR^3)$ (I) mit 1/n Äquivalenten Salz $K^{n+}$ $nX^-$ (II) und 3 Äquivalenten Metall M in einem Lösungsmittel oder einem Lösungsmittelgemisch S1,
ii) die Zugabe von 3 Äquivalenten eines Halogen-Aromaten $R^4$-Y (III), wodurch
iii) ein *in situ* erzeugtes metallorganisches Reagenz mit den vorgelegten Substanzen (I) und (II) zum 1/n $K^{n+}R_3^4B^--R^1$ (IV) reagiert,
iv) die Zugabe eines Hilfsstoff L und
v) gegebenenfalls eines zweiten organischen Lösungsmittel S2, wobei die Verbindung (IV) in der organischen Phase verbleibt und
vi) die Metallsalze $MY(OR^2)$, $MY(OR^3)$ und MXY als ausgefallene Feststoff-Komplexe $MY(OR^2)L$, $MY(OR^3)L$ und MXYL abgetrennt werden.

**3.** Verfahren gemäß Ansprüchen 1 oder 2, **dadurch gekennzeichnet, dass** es sich bei dem Hilfsstoff L um Lewis-basische Verbindungen mit mindestens einer frei zur Verfügung stehenden Koordinationsstelle oder deren Gemische handelt, vorzugsweise ausgewählt aus der Gruppe bestehend aus cyclischen Ethern oder Polyethern oder (Poly)ether-polyolen, schwach sauren makroporösen Kationenaustauschern und schwach alkalischen makroporösen Kationenaustauschern, noch bevorzugter ausgewählt aus der Gruppe bestehend aus cyclischen Ethern, schwach sauren makroporösen Kationenaustauschern und schwach alkalischen makroporösen Kationenaustauschern.

**4.** Verfahren gemäß einem der Ansprüche 1 bis 3, **dadurch gekennzeichnet, dass** es sich bei den Lösungsmitteln oder Lösungsmittelgemischen S1 und S2 unabhängig voneinander um wasserfreie aprotische organische Lösungsmittel bzw. deren Gemische handelt.

**5.** Verfahren gemäß einem der Ansprüche 1 bis 4, **dadurch gekennzeichnet, dass** es sich bei den Organokationen K der Wertigkeit n auf Basis von Stickstoff um Ammoniumionen, Pyridiniumionen Pyridaziniumionen, Pyrimidiniumionen, Pyraziniumionen, Imidazoliumionen, Pyrrolidiniumionen, die gegebenenfalls in einer oder mehreren Seitenketten weitere funktionelle Gruppen wie Ether, Ester, Amide und / oder Carbamate tragen und die auch in oligomerer oder polymerer oder verbrückender Form vorliegen können, handelt.

**6.** Verfahren gemäß einem der Ansprüche 1 bis 4, **dadurch gekennzeichnet, dass** es sich bei den Organokationen K der Wertigkeit n auf Basis von Phosphor um beliebig substituierte Tetraalkyl-Phosphonium-, Trialkyl-Aryl-Phosphonium-, Dialkyl-Diaryl-Phosphonium-, Alkyl-Triaryl-Phosphonium-, oder Tetraaryl-Phosphonium-Salze, die gegebenenfalls in einer oder mehreren Seitenketten weitere funktionelle Gruppen wie Carbonyle, Amide und / oder Carbamate tragen und die auch in oligomerer oder polymerer oder verbrückender Form vorliegen können, handelt.

**7.** Verfahren gemäß einem der Ansprüche 1 bis 4, **dadurch gekennzeichnet, dass** es sich bei den Organokation K der Wertigkeit n auf Basis von Sauerstoff um beliebig substituiertes Pyrylium, auch in annellierter Form wie im Benzopyrylium, Flavylium Naphthoxanthenium oder polymere Kationen mit den genannten Substitutionsmustern handelt.

**8.** Verfahren gemäß einem der Ansprüche 1 bis 4, **dadurch gekennzeichnet, dass** es sich bei den Organokationen K der Wertigkeit n auf Basis von Schwefel um Onium-Verbindungen des Schwefels, die gleiche oder verschiedene gegebenenfalls substituierte $C_4$- bis $C_{14}$-Alkyl-, $C_6$-bis $C_{10}$-Aryl-, $C_7$- bis $C_{12}$-Arylalkyl- oder $C_5$- bis $C_6$-Cycloalkylreste tragen und / oder oligomere oder polymere wiederkehrende verbindende Einheiten zu Sulfoniumsalzen mit $1 \leq n \leq 3$aufbauen sowie Thiopyrylium oder polymere Kationen mit den genannten Substitutionsmustern handelt.

**9.** Verfahren gemäß einem der Ansprüche 1 bis 4, **dadurch gekennzeichnet, dass** es sich bei den Organokationen K der Wertigkeit n auf Basis von Iod um Onium-Verbindungen des Iods, die gleiche oder verschiedene gegebenenfalls substituierte $C_1$- bis $C_{22}$-Alkyl-, $C_6$- bis $C_{14}$-Aryl-, $C_7$- bis $C_{15}$-Arylalkyl- oder $C_5$- bis $C_7$-Cycloalkylreste tragen und / oder oligomere oder polymere wiederkehrende verbindende Einheiten zu Iodoniumsalzen mit $1 \leq n \leq 3$ aufbauen oder um weitere polymere Kationen mit den genannten Substitutionsmustern handelt.

**10.** Verfahren gemäß einem der Ansprüche 1 bis 9, **dadurch gekennzeichnet, dass** $R^1$ für einen gegebenenfalls mit

Hydroxy und / oder Alkoxy und / oder Acyloxy und / oder Halogen substituierten $C_2$- bis $C_{18}$-Alkyl-, $C_3$- bis $C_{18}$-Alkenyl-, $C_3$- bis $C_{18}$-Alkinyl-, $C_5$- bis $C_6$-Cycloalkyl- oder $C_7$- bis $C_{13}$-Aralkylrest steht.

**11.** Verfahren gemäß einem der Ansprüche 1 bis 10, **dadurch gekennzeichnet, dass** $R^2$ und $R^3$ unabhängig voneinander für einen gegebenenfalls verzweigten $C_2$- bis $C_{18}$-Alkyl-Rest stehen oder $R^2$ und $R^3$ bilden gemeinsam einen 4-7 gliedrigen gegebenenfalls substituierten Carbocylus.

**12.** Verfahren gemäß einem der Ansprüche 1 bis 11, **dadurch gekennzeichnet, dass** $R^4$ für einen $C_6$- bis $C_{10}$-Arylrest, der gegebenenfalls durch mindestens einen Rest der Auswahl Halogen, $C_1$-bis $C_4$-Alkyl, Trifluormethyl, $C_1$-bis $C_4$-Alkoxy, Trifluormethoxy, Phenyl und / oder Phenoxy substituiert ist, steht.

## Claims

**1.** Process for preparing triaryl organoborates of the formula $1/n\ K^{n+}R^4_3B^{-}-R^1$ (IV), where one equivalent of organoboronic ester of the formula $B\text{-}R^1(OR^2)(OR^3)$ (I) is initially charged together with 1/n equivalents of salt $K^{n+}\ nX^-$ (II) and 3 equivalents of metal M in a solvent or a solvent mixture S1, 3 equivalents of a haloaromatic $R^4$-Y (III) are added, an auxiliary L and optionally a second organic solvent or solvent mixture S2 is added and the compound $1/n\ K^{n+}\ R^4_3B^{-}-$ $R^1$ (IV) is separated off with the organic phase and

$R^1$ is an optionally hydroxyl- and/or alkoxy- and/or acyloxy- and/or halogen-substituted $C_1$- to $C_{22}$-alkyl, $C_3$- to $C_{22}$-alkenyl, $C_3$- to $C_{22}$-alkynyl, $C_5$-to $C_7$-cycloalkyl or $C_7$- to $C_{13}$-aralkyl radical,
$R^2$ and $R^3$ are independently an optionally branched $C_1$-to $C_{22}$-alkyl radical or an optionally alkyl-substituted $C_3$- to $C_7$-cycloalkyl radical or $R^2$ and $R^3$ together form a 2-8-membered carbon bridge which is optionally substituted by alkyl and/or interrupted by oxygen atoms,
$R^4$ is a $C_6$- to $C_{10}$-aryl radical optionally substituted by at least one radical selected from halogen, $c_1$-to $C_4$-alkyl, trifluoromethyl, $c_1$-to $C_4$-alkoxy, trifluoromethoxy, phenyl and phenoxy,
K is an organocation of valency n and having any substitution, based on nitrogen, phosphorus, oxygen, sulfur and/or iodine, and
L is an auxiliary that forms a complex of sparing solubility in S1 and/or S2 with M salts $MY(OR^2)$, $MY(OR^3)$ and MXY, where L is a Lewis-basic compound selected from the group consisting of open chain or cyclic or polycyclic ethers or polyethers or (poly)ether polyols, amine-functionalized and/or alkylamine-functionalized alcohols or polyols or (poly)ether polyols, weakly basic organic compounds, weakly acidic macroporous cation exchangers and weakly alkaline macroporous cation exchangers,
M is any metal selected from the alkali metals, magnesium, calcium and aluminium,
X is a halide or alkoxide or alkyl sulfide,
Y is iodine or bromine or chlorine,
n is 1, 2 or 3,
S1 is an aprotic organic solvent or a mixture of aprotic solvents and
S2 is an aprotic organic solvent or a mixture of aprotic solvents,

where the solvents or solvent mixtures S1 and S2 are different from the auxiliary L.

**2.** Process for preparing compounds $1/n\ K^{n+}\ R^4_3B^{-}-R^1$ of the formula IV according to Claim 1, comprising the steps of

i) initially charging one equivalent of organoboronic ester $B\text{-}R^1(OR^2)(OR^3)$ (I) together with 1/n equivalents of salt $K^{n+}\ nX^-$ (II) and 3 equivalents of metal M in a solvent or solvent mixture S1,
ii) adding 3 equivalents of a haloaromatic $R^4$-Y (III), as a result of which
iii) an organometallic reagent generated *in situ* reacts with the initially charged substances (I) and (II) to give $1/n\ K^{n+}\ R^4_3B^{-}-R^1$ (IV),
iv) adding an auxiliary L and
v) optionally a second organic solvent S2, where the compound (IV) remains in the organic phase, and
vi) the metal salts $MY(OR^2)$, $MY(OR^3)$ and MXY are separated off as precipitated solid complexes $MY(OR^2)L$, $MY(OR^3)L$ and MXYL.

3. Process according to Claim 1 or 2, **characterized in that** the auxiliary L comprises Lewis-basic compounds having at least one freely available coordination site or mixtures thereof, preferably selected from the group consisting of cyclic ethers or polyethers or (poly)ether polyols, weakly acidic macroporous cation exchangers and weakly alkaline macroporous cation exchangers, more preferably selected from the group consisting of cyclic ethers, weakly acidic macroporous cation exchangers and weakly alkaline macroporous cation exchangers.

4. Process according to any of Claims 1 to 3, **characterized in that** the solvent or solvent mixtures S1 and S2 are independently anhydrous aprotic organic solvents or mixtures thereof.

5. Process according to any of Claims 1 to 4, **characterized in that** the organocations K of valency n based on nitrogen are ammonium ions, pyridinium ions, pyridazinium ions, pyrimidinium ions, pyrazinium ions, imidazolium ions, pyrrolidinium ions, which optionally bear further functional groups such as ethers, esters, amides and/or carbamates in one or more side chains and which may also be in oligomeric or polymeric or bridging form.

6. Process according to any of Claims 1 to 4, **characterized in that** the organocations K of valency n based on phosphorus are tetraalkylphosphonium, trialkylarylphosphonium, dialkyldiarylphosphonium, alkyltriarylphosphonium or tetraarylphosphonium salts having any substitution, which optionally bear further functional groups such as carbonyls, amides and/or carbamates in one or more side chains and which may also be in oligomeric or polymeric or bridging form.

7. Process according to any of Claims 1 to 4, **characterized in that** the organocation K of valency n based on oxygen is pyrylium having any substitution, including in fused form as in benzopyrylium, flavylium, naphthoxanthenium or polymeric cations with the substitution patterns mentioned.

8. Process according to any of Claims 1 to 4, **characterized in that** the organocations K of valency n based on sulfur are onium compounds of sulfur that bear identical or different optionally substituted $C_4$- to $C_{14}$-alkyl, $C_6$- to $C_{10}$-aryl, $C_7$- to $C_{12}$-arylalkyl or $C_5$- to $C_6$-cycloalkyl radicals and/or that form oligomeric or polymeric repeating connecting units to give sulfonium salts with $1 \leq n \leq 3$, and thiopyrylium or polymeric cations with the substitution patterns mentioned.

9. Process according to any of Claims 1 to 4, **characterized in that** the organocations K of valency n based on iodine are onium compounds of iodine that bear identical or different optionally substituted $C_1$- to $C_{22}$-alkyl, $C_6$- to $C_{14}$-aryl, $C_7$- to $C_{15}$-arylalkyl or $C_5$- to $C_7$-cycloalkyl radicals and/or that form oligomeric or polymeric repeating connecting units to give iodonium salts with $1 \leq n \leq 3$, or are further polymeric cations with the substitution patterns mentioned.

10. Process according to any of Claims 1 to 9, **characterized in that** $R^1$ is an optionally hydroxyl- and/or alkoxy- and/or acyloxy- and/or halogen-substituted $C_2$- to $C_{18}$-alkyl, $C_3$- to $C_{18}$-alkenyl, $C_3$- to $C_{18}$-alkynyl, $C_5$- to $C_6$-cycloalkyl or $C_7$- to $C_{13}$-aralkyl radical.

11. Process according to any of Claims 1 to 10, **characterized in that** $R^2$ and $R^3$ are independently an optionally branched $C_2$- to $C_{18}$-alkyl radical or $R^2$ and $R^3$ together form a 4-7-membered, optionally substituted carbocycle.

12. Process according to any of Claims 1 to 11, **characterized in that** $R^4$ is a $C_6$- to $C_{10}$-aryl radical optionally substituted by at least one radical selected from halogen, $c_1$-to $C_4$-alkyl, trifluoromethyl, $c_1$-to $C_4$-alkoxy, trifluoromethoxy, phenyl and/or phenoxy.

**Revendications**

1. Procédé pour la préparation de triaryl-organoborates de formule $1/n \quad K^{n+}R^4{}_3B^{-}-R^1 \quad (IV)$, un équivalent d'un ester d'acide organo-boronique de formule $B\text{-}R^1(OR^2)(OR^3)$ (I) étant chargé avec 1/n équivalent de sel $K^{n+}$ $nX^-$ (II) et 3 équivalents de métal M dans un solvant ou dans un mélange de solvant S1, 3 équivalents d'un composé aromatique halogéné $R^4$-Y (III) étant ajoutés, un adjuvant L et éventuellement un deuxième solvant organique ou mélange de solvant organique S2 étant ajoutés et le composé 1/n $K^{n+}R^4{}_3B^-\text{-}R^1$ (IV) étant séparé avec la phase organique et

$R^1$ représentant un radical $C_{1\text{-}22}$-alkyle, $C_{3\text{-}22}$-alcényle, $C_{3\text{-}22}$-alcynyle, $C_{5\text{-}7}$-cycloalkyle ou $C_{7\text{-}13}$-aralkyle éven-

tuellement substitué par hydroxy et/ou alcoxy et/ou acyloxy et/ou halogène,
$R^2$ et $R^3$ représentant indépendamment l'un de l'autre un radical $C_{1-22}$-alkyle éventuellement ramifié ou un radical $C_{3-7}$-cycloalkyle éventuellement substitué par alkyle ou $R^2$ et $R^3$ formant ensemble un pont carboné de 2 à 8 chaînons éventuellement substitué par alkyle et/ou interrompu par des atomes d'oxygène,
$R^4$ représentant un radical $C_{6-10}$-aryle, qui est éventuellement substitué par au moins un radical choisi parmi halogène, $C_{1-4}$-alkyle, trifluorométhyle, $C_{1-4}$-alcoxy, trifluorométhoxy, phényle et phénoxy,
K représentant un cation organique substitué de manière quelconque, de valence n, à base d'azote, de phosphore, d'oxygène, de soufre et/ou d'iode et
L représentant un adjuvant formant, avec des sels de M MY($OR^2$), MY ($OR^3$) et MXY, un complexe peu soluble dans S1 et/ou S2, L étant un composé basique au sens de Lewis, choisi dans le groupe constitué par des éthers ou des polyéthers ou des (poly)éther-polyols à chaîne ouverte ou cycliques ou polycycliques, des alcools ou des polyol ou des (poly)éther-polyols fonctionnalisés par une amine et/ou une alkylamine, des composés organiques faiblement basiques, des échangeurs de cations macroporeux faiblement acides et des échangeurs de cations macroporeux faiblement alcalins,
M représentant un métal choisi de manière quelconque parmi les métaux alcalins, le magnésium, le calcium et l'aluminium,
X représentant un halogénure ou un alcoxyde ou un alkylsulfure,
Y représentant iode ou brome ou chlore,
n représentant 1, 2 ou 3,
S1 représentant un solvant organique aprotique ou un mélange de solvants aprotiques et,
S2 représentant un solvant organique aprotique ou un mélange de solvants aprotiques,

les solvants ou les mélanges de solvants S1 et S2 étant différents de l'adjuvant L.

**2.** Procédé pour la préparation de composés 1/n $K^{n+}R^4{}_3B^-$ -$R^1$ de formule IV selon la revendication 1 comprenant les étapes de

i) chargement d'un équivalent d'un ester d'acide organo-boronique de formule B-$R^1$($OR^2$)($OR^3$) (I) avec 1/n équivalent de sel $K^{n+}$ $nX^-$ (II) et 3 équivalents de métal M dans un solvant ou dans un mélange de solvant S1,
ii) ajout de 3 équivalents d'un composé aromatique halogéné $R^4$-Y (III), par lequel
iii) un réactif organométallique produit *in situ* réagit avec les substances chargées (I) et (II) pour donner 1/n $K^{n+}R^4{}_3B^-$-$R^1$ (IV),
iv) ajout d'un adjuvant L et
v) éventuellement d'un deuxième solvant organique S2, le composé (IV) restant dans la phase organique et
vi) les sels métalliques MY($OR^2$) , MY($OR^3$) et MXY étant séparés en tant que complexes de solides précipités MY($OR^2$)L, MY($OR^3$)L et MXYL.

**3.** Procédé selon la revendication 1 ou 2, **caractérisé en ce qu'**en ce qui concerne l'adjuvant L, il s'agit de composés basiques au sens de Lewis comportant au moins un site de coordination librement disponible ou de mélanges de ceux-ci, de préférence choisis dans le groupe constitué par des éthers ou des polyéthers ou des (poly)éther-polyols cycliques, des échangeurs de cations macroporeux faiblement acides et des échangeurs de cations macroporeux faiblement alcalins, plus préférablement choisis dans le groupe constitué par des éthers cycliques, des échangeurs de cations macroporeux faiblement acides et des échangeurs de cations macroporeux faiblement alcalins.

**4.** Procédé selon l'une quelconque des revendications 1 à 3, **caractérisé en ce que** les solvants ou mélanges de solvants S1 et S2 sont indépendamment les uns des autres des solvants organiques aprotiques anhydres ou leurs mélanges.

**5.** Procédé selon l'une quelconque des revendications 1 à 4, **caractérisé en ce que** les cations organiques K de valence n à base d'azote sont des ions ammonium, des ions pyridinium, des ions pyridazinium, des ions pyrimidinium, des pyrazinium, des ions imidazolium, des ions pyrrolidinium, qui portent éventuellement dans une ou plusieurs chaînes latérales d'autres groupes fonctionnels comme des éthers, des esters, des amides et/ou des carbamates et qui peuvent être présents également sous forme oligomérique ou polymérique ou pontée.

**6.** Procédé selon l'une quelconque des revendications 1 à 4, **caractérisé en ce que** les cations organiques K de valence n à base de phosphore sont des sels de tétraalkyl-phosphonium, de trialkyl-aryl-phosphonium, de dialkyl-diaryl-phosphonium, d'alkyl-triaryl-phosphonium ou de tétraarylphosphonium substitués d'une manière quelconque, qui portent éventuellement dans une ou plusieurs chaînes latérales d'autres groupes fonctionnels comme des

carbonyles, des amides et/ou des carbamates et qui peuvent être présents également sous forme oligomérique ou polymérique ou pontée.

7. Procédé selon l'une quelconque des revendications 1 à 4, **caractérisé en ce que** les cations organiques K de valence n à base d'oxygène sont un pyrylium substitué d'une manière quelconque, également sous forme annelée comme dans un benzopyrylium, un flavylium, un naphtoxanthénium ou des cations polymériques comportant les motifs de substitution mentionnés.

8. Procédé selon l'une quelconque des revendications 1 à 4, **caractérisé en ce que** les cations organiques K de valence n à base de soufre sont des composés de type onium du soufre, qui portent les radicaux identiques ou différents $C_{4-14}$-alkyle, $C_{6-10}$-aryle, $C_{7-12}$-arylalkyle ou $C_{5-6}$-cycloalkyle et/ou construisent des sels de sulfonium avec des motifs liants répétitifs oligomériques ou polymériques avec $1 \leq n \leq 3$, ainsi que thiopyrilium ou des cations polymériques comportant les motifs de substitution mentionnés.

9. Procédé selon l'une quelconque des revendications 1 à 4, **caractérisé en ce que** les cations organiques K de valence n à base d'iode sont des composés de type onium de l'iode, qui portent les radicaux identiques ou différents $C_{1-22}$-alkyle, $C_{6-14}$-aryle, $C_{7-15}$-arylalkyle ou $C_{5-7}$-cycloalkyle et/ou construisent des sels d'iodonium avec des motifs liants répétitifs oligomériques ou polymériques avec $1 \leq n \leq 3$, ou des cations polymériques supplémentaires comportant les motifs de substitution mentionnés.

10. Procédé selon l'une quelconque des revendications 1 à 9, **caractérisé en ce que** $R^1$ représente un radical $C_{2-18}$-alkyle, $C_{3-18}$-alcényle, $C_{3-18}$-alcynyle, $C_{5-6}$-cycloalkyle ou $C_{7-13}$-aralkyle éventuellement substitué par hydroxy et/ou alcoxy et/ou acyloxy et/ou halogène.

11. Procédé selon l'une quelconque des revendications 1 à 10, **caractérisé en ce que** $R^2$ et $R^3$ représentent indépendamment l'un de l'autre un radical $C_{2-18}$-alkyle éventuellement ramifié ou $R^2$ et $R^3$ forment ensemble un carbocycle de 4 à 7 chaînons éventuellement substitué.

12. Procédé selon l'une quelconque des revendications 1 à 11, **caractérisé en ce que** $R^4$ représente un radical $C_{6-10}$-aryle, qui est éventuellement substitué par au moins un radical choisi parmi halogène, $C_{1-4}$-alkyle, trifluorométhyle, $C_{1-4}$-alcoxy, trifluorométhoxy, phényle et/ou phénoxy.

**Figur 1**

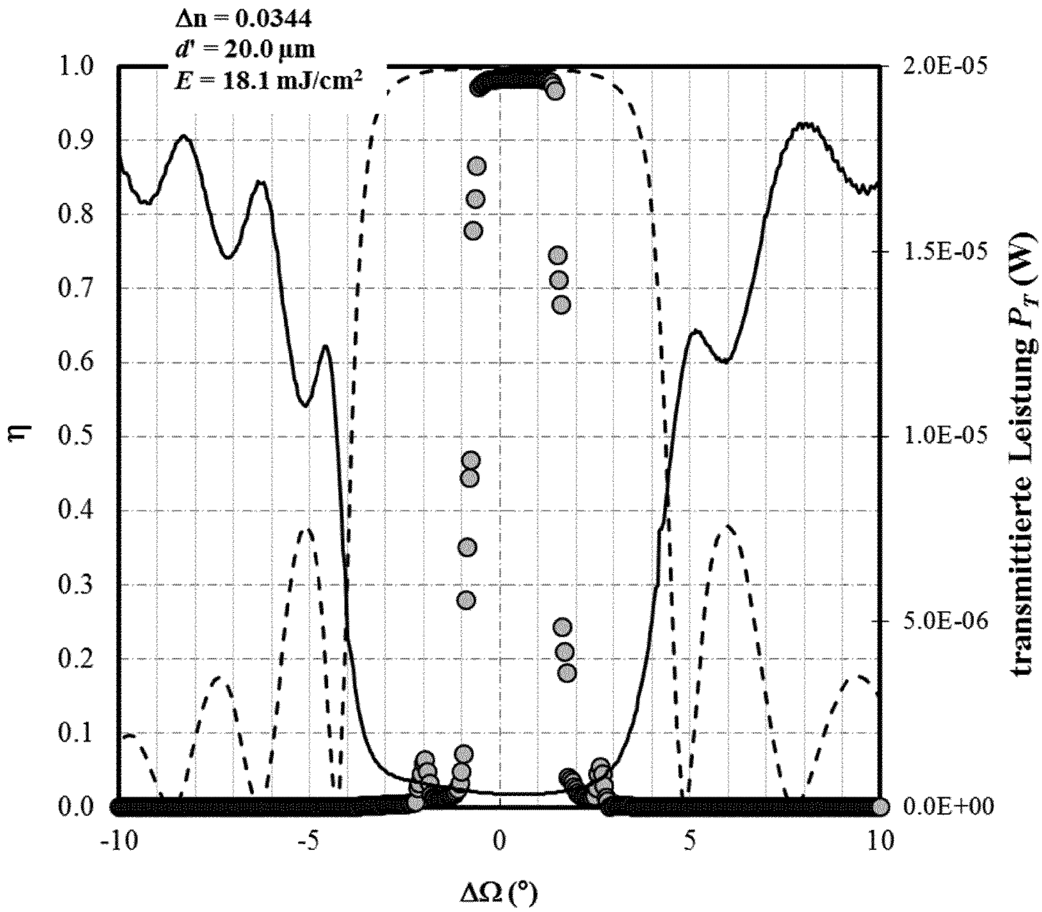
Δn = 0.0344
d' = 20.0 µm
E = 18.1 mJ/cm²
η
1.0
0.9
0.8
0.7
0.6
0.5
0.4
0.3
0.2
0.1
0.0
-10
-5
0
5
10
ΔΩ (°)
2.0E-05
1.5E-05
1.0E-05
5.0E-06
0.0E+00
transmittierte Leistung P_T (W)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19850139 A1
- JP 2002226486 A
- JP 2001233881 A
- WO 2015091427 A1
- JP 2009029857 A
- WO 2018087062 A1
- US 3125555 A
- EP 0223587 A
- WO 2012062655 A
- WO 2015091427 A
- WO 2015055576 A1
- US 5194472 A
- US 2857310 A
- DE 3723797
- US 4954635 A
- WO 2000030444 A
- US 5565290 A


### In der Beschreibung aufgeführte Nicht-Patentliteratur


- **SARKER, ANANDA et al.** *Journal of Polymer Science, Part A: Polymer Chemistry,* 1996, vol. 34 (13), 2817-2824
- **HOWELL, BARBARA et al.** Photopolymerization: Fundamentals and Applications. *ACS Symposium Series,* 1997, vol. 673, 219-232
- *Pure & Appl. Chem.,* 1994, vol. 66 (5), 1077-1184
- **REICHARDT, C.** Solvents and Solvent Effects in Organic Chemistry. Wiley-VCH, 2003
- **H. BERNETH.** Ullmann's Encyclopedia of Industrial Chemistry, Azine Dyes. Wiley-VCH Verlag, 2008
- **H. BERNETH.** Ullmann's Encyclopedia of Industrial Chemistry, Methine Dyes and Pigments. Wiley-VCH Verlag, 2008
- **T. GESSNER ; U. MAYER.** Ullmann's Encyclopedia of Industrial Chemistry, Triarylmethane and Diarylmethane Dyes. Wiley-VCH Verlag, 2000
- *J. Comput. Aid. Mol. Des.,* 2005, vol. 19, 453
- *Virtual Computational Chemistry Laboratory, http://www.vcclab.org berechnet*
- *Organometallics,* 1983, vol. 2 (10), 1311-16
- *CHEMICAL ABSTRACTS,* 64-20-0, 1643-19-2, 1643-19-2, 57-09-0, 121-54-0, 61546-01-8, 64697-40-1, 114569-84-5, 81505-35-3, 1224595-52-1
- *Fuel Processing Technology,* 2014, vol. 118, 296-301
- *J. Am. Chem. Soc.,* 2002, vol. 124 (11), 2604-2613
- *Zhurnal Neorganicheskoi Khimii,* 1978, vol. 23, 825-6
- *Chemische Berichte,* 1984, vol. 117 (4), 1487-96
- *J. Am. Chem. Soc.,* 1955, vol. 77, 485-6
- *Taiwan Kexue,* 1959, vol. 13, 95-8
- *Chemical Engineering Science,* vol. 69 (1), 483-491
- *Chem. Ber.,* 1984, vol. 117, 1487-1496
- Izvestiya Vysshikh Uchebnykh Zavedenii. *Khimiya i Khimicheskaya Tekhnologiya,* 1973, vol. 16 (6), 891
- *Chemicky-Prumysl,* 1967, vol. 17 (2), 67-9